# EUROPEAN PATENT SPECIFICATION

(11) **EP 0 674 651 B1**
(45) Date of publication and mention of the grant of the patent: **28.10.1998**
(21) Application number: 94906449.7
(22) Date of filing: 17.12.1993
(51) Int. Cl.: C07J 3/00, A61K 31/58, C07C 211/40, C07C 211/52

(54) **SUBSTITUTED 6-AZAANDROSTENONES**
SUBSTITUIERTE 6-AZA-ANDROSTENONE
6-AZAANDROSTENONES SUBSTITUEES

(30) Priority: 18.12.1992 US 993930; 18.06.1993 US 80665
(43) Date of publication of application: 04.10.1995
(73) Proprietor: GLAXO WELLCOME INC., Research Triangle Park, North Carolina 27709 (US)
(72) Inventor: ANDREWS, Robert Carl, Reasearch Triangle Park, NC 27709 (US); CRIBBS, Cynthia Markert, Research Triangle Park, NC 27709 (US); FRYE, Stephen Vernon, Research Triangle Park, NC 27709 (US); HAFFNER, Curt Dale, Research Triangle Park, NC 27709 (US); MALONEY, Patrick Reed, Research Triangle Park, NC 27709 (US)
(74) Representative: Learoyd, Stephanie Anne
(86) International application number: US9312419
(87) International publication number: WO9414833

(56) References cited:
- WO-A-93/13124
- TETRAHEDRON, vol.24, no.2, January 1968, OXFORD, GB pages 845 - 857 J. P. KUTNEY ET AL 'Aza steroids - VII. Synthesis of ring A-oxygenated 6-aza-steroids'
- CANADIAN JOURNAL OF CHEMISTRY, vol.58, no.23, 1 December 1980, OTTAWA, CA pages 2666 - 2678 D. VINOD ET AL 'Resolution of conflicting migratory reports in ring expansion of 3-keto steroids to oxygen and nitrogen'

## Description

The present invention relates to certain substituted 17β-substituted -6-azaandrost-4-en-3-ones and their use as 5α-testosterone reductase inhibitors.

### BACKGROUND OF THE INVENTION

Androgens are responsible for many physiological functions in both males and females. Androgen action is mediated by specific intracellular hormone receptors expressed in androgen responsive cells. Testosterone, the major circulating androgen, is secreted by Leydig cells of the testes under the stimulation of pituitary-derived luteinizing hormone (LH). However, reduction of the 4,5 double bond of testosterone to dihydrotestosterone (DHT) is required in some target tissues, such as prostate and skin, for androgen action. Steroid 5α-reductases in target tissues catalyze conversion of testosterone to DHT in an NADPH dependent fashion as shown in Scheme A.

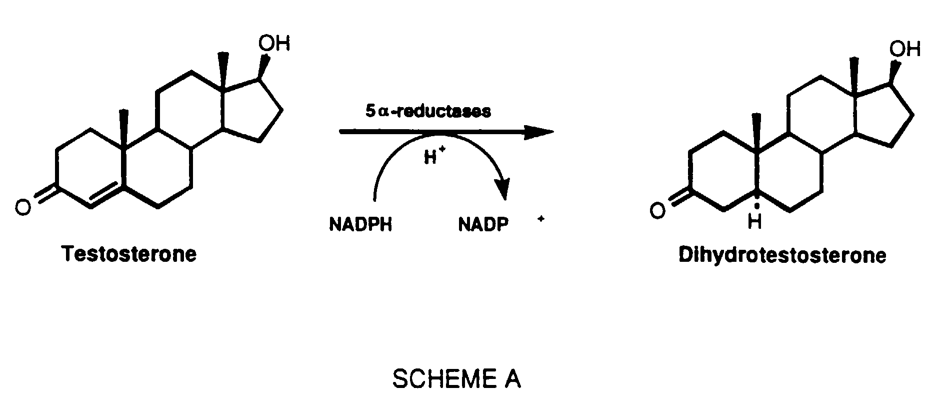

The requirement for DHT to act as an agonist in these target tissues has been highlighted by studies of steroid 5α-reductase deficient individuals who have vestigial prostate glands and do not suffer from acne vulgaris or male pattern baldness (see McGinley, J. *et al., The New England J. of Medicine,* **300**, 1233 (1979)). Thus, inhibition of the conversion of testosterone to DHT in these target tissues is anticipated to be useful in the treatment of a variety of androgen responsive diseases, *e.g.*, benign prostatic hyperplasia, prostate cancer, acne, male pattern baldness, and hirsutism.

Additionally, it has recently been discovered that two isozymes of 5α-reductase exist in humans which differ in their tissue distribution, affinity for testosterone, pH profile and sensitivity to inhibitors (see Russell, D.W. *et al., J. Clin. Invest.,* **89**, 293 (1992); Russell, D.W. *et al., Nature*, **354**, 159 (1991)). The steroid 5α-reductase deficient individuals studied by Imperato-McGinley are deficient in the type 2, 5α-reductase enzyme (Russell, D.W. *et al., J. Clin. Invest.,* **90**, 799 (1992); Russell, D.W. *et al., New England J. Med.,* **327**, 1216 (1992)), which is the predominant isozyme present in the prostate, while the type 1 isozyme is predominant in the skin. The relative value of isozyme specific and dual inhibitors of the two isozymes of 5α-reductase will depend upon the type of disease treated (benign prostatic hyperplasia, prostate cancer, acne, male pattern baldness, or hirsutism) as well as the stage of the disease (prevention versus treatment) and the anticipated side-effects in the intended patients (for example treatment of acne vulgaris in pubescent males).

Because of their valuable therapeutic potential, testosterone 5α-reductase inhibitors [hereinafter "5α-reductase inhibitors"] have been the subject of active research worldwide. For example, see: Hsia, S. and Voight, W., *J. Invest. Derm.,* **62**, 224 (1973); Robaire, B. *et al., J. Steroid Biochem.,* **8**, 307 (1977); Petrow, V. *et al., Steroids,* **38**, 121 (1981); Liang, T. *et al., J. Steroid Biochem.,* **19**, 385 (1983); Holt, D. *et al., J. Med. Chem.,* **33**, 937 (1990); U.S. Patent No. 4,377,584, U.S. Patent No. 4,760,071 and U.S. Patent No. 5,017,568. Two particularly promising 5α-reductase inhibitors are MK-906 (Merck), known by the generic name, finasteride, and marketed under the trademark, Proscar; and SKF-105657 (SmithKline Beecham), shown in Scheme B.

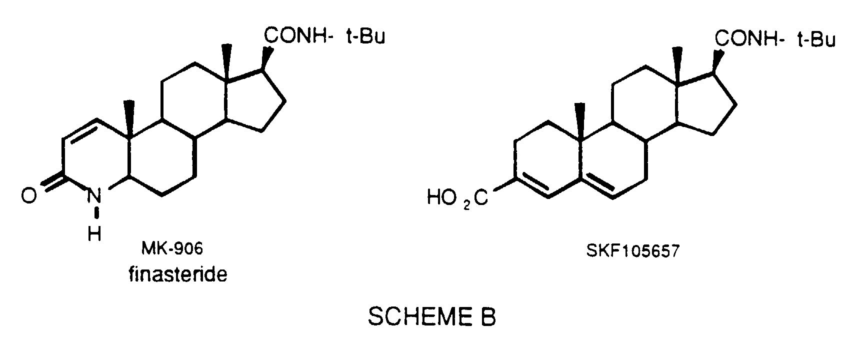

The potent inhibition of bovine adrenal and porcine granulosa cell 3β-hydroxy-Δ⁵-steroid dehydrogenase / 3-keto-Δ⁵-steroid isomerase (3βHSD) by the 4-azasteroid derivative, 4-MA, shown in Scheme C and not by the drug finasteride (Tan, C.H.; Fong, C.Y.; Chan, W.K. *Biochem. Biophys. Res. Comm.,* **144**, 166 (1987) and Brandt, M.; Levy, M.A. *Biochemistry,* **28**, 140 (1989)) along with the critical role of 3βHSD in steroid biosynthesis (Potts, G.O. *et al., Steroids,* **32**, 257 (1978)), suggests that optimal inhibitors of type 1 and 2 5α-reductase should also be selective versus human adrenal 3βHSD.

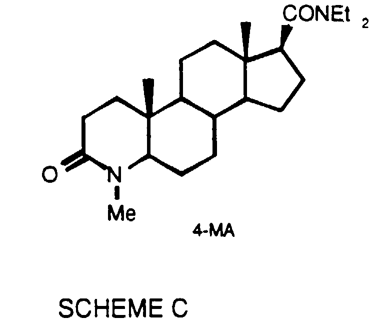

The importance of selectivity in 5α-reductase inhibitors has been emphasized by reports of hepatotoxicity in certain 4-azasteroids such as 4-MA (McConnell, J.D. *The Prostate Suppl.,* **3**, 49 (1990) and Rasmusson, G.H. *et al. J. Med. Chem. ,* **27**, 1690 (1984)).

WO93/13124, published after but filed prior to the priority date of the present application, citable under A54(3) only, refers to 6 aza androstenones. The compounds of the present case differ from those in WO93/13124 in the substituents (hereinafter referred to as Y and Z) at C-17 of the aza androstenone strucure.

### SUMMARY OF THE INVENTION

One aspect of the present invention are the compounds of formula (I), 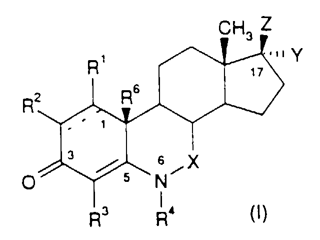 wherein
R¹ and R²
   i) are independently hydrogen or lower alkyl and the bond between the carbons bearing R¹ and R² is a single or a double bond,
      or
   ii) taken together are a -CH₂- group forming a cyclopropane ring, and the bond between the carbons bearing R¹ and R² is a single bond;
R³ is,
   hydrogen, -Alk¹-H (optionally substituted with one or more halogens), lower cycloalkyl, lower cycloalkyl-lower alkyl), halogen, -(Alk¹)ₙ-CO₂H, -(Alk¹)ₙ-CO₂R⁷, -(Alk¹)ₙ-Ar¹, -(Alk¹)ₙ-CONR⁸R⁹, -(Alk¹)ₙ-NR⁸R⁹, -(Alk¹)ₙ-S(O)ᵣR⁷, -(Alk¹)ₙ-CN, -(Alk¹)-OH, -(Alk¹)ₙ-COR⁷; or -(Alk¹)ₙ-OR⁷ ;
   wherein
   Alk¹ is lower alkylene, lower alkenylene or lower alkynylene,
   n is 0 or 1,
   r is 0, 1 or 2,
   R⁷ is -Alk¹-H, -(Alk¹)ₙ-Ar¹ or lower cycloalkyl,
   R⁸ and R⁹ are independently hydrogen, -Alk¹-H or lower cycloalkyl,
   Ar¹ is a homocyclic aryl group of 6 to 14 carbons;
R⁴ is,
   hydrogen, -Alk¹-H, lower cycloalkyl, lower cycloalkyl-lower alkyl, -(Alk¹)ₙ-S(O)ᵣR⁷, -(Alk¹)ₙ-phthalimidyl, -(Alk¹)-CO₂H, -(Alk¹)ₙ-CO₂R⁷, -(Alk¹)ₙ-COR⁷, -(Alk¹)ₙ-Ar¹, -(Alk¹)ₙ-CONR⁸R⁹, -(Alk¹)ₙ-NR⁸R⁹, -(Alk¹)ₙ-OH or -(Alk¹)ₙ-OR⁷;
X is, 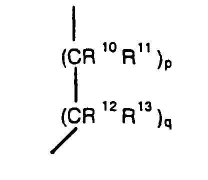 wherein
R¹⁰, R¹¹, R¹² and R¹³ are independently hydrogen or lower alkyl, p and q are independently either 0 or 1;
   and,
   i) Y is hydrogen or hydroxy and
      Z is -(Alk²)ₙ-COR⁵, -(Alk²)ₙ-CO₂R⁵, -(Alk²)ₙ-COSR⁵,
      -(Alk²)ₙ-CONR¹⁴R¹⁵,-(Alk²)-OCO₂R⁵,-(Alk²)-OCOR⁵,
      -(Alk²)-OCONR¹⁴R¹⁵, -(Alk²)-OR⁵, -(Alk²)-NR^{5'}COR⁵, -(Alk²)-NR^{5'}CO₂R⁵, -(Alk²)-NR⁵CONR¹⁴R¹⁵, -(Alk²)ₙ-CONR⁵NR¹⁴R¹⁵,
      -(Alk²)ₙ-CONR⁵CONR¹⁴R¹⁵, -(Alk²)-NR⁵CSNR¹⁴R¹⁵ or -(Alk²)ₙ-CONR⁵CSNR¹⁴R¹⁵;
      wherein

   Alk² is (C₁₋₁₂) alkylene, (C₂₋₁₂) alkenylene or (C₂₋₁₂) alkynylene, R⁵ and R^{5'} are independently hydrogen, -Alk¹-H (optionally substituted independently with one or more CO₂H, CO₂R⁷, Ar², Ar³ or cyano groups) -(Alk¹)ₙ-(lower cycloalkyl (optionally substituted independently with one or more -Alk¹-H groups)), adamantyl, norbornyl, Ar², Ar³, (lower cycloalkyl)-Ar² or (lower cycloalkyl)-Ar³;
      wherein
   Ar² is a homocyclic aromatic group of 6 to 14 carbon ring atoms (optionally substituted independently with one or more -Alk²-H (optionally substituted independently with one or more halogens), -(Alk¹)ₙCOR^{7,} -(Alk¹)ₙ-OH,
      -(Alk¹)ₙ-OR¹⁶, -(Alk¹)ₙ-Ar³, -(Alk¹)ₙ-CO₂H, -(Alk¹)ₙ-CO₂R⁷, S(O)ᵣR⁷, NR⁸S(O)ᵣR¹⁶, NR⁸R⁹, CONR⁸R⁹, lower cycloalkyl, lower alkoxy, -(Alk¹)ₙ-Ar¹ (optionally substituted with one or more -Alk¹-H or halogen), methylenedioxy, ethylenedioxy, morpholino, thiomorpholino, cyano, nitro or halogens); wherein
   R¹⁶ is -Alk¹-H (optionally substituted independently with one or more halogens), lower cycloalkyl (optionally substituted independently with one or more halogens, or -Alk¹-H (optionally substituted independently with one or more halogens)) or -(Alk¹)ₙ-Ar¹ (wherein Ar¹ is optionally substituted independently with one or more, lower alkoxy, cyano groups, halogens or -Alk¹-H (optionally substituted independently with one or more halogens));
   Ar³ is an aromatic group of 5 to 14 ring atoms, at least one of which is O, N or S, (optionally substituted independently with one or more -Alk¹-H (optionally substituted independently with one or more halogens), lower cycloalkyl, lower alkoxy, CO₂H, CO₂R⁷, -(Alk¹)ₙ-Ar¹, cyano or halogen);
   R¹⁴ and R¹⁵ are,
      a) independently, hydroxy, hydrogen, -Alk²-H, lower alkoxy,
         -(Alk¹)ₙ-adamantyl, -(Alk¹)ₙ-myrantyl, -(Alk¹)ₙ-norbornyl, -(Alk¹)ₙ-fluorenyl, -(Alk¹)ₙ-fluorenonyl, -(Alk¹)ₙ-indanyl (optionally substituted with one or more -Alk¹-H), -Alk¹-H (optionally substituted independently with one or more, halogens, cyano, cycloalkyl, SR⁵, COR⁵, CONR⁵R⁷, NR^{5'}COR⁵, NR^{5'}CO₂R⁵, NR^{5'}CONHR⁵, CO₂R⁵, OR⁵, Ar² or Ar³), Ar² or Ar³ or a saturated C₄₋₁₈ bicyclic ring or C₃₋₁₁ saturated ring, optionally containing an oxygen or sulfur atom (said rings optionally substituted independently with one or more cyano, R¹⁶, Ar², Ar³);
      b) alkylene groups (optionally substituted with one or more R⁷ groups, taken together with the linking nitrogen to form a 4 to 8 atom heterocyclic group) 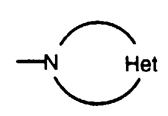 wherein;
         Het represents -O-, -CH₂-, -S(O)ᵣ-, -(NH)- or -(N(Alk¹-H))-; with the proviso that

   when Z is
      -(Alk²)ₙ-COR⁵, -(Alk²)ₙ-CO₂R⁵ or -(Alk²)ₙ-CO-thiopyridyl and
   R⁵ is
      hydrogen, -Alk¹-H, lower cycloalkyl, or adamantyl or
   when Z is
      -(Alk²)ₙ-CONR¹⁴R¹⁵ and
   R¹⁴ and R¹⁵ are
      a) independently hydrogen, -Alk²-H, lower cycloalkyl, lower alkoxy, adamantyl, -Ar¹, benzyl, diphenylmethyl, triphenylmethyl or -(Alk¹)ₙ-norbornyl; or
      b) carbon atoms, optionally substituted with one or more lower alkyl groups, taken together with the linking nitrogen to form a 4 to 8 atom heterocylic ring as herein before defined,
   Y is hydroxy; or
ii) Y is hydrogen and
   Z is
      OR⁵, OCOR⁵, OCONR¹⁴R¹⁵, NR^{5'}COR⁵, NR^{5'}CO₂R⁵, NR⁵CONR¹⁴R¹⁵ or NR⁵CSR¹⁴R¹⁵; and
iii) Y and Z taken together are
   =O, =CH-(Alk¹)ₙ-COR⁵, =CH-(Alk¹)ₙ-CO₂R⁵ or =CH-(Alk¹)ₙ-CONR¹⁴R¹⁵;
   R⁶ is,
      hydrogen or methyl; and pharmaceutically acceptable salts thereof.

Other aspects of the invention are:
1. An invitro method of inhibiting testosterone-5α-reductases comprising contacting testosterone-5α-reductases with a compound of formula (I).
2. Use of a compound of formula (I) for the manufacture of a medicament for the treatment of an androgen responsive or mediated disease either alone or in combination with an alpha 1 adrenergic blocker (eg terazozin), antiandrogen (eg flutamide) or an antioestrogen.
3. Pharmaceutical formulations containing a compound of formula (I) as an active ingredient.

### DETAILED DESCRIPTION OF THE INVENTION

As used herein the term "lower" in reference to alkyl and alkoxy means 1-6 carbons, straight or branched chain, e.g., methyl, ethyl, propyl, butyl, pentyl and hexyl; and methoxy, ethoxy, propoxy, butoxy, pentoxy and hexoxy respectively.

In reference to alkenyl or alkynyl "lower" means 2-7 carbons, straight or branched chain e.g., ethenyl, propenyl, butenyl, pentenyl and hexenyl; and ethynyl, propynyl, butynyl, pentynyl and hexynyl respectively. In reference to cycloalkyl "lower" means 3-7 carbons, i.e., cyclopropyl, cyclobutyl, cyclopentyl, cyclohexyl, and cycloheptyl, preferably 3-6 carbons. The term "lower cycloalkyl-lower alkyl" means a lower alkyl bearing a lower cycloalkyl, e.g., cyclopropylmethyl which may also be named methylene-cyclopropyl. Preferably "-Alk²-" is the same as "-Alk¹".

The term "alkanoyl of 2-6 carbons" refers to alkyl, straight or branched, carboxylic acid groups with a total of 2-6 carbons attached to the structure of formula (I) at a carbon of the alkyl portion of the group, e.g., -CH₂COOH, -(CH₂)₂COOH, -(CH₂)₃COOH, -(CH₂)₄COOH and -(CH₂)₅COOH. The term "halogen" means fluoro, chloro, bromo and iodo moieties. Where -(Alk¹)-H is optionally substituted with one or more halogens, the fluoro moiety is preferable, e.g., trifluoromethyl.

The term "aromatic group" includes, but is not limited to, phenyl, napthyl, anthryl, thiophenyl, isothiazolyl, pyrrolyl, imidazolyl, oxazolyl, isoxaolyl, quinolyl, isoquinolyl, indanyl, pyridyl and furyl. The term "aryl" means homocylic aromatic groups having 6 to 14 carbons, e.g., phenyl, naphthyl and anthryl.

Where R¹⁴ and R¹⁵ are carbon atoms, optionally substituted with one or more lower alkyl groups, taken together with the linking nitrogen form a 4 to 8 atom heterocyclic group, such groups which may be formed include, but are not limited to, pyrrolidinyl, imidazolidinyl, pyrazolidinyl, piperidinyl, piperazinyl, morpholinyl or thiomorpholinyl each optionally substituted with one or more lower alkyl groups.

Examples of C₃₋₁₁ saturated rings containing an oxygen or sulfur atom include, but are not limited to tetrahydropyran and tetrahydrothiopyran. An example of a saturated C₄₋₁₈ bicyclic ring is bicyclononyl.

Unless specified otherwise, attachment of heteroaromatic or non-aromatic heterocyclic groups containing nitrogen and/or sulfur may be through any carbon or nitrogen. However, if the heterocyclic group is non-aromatic, the preferred position of attachment is through the nitrogen. Further, if the group is a six member, hetero-aromatic ring, attachment through a carbon atom is preferred.

Preferably R³ is hydrogen, halogen, lower alkyl, lower cycloalkyl or lower cycloalkyl-lower alkyl, especially hydrogen, halogen or lower alkyl.

R⁴ is preferably hydrogen, lower alkyl, lower cycloalkyl or lower cycloalkyl-lower alkyl, especially hydrogen or lower alkyl.

X is preferably -CH₂-.

Preferably Y is hydrogen and Z is -(Alk²)ₙ-COR⁵, -(Alk²)ₙ-CONR¹⁴R¹⁵, -(Alk²)-OCO₂R⁵, - (Alk²)-OCOR⁵, -(Alk²)-NR^{5'}COR⁵, -(Alk²)ₙ-CONR⁵NR¹⁴R¹⁵, -(Alk²)-NR⁵CONR¹⁴R¹⁵, or -(Alk²)ₙ-CONR⁵CONR¹⁴R¹⁵, especially -COR⁵, -CONR¹⁴R¹⁵, -CH₂OCO₂R⁵, -CH₂OCOR⁵, -CH₂NR^{5'}COR⁵, -CONR⁵NR¹⁴R¹⁵, -CH₂NR⁵CONR¹⁴R¹⁵ or -CONR⁵CONR¹⁴R¹⁵.

Compounds wherein Y is hydrogen and Z is -CONR¹⁴R¹⁵ are particularly of interest.

Ar¹ is preferably a phenyl group, and Ar² is preferably a phenyl group optionally substituted independently with one or more -Alk²-H (optionally substituted independently with one or more halogens), -OR¹⁶, -S(O)ᵣR⁷, Ar¹, methylenedioxy, ethylenedioxy, morpholino, thiomorpholino, cyano, nitro or halogen groups.

Preferably R¹⁴ is hydrogen or hydroxy, and R¹⁵ is hydrogen, lower cycloalkyl (optionally substituted independently with one or more R⁷ or Ar² groups), -(Alk¹)-adamantyl, -(Alk¹)ₙ-myrantyl, -(Alk¹)ₙ-norbornyl, -(Alk¹)ₙ-fluorenyl, -(Alk¹)ₙ-indanyl, -Alk¹-H (optionally substituted independently with one or more lower cycloalkyl, SR⁵, OR⁵, Ar² or Ar³ groups), Ar² or Ar³; or
R¹⁴ and R¹⁵ are carbon atoms, optionally substituted with one or more R⁷ groups, taken together with the linking nitrogen to form a 5 to 7 atom heterocyclic group 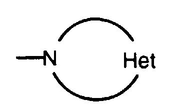 wherein Het represents -CH₂-.

Preferably Ar³ is an aromatic group of five or six ring atoms, at least one of which is O, N or S, optionally substituted independently with one or more Alk¹H groups, especially optionally substituted pyrrolyl, thienyl, furyl and pyridyl groups.

R⁵ is preferably hydrogen, lower alkyl optionally substituted independently with one or more Ar² groups, (lower alkyl)ₙ-lower cycloalkyl, menthyl, adamantyl, norbornyl or Ar².

A particular group of the compounds of formula (I) are the compounds wherein:
R¹ and R²
   i) are independently hydrogen or lower alkyl and the bond between the carbons bearing R¹ and R² is a single or a double bond,
      or
   ii) taken together are a -CH₂- group forming a cyclopropane ring, and the bond between the carbons bearing R¹ and R² is a single bond;
R³ is,
   hydrogen, -Alk¹-H (optionally substituted with one or more halogens), lower cycloalkyl, lower cycloalkyl-lower alkyl), halogen, -(Alk¹)ₙ-CO₂H, -(Alk¹)ₙ-CO₂R⁷, -(Alk¹)ₙ-Ar¹, -(Alk¹)ₙ-CONR⁸R⁹, -(Alk¹)ₙ-NR⁸R⁹, -(Alk¹)ₙ-S(O)ᵣR⁷, -(Alk¹)ₙ-CN, -(Alk¹)-OH, -(Alk¹)ₙ-COR⁷; or -(Alk¹)ₙ-OR⁷;
   wherein
   Alk¹ is lower alkylene, lower alkenylene or lower alkynylene,
   n is 0 or 1,
   r is 0, 1 or 2,
   R⁷ is -Alk¹-H, -(Alk¹)ₙ-Ar¹ or lower cycloalkyl,
   R⁸ and R⁹ are independently hydrogen, -Alk¹-H or lower cycloalkyl,
   Ar¹ is a homocyclic aryl group of 6 to 14 carbons;
R⁴ is,
   hydrogen, -Alk¹-H, lower cycloalkyl, lower cycloalkyl-lower alkyl, -(Alk¹)ₙ-S(O)ᵣR⁷, -(Alk¹)ₙ-phthalimidyl, -(Alk¹)-CO₂H, -(Alk¹)ₙ-CO₂R⁷, -(Alk¹)ₙ-COR⁷, -(Alk¹)ₙ-Ar¹, -(Alk¹)ₙ-CONR⁸R⁹, -(Alk¹)ₙ-NR⁸R⁹, -(Alk¹)ₙ-OH or -(Alk¹)ₙ-OR⁷;
X is, 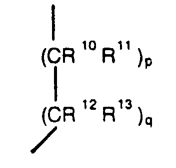 wherein
R¹⁰, R¹¹, R¹² and R¹³ are independently hydrogen or lower alkyl, p and q are independently either 0 or 1;
   and,
   i) Y is hydrogen or hydroxy and
      Z is -(Alk²)ₙ-COR⁵, -(Alk²)ₙ-CO₂R⁵, -(Alk²)ₙ-COSR⁵,
      -(Alk²)ₙ-CONR¹⁴R¹⁵,-(Alk²)-OCO₂R⁵,-(Alk²)-OCOR⁵,
      -(Alk²)-OCONR¹⁴R¹⁵, -(Alk²)-OR⁵, -(Alk²)-NR^{5'}COR⁵, -(Alk²)-NR^{5'}CO₂R⁵, -(Alk²)-NR⁵CONR¹⁴R¹⁵, -(Alk²)ₙ-CONR⁵NR¹⁴R¹⁵, -(Alk²)ₙ-CONR⁵CONR¹⁴R¹⁵, -(Alk²)-NR⁵CSNR¹⁴R¹⁵ or -(Alk²)ₙ-CONR⁵CSNR¹⁴R¹⁵;
      wherein
      Alk² is (C₁₋₁₂) alkylene, (C₂₋₁₂) alkenylene or (C₂₋₁₂) alkynylene, R⁵ and R^{5'} are independently hydrogen, -Alk¹-H (optionally substituted independently with one or more CO₂H, CO₂R⁷, Ar², Ar³ or cyano groups) -(Alk¹)ₙ-(lower cycloalkyl (optionally substituted independently with one or more -Alk¹-H groups)), adamantyl, norbornyl, Ar², Ar³, (lower cycloalkyl)-Ar² or (lower cycloalkyl)-Ar³;
         wherein
      Ar² is a homocyclic aromatic group of 6 to 14 carbon ring atoms (optionally substituted independently with one or more -Alk²-H (optionally substituted independently with one or more halogens), -(Alk¹)ₙ-OH, -(Alk¹)ₙ-OR¹⁶, -(Alk¹)ₙ-Ar³, -(Alk¹)ₙ-CO₂H, -(Alk¹)ₙ-CO₂R⁷, S(O)ᵣR⁷, NR⁸S(O)ᵣR¹⁶, NR⁸R⁹, CONR⁸R⁹, lower cycloalkyl, lower alkoxy, -(Alk¹)ₙ-Ar¹, methylenedioxy, ethylenedioxy, morpholino, thiomorpholino, cyano, nitro or halogens);
         wherein
      R¹⁶ is -Alk¹-H (optionally substituted independently with one or more halogens), lower cycloalkyl (optionally substituted independently with one or more halogens, or -Alk¹-H (optionally substituted independently with one or more halogens)) or -(Alk¹)ₙ-Ar¹ (wherein Ar¹ is optionally substituted independently with one or more, lower alkoxy, cyano groups, halogens or -Alk¹-H (optionally substituted independently with one or more halogens));
      Ar³ is an aromatic group of 5 to 14 ring atoms, at least one of which is O, N or S, (optionally substituted independently with one or more -Alk¹-H (optionally substituted independently with one or more halogens), lower cycloalkyl, lower alkoxy, CO₂H, CO₂R⁷, -(Alk¹)ₙ-Ar¹, cyano or halogen);
      R¹⁴ and R¹⁵ are,
         a) independently, hydroxy, hydrogen, -Alk²-H, lower cycloalkyl (optionally substituted independently with one or more cyano, R¹⁶, Ar², Ar³), lower alkoxy, -(Alk¹)ₙ-adamantyl, -(Alk¹)ₙ-myrantyl, -(Alk¹)ₙ-norbornyl, -(Alk¹)ₙ-fluorenyl, -(Alk¹)ₙ-indanyl, -Alk¹-H (optionally substituted independently with one or more, halogens, cyano, cycloalkyl, SR⁵, COR⁵, CONR⁵R⁷, NR^{5'}COR⁵, NR^{5'}CO₂R⁵, NR^{5'}CONHR⁵, CO₂R⁵, OR⁵, Ar² or Ar³), Ar² or Ar³;
         b) alkylene groups (optionally substituted with one or more R⁷ groups, taken together with the linking nitrogen to form a 4 to 8 atom heterocyclic group) 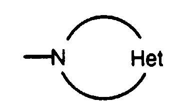 wherein;
      Het represents -O-, -CH₂-, -S(O)ᵣ-, -(NH)- or

         -(N(Alk¹-H))-;

         with the proviso that
         when Z is
            -(Alk²)ₙ-COR⁵, -(Alk²)ₙ-CO₂R⁵ or -(Alk²)ₙ-CO-thiopyridyl and
         R⁵ is
            hydrogen, -Alk¹-H, lower cycloalkyl, -(Alk¹)ₙ-Ar¹, adamantyl or
         when Z is

            -(Alk²)ₙ-CONR¹⁴R¹⁵

            and
      R¹⁴ and R¹⁵ are
         a) independently hydrogen, -Alk²-H, lower cycloalkyl, lower alkoxy, adamantyl, -Ar¹, benzyl, diphenylmethyl, triphenylmethyl or -(Alk¹)ₙ-norbornyl; or
         b) carbon atoms, optionally substituted with one or more lower alkyl groups, taken together with the linking nitrogen to form a 4 to 8 atom heterocylic ring as herein before defined,
      Y is hydroxy; or
   ii) Y is hydrogen and
      Z is
         OR⁵, OCOR⁵, OCONR¹⁴R¹⁵, NR^{5'}COR⁵, NR^{5'}CO₂R⁵, NR⁵CONR¹⁴R¹⁵ or NR⁵CSR¹⁴R¹⁵; and
   iii) Y and Z taken together are
      =O, =CH-(Alk¹)ₙ-COR⁵, =CH-(Alk¹)ₙ-CO₂R⁵ or =CH-(Alk¹)ₙ-CONR¹⁴R¹⁵ ;
      R⁶ is,
         hydrogen or methyl;
   and pharmaceutically acceptable salts thereof.

A subgroup of the compounds of formula (I) are the compounds wherein:
R¹ and R² are,
   i) independently hydrogen or lower alkyl and the bond between the carbons bearing R¹ and R² is a single or a double bond, or
   ii) taken together are a -CH₂- group to form a cyclopropane ring, and the bond between the carbons bearing R¹ and R² is a single bond;
R³ is
   hydrogen, -Alk¹-H, -Alk¹-H substituted with one or more halogens, lower cycloalkyl, lower cycloalkyl-lower alkyl,
   halogen,-(Alk¹)ₙ-CO₂H, -(Alk¹)ₙ-CO₂R⁷, -(Alk¹)ₙ-Ar¹, -(Alk¹)ₙ-CONR⁸R⁹, -(Alk¹)ₙ-NR⁸R⁹, -(Alk¹)ₙ-S(O)ᵣR⁷,
   -(Alk¹)ₙ-CN, -(Alk¹)-OH or -(Alk¹)ₙ-OR⁷;
   wherein
   Alk¹ is lower alkylene, lower alkenylene or lower alkynylene,
   n is 0 or 1,
   r is 0, 1 or 2,
   R⁷ is -Alk¹-H, -(Alk¹)ₙ-Ar¹ or lower cycloalkyl,
   R⁸ and R⁹ are independently hydrogen, -Alk¹-H or lower cycloalkyl,
   Ar¹ is a homocyclic aryl group of 6 to 14 carbons;
R⁴ is
   hydrogen, -Alk¹-H, lower cycloalkyl, lower cycloalkyl-lower alkyl, -(Alk¹)ₙ-S(O)ᵣR⁷, -(Alk¹)ₙ-phthalimidyl, (-Alk¹-)CO₂H, -(Alk¹)ₙ-CO₂R⁷, -(Alk¹)ₙ-Ar¹, -(Alk¹)ₙ-CONR⁸R⁹, -(Alk¹)ₙ-NR⁸R⁹, -(Alk¹)ₙ-OH or -(Alk¹)ₙ-OR⁷;
X is, 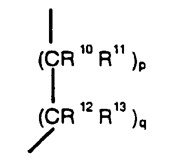 wherein
R¹⁰, R¹¹, R¹² and R¹³ are independently hydrogen or lower alkyl, p and q are independently either 0 or 1;
Y and Z are,
   i) Y is hydrogen or hydroxy and
      Z is -(Alk²)ₙ-COR⁵, -(Alk²)ₙ-CO₂R⁵, -(Alk²)ₙ-COSR⁵,
         -(Alk²)ₙ-CONR¹⁴R¹⁵, -(Alk²)-OCOR⁵, -(Alk²)-OCONR¹⁴R¹⁵, -(Alk²)-OR⁵, -(Alk²)-NR⁵COR⁵, -(Alk²)-NR⁵CO₂R⁵, -(Alk²)-NR⁵CONR¹⁴R¹⁵, -(Alk²)-ₙCONR⁵NR¹⁴R¹⁵, -(Alk²)ₙ-CONR⁵CONR¹⁴R¹⁵, (-Alk²-)NR⁵CSNR¹⁴R¹⁵ or -(Alk²)ₙ-CONR⁵CSNR¹⁴R¹⁵;
         wherein
      Alk² is (C₁₋₁₂) alkylene, (C₂₋₁₂) alkenylene or (C₂₋₁₂ alkynylene, R⁵ is hydrogen, -Alk¹-H, -(Alk¹)ₙ-(lower cycloalkyl), adamantyl, -(Alk¹)ₙ-Ar², -(Alk¹)ₙ-Ar³, (lower cycloalkyl)ₙAr², (lower cycloalkyl)ₙAr³ or -Alk¹- substituted independently with one or more CO₂H, CO₂R⁷, Ar² or Ar³ groups;
         wherein
      Ar² is an aromatic group of 6 to 14 carbon ring atoms, optionally substituted with one or more Alk¹, Alk¹ substituted with one or more halogens, -(Alk¹)ₙ-OH, -(Alk¹)ₙ-OR⁷, -(Alk¹)ₙ-CO₂H, -(Alk¹)ₙ-CO₂R⁷, S(O)ᵣR⁷ or NR⁸R⁹, lower cycloalkyl, lower alkoxy, -(Alk¹)ₙ-Ar¹, methylenedioxy, ethylenedioxy, morpholino, thiomorpholino, cyano or halogen groups;
      Ar³ is an aromatic group of 5 to 14 ring atoms, at least one of which is O, N or S, optionally substituted with one or more Alk¹, lower cycloalkyl, lower alkoxy, CO₂H, CO₂R⁷, -(Alk¹)ₙ-Ar¹, cyano or halogen groups;
      R¹⁴ and R¹⁵ are,
         a) independently, hydrogen or -Alk²-H, lower cycloalkyl, lower alkoxy, adamantyl, -(Alk¹)ₙ-norbornyl, Ar², Ar³, -Alk¹- substituted independently with one or more, SR⁵, COR⁵, CONR⁵R⁷, NR⁵COR⁵, NR⁵CO₂R⁵, NR⁵CONHR⁵ CO₂R⁵, OR⁵, Ar² or Ar³ groups, -(Alk¹)ₙ-fluorenyl, or -(Alk¹)ₙ-indanyl; or
         b) carbon atoms, optionally substituted with one or more lower alkyl groups, taken together with the linking nitrogen to form a 4 to 8 atom heterocyclic group 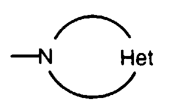 wherein;
         Het represents -O-, -CH₂-, -S(O)ᵣ-, -(NH)- or -(N(Alk¹))-;
         with the proviso that when Z is (-Alk²-)ₙCOR⁵, (-Alk²-)ₙCO₂R⁵ or -(Alk²)ₙ-CO-thiopyridyl and R⁵ is hydrogen, -Alk¹-H, lower cycloalkyl, -(Alk¹)ₙ-Ar¹, adamantyl or
         when Z is (-Alk²-)ₙCONR¹⁴R¹⁵ and R¹⁴ and R¹⁵ are
         (a) independently hydrogen, -Alk²-H, lower cycloalkyl, lower alkoxy, adamantyl, -Ar¹, benzyl, diphenylmethyl, triphenylmethyl or -(Alk¹)ₙ-norbornyl; or
         (b) carbon atoms, optionally substituted with one or more lower alkyl groups, taken together with the linking nitrogen to form a 4 to 8 atom heterocylic ring as herein before defined,
      Y is hydroxy;
   ii) Y is hydrogen and
      Z is OR⁵, OCOR⁵, OCONR¹⁴R¹⁵, NR⁵COR⁵, NR⁵CO₂R⁵, NR⁵CONR¹⁴R¹⁵ or NR⁵CSR¹⁴R¹⁵;
   iii) Y and Z taken together are =O, =CH-(Alk¹)ₙ-COR⁵,
      =CH-(Alk¹)ₙ-CO₂R⁵ or =CH-(Alk¹)ₙ-CONR¹⁴R¹⁵;
   R⁶ is hydrogen or methyl;
      and pharmaceutically acceptable salts thereof.

Particular groups of compounds of formula (I) are the compounds of formulas (IA), (IB), (IC) and (ID)

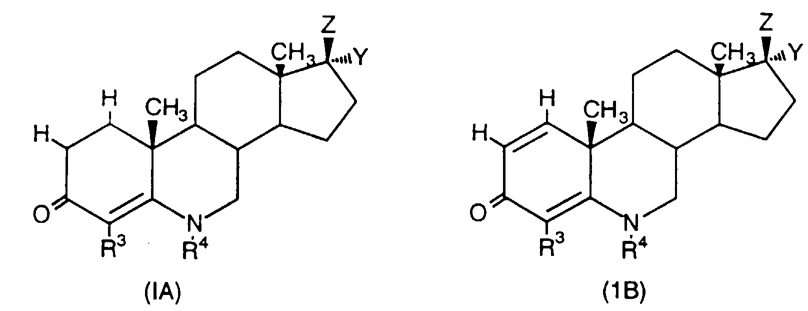

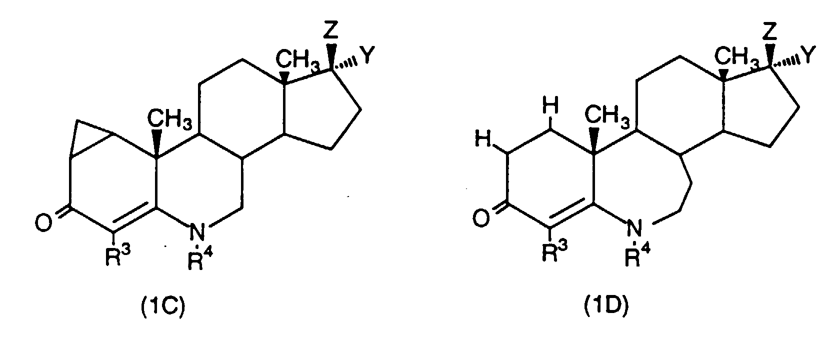

Compounds of formula (IA) are especially of interest.

In an alternative aspect the invention provides compounds of formula (I) wherein Z is -COR⁵ including:
17β-(1-Oxo-2-cyclohexylethyl)-6-azaandrost-4-en-3-one
17β-(1-Oxo-1-(2,4-difluorophenyl)methyl)-6-azaandrost-4-en-3-one
17β-(1-Oxo-1-(4-isopropoxyphenyl)methyl)-6-azaandrost-4-en-3-one
17β-(1-Oxo-3,3-diphenylpropyl)-6-azaandrost-4-en-3-one
17β-(1-Oxo-1-(2-norbornyl)methyl)-6-azaandrost-4-en-3-one

A particular aspect of the invention provides compounds of formula (I) wherein Z is -CONR¹⁴R¹⁵, R¹⁴ is hydrogen and R¹⁵ is Ar² or C₃₋₁₁ saturated ring, optionally containing an oxygen or a sulfur atom, (optionally substituted independently with one or more R⁷ or Ar² groups). Preferably R¹⁵ is a group of formula Ar^{2a} 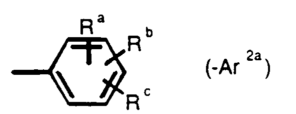 wherein R^{a} and R^{b} are independently hydrogen, lower alkyl, trifluoromethyl, halogen or phenyl (optionally substituted with one or more halogens or branchedC₄₋₇ alkyl) and R^{c} is hydrogen or one or more halogens, or R¹⁵ is a C₃₋₁₁ saturated ring, optionally containing an oxygen or a sulfur atom, substituted with a group of formula Ar^{2a}. In a particular group of compounds Ar^{2a} is a group of formula Ar^{2aa} 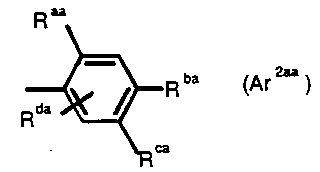 where R^{aa} is branchedC₄₋₇ alkyl, trifluoromethyl or phenyl optionally substituted with one or more halogens; one of R^{ba} and R^{ca} is branched C₄₋₇alkyl, trifluoromethyl, halogen or phenyl optionally substituted with one or more halogens, and the other is hydrogen or halogen; and R^{da} is hydrogen or halogen.

Particular compounds of the invention include :
17β-N-((2,6-Di-*i*-propyl)phenyl)-carbamoyl-6-azaandrost-4-en-3-one
17β-N-(2,4,6-trimethyl)phenyl-carbamoyl-6-azaandrost-4-en-3-one
17β-N-(2-Chloro-5-trifluoromethyl)phenyl-carbamoyl-6-azaandrost-4-en-3-one
17β-N-(2,6-Dimethyl-4-bromo)phenyl-carbamoyl-6-azaandrost-4-en-3-one
17β-N-(2,6-Dimethyl-4-*t*-butyl)phenyl-carbamoyl-6-azaandrost-4-en-3-one
17β-N-(2,6-Dibromo-4-isopropyl)phenyl-carbamoyl-6-azaandrost-4-en-3-one
17β-N-(2,5-Ditrifluoromethyl)phenyl-carbamoyl-6-azaandrost-4-en-3-one
17β-N-(2-Phenyl)phenyl-carbamoyl-6-azaandrost-4-en-3-one
17β-N-(2,6-Diethyl-3,5-dichloro)phenyl-carbamoyl-6-azaandrost-4-en-3-one
17β-N-(2,6-Diethyl-3-chloro)phenyl-carbamoyl-6-azaandrost-4-en-3-one
17β-N-(2-*t*-Butyl)phenyl-carbamoyl-6-azaandrost-4-en-3-one
17β-N-(2,4,6-Trichloro)phenyl-carbamoyl-6-azaandrost-4-en-3-one
17β-N-(2-Bromo-5-trifluoromethyl)phenyl-carbamoyl-6-azaandrost-4-en-3-one
17β-N-(2-*t*-Butyl-6-methyl)phenyl-carbamoyl-6-azaandrost-4-en-3-one
17β-N-1-(4-Chlorophenyl)cyclopentyl-carbamoyl-6-azaandrost-4-en-3-one
17β-N-(2,6-Dibromo-4-chloro)phenyl-carbamoyl-6-azaandrost-4-en-3-one
17β-N-(2,6-Diethyl-4-bromo)phenyl-carbamoyl-6-azaandrost-4-en-3-one
17β-N-(2-Bromo-4-*t*-butyl)phenyl-carbamoyl-6-azaandrost-4-en-3-one
17β-N-(2-Chloro-4-*t*-butyl)phenyl-carbamoyl-6-azaandrost-4-en-3-one
17β-N-(5-Bromo-2-*t*-butyl)phenyl-carbamoyl-6-azaandrost-4-en-3-one
17β-N-(5-Chloro-2-*t*-butyl)phenyl-carbamoyl-6-azaandrost-4-en-3-one
17β-N-(2,6-Diethyl)phenyl-carbamoyl-6-azaandrost-4-en-3-one
17β-N-(4-Bromo-2-*t*-butyl)phenyl-carbamoyl-6-azaandrost-4-en-3-one
17β-N-(2-*t*-Butyl-5-cyano)phenyl-carbamoyl-6-azaandrost-4-en-3-one
17β-N-(2-(O-4-Tolyl)-5-trifluoromethyl)phenyl-carbamoyl-6-azaandrost-4-en-3-one
17β-N-(2-(O-4-Chlorophenyl)-5-trifluoromethyl)phenyl-carbamoyl-6-azaandrost-4-en-3-one
17β-N-(2-Nitro-4-*t*-butyl)phenyl-carbamoyl-6-azaandrost-4-en-3-one
17β-N-(2-(O-Phenyl)-5-(1,1-dimethyl)propyl)phenyl-carbamoyl-6-azaandrost-4-en-3-one
17β-N-(2-Ethylsulfonyl-5-trifluoromethyl)phenyl-carbamoyl-6-azaandrost-4-en-3-one
17β-N-(3,5-Di-*t*-butyl)phenyl-carbamoyl-6-azaandrost-4-en-3-one
17β-N-(2-*t*-Butyl-5-trifluoromethyl)phenyl-carbamoyl-6-azaandrost-4-en-3-one
17β-N-(2-*t*-Butyl-5-phenyl)phenyl-carbamoyl-6-azaandrost-4-en-3-one
17β-N-(2,6-Di-*i*-propyl)phenyl-carbamoyl-6-azaandrost-1,4-dien-3-one
17β-N-(2,6-Di-*i*-propyl)phenyl-carbamoyl-6-azaandrost-4-methyl-1,4-dien-3-one
17β-N-1-(4-Trifluoromethylphenyl)cyclopentyl-carbamoyl-6-azaandrost-4-en-3-one
17β-N-1-(4-Fluorophenyl)cyclohexyl-carbamoyl-6-azaandrost-4-en-3-one
17β-N-1-(4-Methoxyphenyl)cyclohexyl-carbamoyl-6-azaandrost-4-en-3-one
17β-N-1-(4-Methoxyphenyl)cyclopentyl-carbamoyl-6-azaandrost-4-en-3-one
17β-N-(2,5-bis(Trifluoromethyl))phenyl-carbamoyl-6-azaandrost-4-methyl-4-en-3-one
17β-N-(2-t-Butyl-5-trifluoromethyl)phenyl-carbamoyl-6-azaandrost-4-methyl-4-en-3-one
17β-N-(2,5-Di-t-butyl)phenyl-carbamoyl-6-azaandrost-4-methyl-4-en-3-one
17β-N-(2-t-Butyl-5-(4-chlorophenyl))phenyl-carbamoyl-6-azaandrost-4-en-3-one
17β-N-1-(4-t-Butylphenyl)cyclopentyl-carbamoyl-6-azaandrost-4-en-3-one
17β-N-1-(4-t-Butylphenyl)cyclohexyl-carbamoyl-6-azaandrost-4-en-3-one
17β-N-1-(4-Chlorophenyl)cyclopentyl-carbamoyl-6-azaandrost-4-methyl-4-en-3-one
17β-N-(2,5-bis(Trifluoromethyl))phenyl-carbamoyl-6-azaandrost-4-chloro-4-en-3-one
17β-N-(2-*t*-Butyl-5-trifluoromethyl)phenyl-carbamoyl-6-azaandrost-4-chloro-4-en-3-one
17β-N-1-(4-*t*-Butylphenyl)cycloheptyl-carbamoyl-6-azaandrost-4-en-3-one
17β-N-1-(4-*t*-Butylphenyl)cyclohexyl-carbamoyl-6-azaandrost-4-chloro-4-en-3-one
17β-N-(2,6-Diethyl-4-(4-chlorophenyl))phenyl-carbamoyl-6-azaandrost-4-en-3-one
17β-N-4-(4-*t*-Butylphenyl)tetrahydrothiopyranyl-carbamoyl-6-azaandrost-4-en-3-one
17β-N-9-(4-*t*-Butylphenyl)bicyclo[3.3.1]nonyl-carbamoyl-6-azaandrost-4-en-3-one
17β-N-4-(4-*t*-Butylphenyl)tetrahydropyranyl-carbamoyl-6-azaandrost-4-en-3-one
17β-N-1-(4-Chlorophenyl)cyclohexyl-carbamoyl-6-azaandrost-4-en-3-one
17β-N-(2-*t*-Butyl-5-(4-*t*-butyl)phenyl)phenyl-carbamoyl-6-azaandrost-4-en-3-one or
17β-N-1-(4-Chlorophenyl)cyclopentyl-carbamoyl-6-azaandrost-4-chloro-4-en-en-3-one
and pharmaceutically acceptable salts thereof.

A particular compound is 17β-N-(2-*t*-Butyl-5-trifluoromethyl)phenyl-carbamoyl-6-azaandrost-4-en-3-one and pharmaceutically acceptable salts thereof.

One particular aspect of the invention provides compounds of formula (I) wherein R¹ and R² are as hereinbefore defined, R³ is hydrogen or lower alkyl;
R⁴ is hydrogen or lower alkyl;
Y is hydrogen; and
Z is -COR⁵, -CONR¹⁴R¹⁵, -CH₂OCO₂R⁵, -CH₂OCOR⁵, -CH₂NR^{5'}COR⁵, -CONR⁵NR¹⁴R¹⁵, -CH₂NR⁵CONR¹⁴R⁵, or -CON⁵CONR¹⁴R¹⁵ wherein R⁵ and R^{5'} are independently hydrogen, lower alkyl optionally substituted independently with one or more Ar² groups (lower alkyl)ₙ-lower cycloalkyl, menthyl, adamantyl, norbornyl or Ar²;
Ar² is a phenyl group optionally substituted independently with one or more -Alk-H (optionally substituted independently with one or more halogens), -OR¹⁶, -S(O)ᵣR⁷, phenyl, methylenedioxy, ethylenedioxy, morpholino, thiomorpholino, cyano, nitro or halogen groups;
Alk is lower alkylene, lower alkenylene or lower alkynylene;
n is 0 or 1;
r is 0, 1 or 2;
R⁷ is -Alk-H, -(Alk)ₙ-phenyl or lower cycloalkyl;
R¹⁶ is -Alk-H (optionally substituted independently with one or more halogens), lower cycloalkyl (optionally substituted independently with one or more halogens or -Alk-H (optionally substituted independently with one or more halogens)) or -(Alk)ₙ-phenyl (wherein phenyl is optionally substituted independently with one or more lower alkoxy, cyano, halogen or -Alk-H groups (optionally substituted independently with one or more halogens)); R¹⁴ is hydrogen or hydroxy, and R¹⁵ is hydrogen, lower cycloalkyl (optionally substituted independently with one or more R⁷ or Ar² groups), -(Alk)-adamantyl, -(Alk)ₙ-myrantyl, -(Alk)ₙ-norbornyl, -(Alk)ₙ-fluorenyl, -(Alk)ₙ-indanyl, -Alk-H (optionally substituted independently with one or more lower cycloalkyl, SR⁵, OR⁵, Ar² or Ar³ groups), Ar² or Ar³, or R¹⁴ and R¹⁵ taken together with the linking nitrogen form a pyrrolidinyl, piperidinyl or perhydroazepinyl ring optionally substituted with one or more R⁷ groups; Ar³ is a pyrrolyl, thienyl, furyl or pyridyl group optionally substituted independently with one or more Alk-H groups; with the provisos that
when Z is COR⁵, R⁵ is substituted lower alkyl, lower alkyl lower cycloalkyl, menthyl, norbornyl or Ar²;
when Z is CONR¹⁴R¹⁵ and R¹⁵ is hydrogen, -Alk-H, lower cycloalkyl, lower alkoxy, adamantyl, phenyl, benzyl, diphenylmethyl, triphenylmethyl or -(Alk)ₙ-norbornyl, R¹⁴ is hydroxy; and
when Z is CONR¹⁴R¹⁵ and R¹⁴ and R¹⁵ taken together with the linking nitrogen form a pyrrolidinyl, piperidinyl or perhydroazepinyl ring, said ring is substituted with one or more lower alkenyl, lower alkynyl, -(Alk)ₙ-phenyl or lower cycloalkyl groups; and pharmaceutically acceptable salts thereof. Compounds of formula (IA) are especially of interest. In a still further aspect of the invention of partiucalr interest, Z is CONR¹⁴R¹⁵.

Some of the substituents of the compound of formula (I) may cause asymmetry about the atoms to which they are attached giving rise to either α or β stereochemical configuration. (For a detailed explanation of stereochemical configuration see March, *J*. *Advanced Organic Chemistry,* 3rd Ed., ch 4, John Wiley & Sons, New York (1985).) Unless otherwise indicated, either the α and β stereo configurations are intended for the substituents.

The compounds of formula (I) can be used in the form of an acid addition salt derived from inorganic or organic acids. Where the salt of a compound of formula (I) is to be used for a human or veterinary medicinal application the salt must be pharmaceutically acceptable. However, non-pharmaceutically acceptable salts of the compounds of formula (I) may be useful as intermediates in the preparation of a corresponding pharmaceutically acceptable salt. Pharmaceutically acceptable salts include, but are not limited to, salts with inorganic acids such as hydrochloride, sulfate, phosphate, diphosphate, hydrobromide and nitrate salts or salts with an organic acid such as the acetate, malate, maleate, fumarate, tartrate, succinate, citrate, lactate, methanesulfonate, p-toluenesulfonate, palmitoate, salicylate and stearate salts.

### Preparation of Compounds

According to one general process (A) the compounds of the present invention may be prepared by the procedure shown in step 8 of Scheme I, wherein R¹- R⁴, R⁶, Y and Z are as defined for formula (I) and "JO" is a protected hydroxy group:

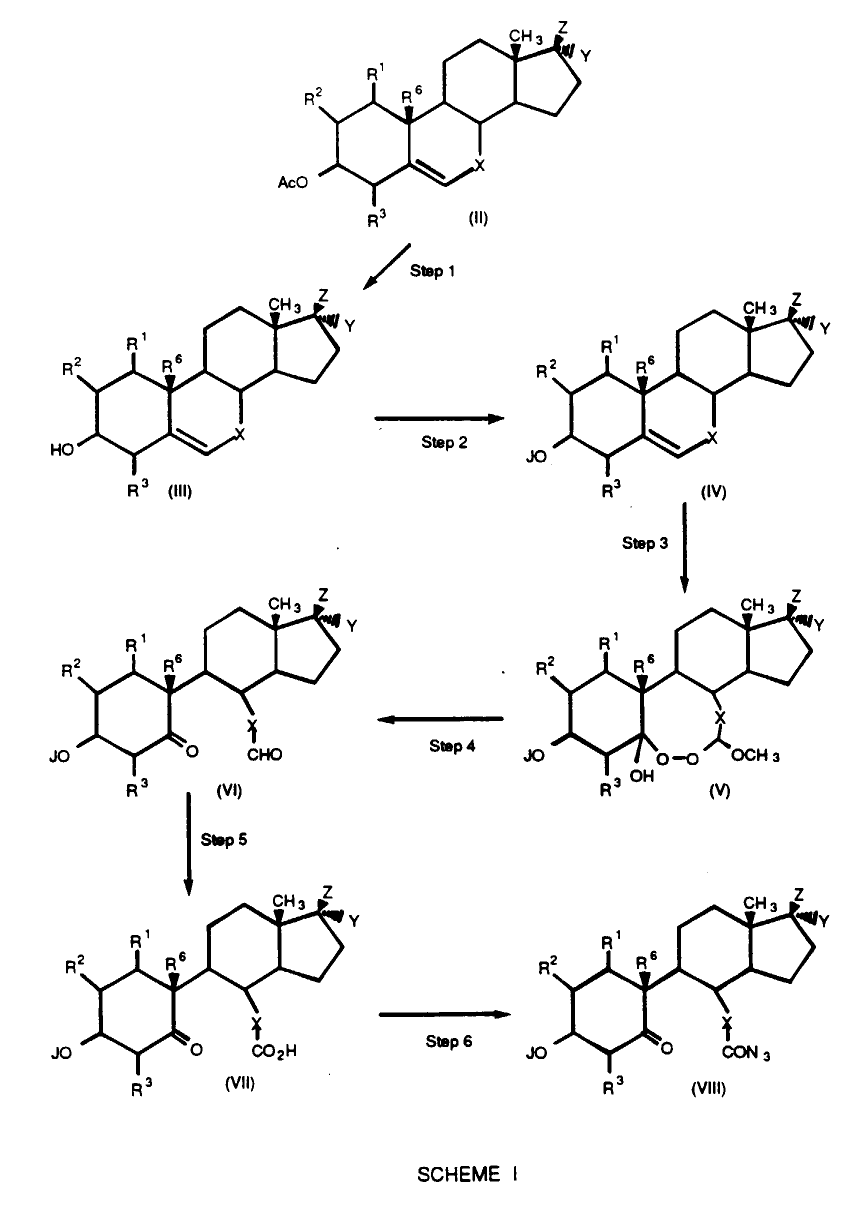

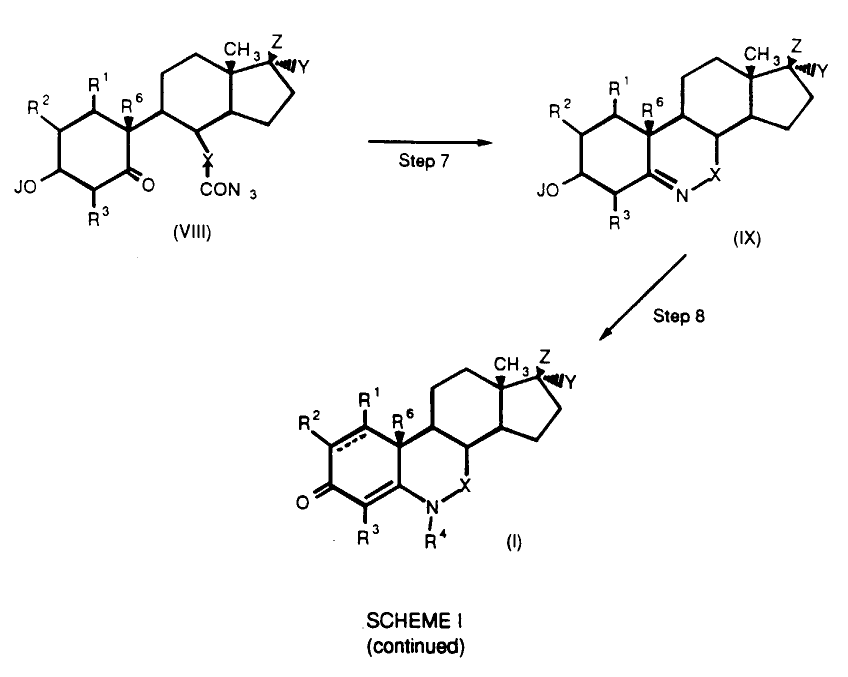

In Step 1 of Scheme I when Z is CO₂H, the acid group at the 17 position of a compound of formula (II) is converted to the corresponding ketone, ester or amide of compound (III) accompanied by deprotection of the hydroxy group at the 3 position. Alternatively a compound of formula (III) wherein Z is CO₂CH₃ and Y is H may be prepared from pregnenolone as described by Rasmusson, et at., *J. Med. Chem.,* **27**, 1690 (1984).

This may be accomplished by activating the carboxylic acid group toward nucleophilic displacement by treatment with an activating agent such as N,N-bis(2-oxo-3-oxazolidinyl)phosphorinic chloride (BOP-CI) or conversion to the corresponding acid halide group by treatment with a halogenating agent such as oxalyl chloride or thionyl chloride in an aprotic solvent such methylene chloride or toluene at -5 to 10°C. The intermediate activated carboxylic acid, e.g., an acid chloride, may be reacted with H-NR¹⁴R¹⁵ or HOR⁵ (wherein R⁵, R¹⁴ and R¹⁵ are as defined for formula (I)) at or above room temperature in an aprotic solvent. When R⁵ is alkyl, alkenyl, lower cycloalkyl, or adamantyl, the activated acid is treated with R⁵M (wherein M is a metal, such as magnesium or lithium) in a polar, aprotic solvent such as THF or diethyl ether containing catalytic Cut, at a temperature in the range of about 0 to about -78°C.

Additionally, when, H-NR¹⁴R¹⁵ is a hindered, nonnucleophilic aniline the corresponding metal salt may be prepared and the activated acid treated with M-NR¹⁴R¹⁵ (wherein M is a metal, such as magnesium or lithium) in a polar, aprotic solvent such as THF or diethyl ether at a temperature in the range of about 0 to about -78°C.

The amines, H-NR¹⁴R¹⁵ are commercially available or conveniently prepared by methods known in the art. For example, when either R¹⁴ or R¹⁵ is an alkyl or aryl substituted aromatic residue the alkyl group may be introduced as described by Reetz, M.T. *et al., Angew. Chem. Int. Ed. Engl.,* **19**, 900 and 901 (1980) or the aryl group introduced as described by Stille, J.K. *Pure Appl. Chem.,* **57**, 1771 (1985). When either R¹⁴ or R¹⁵ is an aryl substituted cycloalkyl residue the amine may be prepared by Curtius rearrangement of the corresponding acid, where available, or by the method of He, X. *et al., J. Med. Chem.,* **36**,1188 (1993), *i.e.* by reacting the corresponding cycloalkanone with the appropriate aryl Grignard reagent followed by conversion of the resulting alcohol to the amine by treatment with sodium azide and trifluoroacetic acid followed by reduction of the azide with lithium aluminum hydride. Aryl substituted cyclopropylamines are prepared by rhodium catalyzed insertion of the appropriate aryl-α-diazo-ester (prepared by the method of Baum, J.S. *et al., Synthetic Comm.,* **17***,* 1709 (1987)) into the appropriate olefin (as described by Davies, H.W. *et al., Tetrahedron Lett.,* **30**, 5057 (1989)) followed by saponification of the ester and Curtius rearrangement of the acid to give the desired amine.

In Step 2, a compound of formula (III) is treated with a suitable hydroxy protecting group such as for example a silicon derivative such as a trisubstituted silyl halide, a tetrahydropyran derivative or an arylalkyl group such as a paramethoxybenzyl group. Typically the compound of formula (III) is treated with a trialkylsilyl halide, e.g., triisopropylsilyl chloride, at about 25 to 75°C in an aprotic solvent such as dimethylformamide to protect the hydroxy group in the 3-position to yield the corresponding trisubstituted silylated compound of formula (IV).

In Step 3, a compound of formula (IV) is treated with ozone in methanol alone or as a mixture with one or more polar, protic or aprotic solvents, e.g., methylene chloride and methanol, at a temperature substantially below 0°C, e.g., from about -50 to about -80°C to yield a corresponding compound of formula (V).

In Step 4, the compound of formula (V) in methanol alone or as a mixture with one or more polar, protic or aprotic solvents, e.g,, methylene chloride and methanol, at about -20°C is treated with a reductant such as zinc and acetic acid then allowed to slowly warm to room temperature to yield the aldehyde of formula (VI). Alternatively the compound of formula (V) may be taken directly to step 5.

In Step 5, a compound of formula (V or VI) is reacted with an oxidant, such as Jones reagent (see Bowden, et al., *J*. *Chem. Soc.,* **39**, (1946)) at about 0°C, to yield the corresponding compound of formula (VII).

In Step 6, a compound of formula (VII) is converted to an activated carboxylate derivative such as an acid halide, e.g., chloride, by treatment with a halogenating agent, e.g., oxalyl chloride. The resulting acid halide is reacted with an alkali metal azide, e.g., sodium azide, at about 0 to 30°C in an aqueous solvent mixture, such as water and acetone, to yield the corresponding acyl azide compound of formula (VIII). Alternatively, the acid is treated with triphenyl phosphoryl azide in an aprotic solvent such as toluene to yield the acyl azide directly.

In Step 7, an acyl azide compound of formula (VIII) is rearranged with ring closure by warming to reflux in an aprotic solvent, such as toluene, to induce rearrangment to the corresponding isocyanate followed by stirring with a weak acid such as silica get or by reaction with a strong, sterically hindered base, e.g., potassium *t*-butoxide, in a protic or aprotic solvent at a temperature in the range of about 90 to about 180°C to generate the corresponding compound of formula (IX).

Finally, in Step 8 (general process A), the hydroxy function of a compound of formula (IX) is deprotected and oxidized. Thus, in general process (A1) compounds of formula (I) wherein R⁴ is hydrogen may be prepared by converting the protected hydroxy group of a compound of formula (IX) to the corresponding hydroxy group, i.e., the hydroxy group is deprotected by conventional means. Thus, for example a trisubstituted silyl group may be removed by reaction with aqueous hydrogen fluoride in a polar solvent such as acetonitrile at about 0°C to room temperature. Next the hydroxy group is oxidized by reaction with a suitable oxidizing agent, for example, with Jones reagent with migration of the double bond to the 4,5 position to generate the corresponding compound of formula (I) where R⁴ is hydrogen.

Alternatively, in general process (A2) for the preparation of compounds of formula (I), wherein R⁴ is an acyl group, the compound of formula (IX) is treated with an acylating agent such as di-*t*-butyldicarbonate to acylate the 6-nitrogen with migration of the double bond to the 4, 5 position. The hydroxy protecting group is then removed in a conventional manner, for example, a trisubstituted silyl protecting group may be removed with a reagent such as tetrabutylammonium fluoride and treated with an oxidant such as pyridinium dichromate or manganese dioxide to generate the corresponding compound of formula (I) where R⁴ is *t*-butoxycarbonyl.

Alternatively, intermediate compounds of formula (IX) may be prepared from the corresponding compounds of formula (VII) according to Scheme 1A.

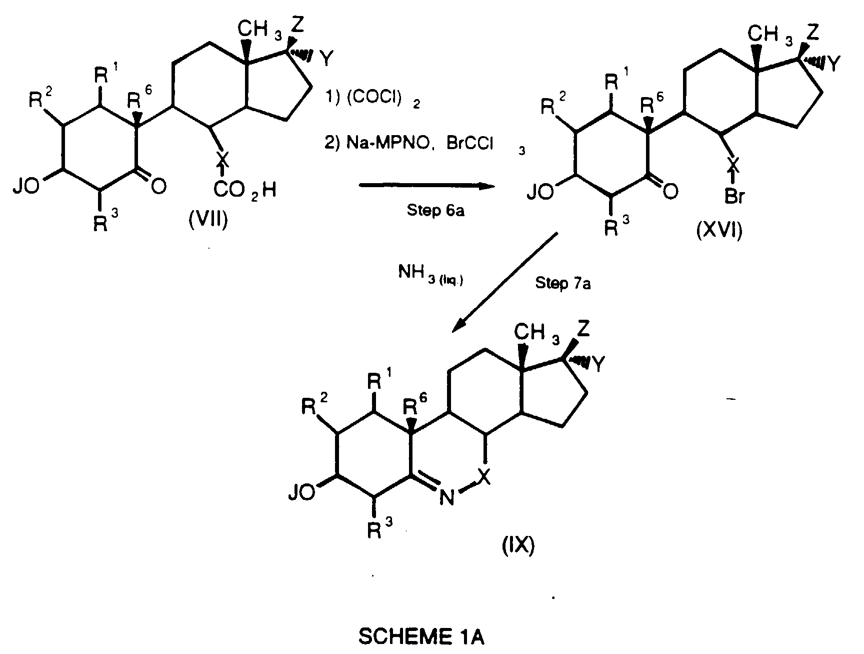

In step 6a of Scheme 1A, a compound of formula (VII) is reacted sequentially with 1) (COCl)₂ in a non polar solvent, *e.g.,* toluene or methylene chloride, in the presence of a tertiary amine, *e.g.,* triethyl amine or pyridine, then 2) with 2-mercaptopyridine N-oxide sodium salt (Na-MPNO) in BrCCl₃ to yield the corresponding compound of formula (XVI). In step 7a the compound of formula (XVI) is reacted with liquid ammonia at about 50°C in the presence of an ammonium salt of a weak acid, *e.g.,* ammonium carbonate, in a pressure vessel to yield the corresponding compound of formula (IX).

Alternatively, according to another general process (B), the compounds of formula (I) wherein X is 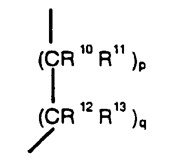 and both p and q are 1, and R¹⁰, R¹¹, R¹² and R¹³ are hydrogen, may be prepared by the procedure shown in Step 5 of Scheme II wherein R¹⁻⁶ are as defined for formula (I):

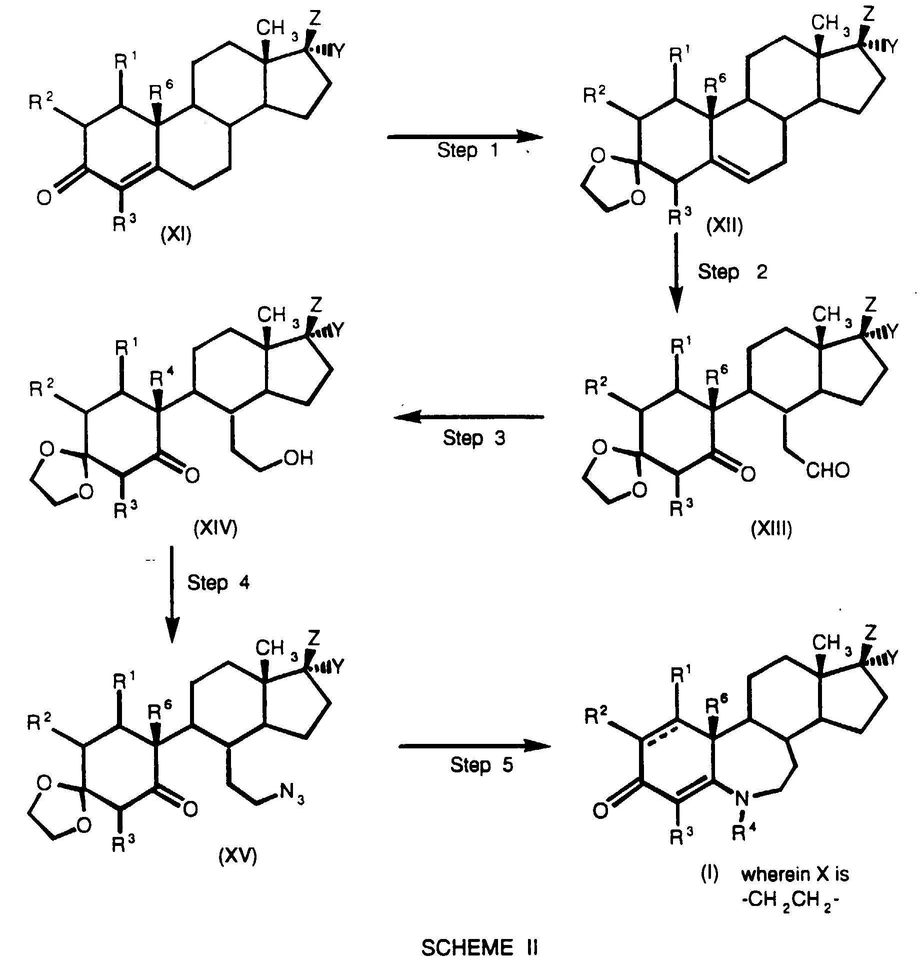

In Step 1 of Scheme II, the enone function of compound (XI) is protected as a ketal with concomitant migration of the double bond to the 5, 6 position by refluxing with ethylene glycol in the presence of an acid, such as p-toluenesulfonic acid, in a solvent such as toluene which allows azeotropic removal of water to yield the corresponding compound of formula (XIII).

In Step 2, a compound of formula (XII) is treated with ozone in methanol alone or with one or more polar, protic or aprotic solvents mixtures, e.g., methylene chloride and methanol, at a temperature substantially below 0°C, e.g., from about -50 to about -80°C, followed by treatment at about -20°C with a reductant, such as zinc and acetic acid, then allowed to slowly warm to room temperature to yield the aldehyde of formula (XIII).

In Step 3, a compound of formula (XIII) is reduced with a selective reducing agent, such as lithium tri-t-butoxyaluminumhydride in an aprotic solvent such as THF or diethyl ether to give the corresponding alcohol of formula (XIV).

In Step 4, the alcohol functionality of a compound of formula (XIV) is converted to a leaving group, such as the corresponding methanesulfonate by treatment with methanesulfonyl chloride in an aprotic solvent such as methylene chloride in the presence of a hindered tertiary amine base such as triethylamine. Once transformed to a leaving group, the alcohol is displaced by treatment with a source of azide, such as sodium azide, in a polar, aprotic solvent, such as DMF, to give the corresponding alkyl azide of formula (XV).

In Step 5, a compound of formula (XIV) is treated with a reductant such as triphenylphosphine in THF at reflux followed by a strong protic acid such as 4M HCI to give the corresponding compound of formula (I) where X is -CH₂CH₂-.

Alternatively, according to another general process (C), a compound of formula (I) may be converted into another compound of formula (I) using conventional procedures. Thus for example, a double bond may then be inserted between the carbon in the 1 position and the carbon in the 2 position by conventional means such as dehydrogenation with 2,3-dichloro-5,6-dicyano-1,4-benzoquinone by refluxing in an aprotic solvent such as dioxane to produce a compound of formula (I) which is unsaturated in the 1, 2 position. A compound of formula (I) with a double bond in the 1, 2 position may then be treated with the anion of trimethylsulfoxonium iodide, prepared by deprotonation with a base such as sodium hydride, in an aprotic, polar solvent such as DMSO to give a compound of formula (I) wherein R¹ and R² taken together form a cyclopropane ring.

Optionally, a compound of formula (I) wherein R³ is H and R⁴ is acyl or acyloxy, such as *t*-butoxycarbonyl, may be treated with bromine at 0°C in an aprotic solvent such as methylene chloride to give the corresponding compound of formula (I) wherein R³ is Br, which may then be treated with an organotin species such as phenyltrimethyltin in the presence of a palladium catalyst such as PdCl₂(PPh₃)₂ and lithium chloride in a polar aprotic solvent such as dimethylformamide to give the corresponding compound of formula (I) wherein R³ is methyl.

Additionally, a compound of formula (I) wherein R⁴ is acyl or acyloxy, such as t-butoxycarbonyl, may be treated with a strong hindered base such as lithium diisopropylamide at -78°C in an aprotic solvent such as THF followed by an electrophile, such as methyl iodida to give compounds of formula (I) wherein R⁴ is methyl or lower alkyl.

Also, a compound of formula (I) wherein R³ is H may be treated with cuprous cyanide or N,N-dimethylmethyleneammonium iodide in polar, aprotic solvents such as DMF or acetonitrile to give compounds of formula (I) wherein R³ is -CN and -CH₂N(CH₃)₂ respectively.

Additionally, a compound of formula (I) wherein R³ is H may be treated with a halogenated succinimide such as N-iodosuccinimide in a solvent such as THF to give a compound of formula (I) wherein R³ is I.

The compounds of formula (I) wherein R⁴ is hydrogen may be reacted, via a nucleophilic reaction of the corresponding sodium or potassium salt, with L-lower alkyl, L-lower alkenyl, L-alkanoyl of 2 to 6 carbons, L-Alk¹, L-lower cycloalkyl, L-lower cycloalkyl-lower alkyl, cycloalkyl, L-(Alk¹)ₙS(O)ᵣR⁷, L-(Alk¹)ₙ-phthalimidyl, L-(Alk¹)-CO₂H, L-(Alk¹)ₙ-CO₂R⁷, L-(Alk¹)ₙ-Ar¹, L-(Alk¹)ₙ-CONR⁸R⁹, L-(Alk¹)ₙ-NR⁸R⁹, L-(Alk¹)ₙ-OH or L-(Alk¹)ₙ-OR⁷ at a temperature of about 5 to about 100°C in a polar, aprotic solvent such as dimethylformamide, to yield the compounds of formula (I) wherein R⁴ is other than hydrogen. The groups, R⁷-R⁹, Ar¹ and n, are as defined for formula (I) and L is a leaving group, such as defined in March, J., *Advanced Organic* *Chemistry,* 3d. Ed., 179, John Wiley & Sons, New York (1985) and in Hendrickson, J, et al., *Organic Chemistry,* 3d. Ed., 375-377, McGraw Hill, New York (1970), e.g., a halogen atom.

Additionally, a compound of formula (I) wherein Z is CO₂R⁵, and in particular wherein R⁵ is CH₃, may be treated with a strong base, such as lithium hydroxide in a solvent system such as THF or dioxane and water to give a compound of formula (I) where Z is CO₂H. An acid of this formula may then be treated as described in Step 1 of Scheme 1 to yield the corresponding compounds of formula (I) wherein Z is COR⁵, CO₂R⁵ or CONR¹⁴R¹⁵.

A particular step for the preparation of compounds of formula (I) wherein Z is -(Alk²)ₙCONR¹⁴R¹⁵ involves reacting a compound of formula (I) wherein Z is (Alk²)ₙCO₂R⁵ with an amine of formula HNR¹⁴R¹⁵, especially compounds wherein R¹⁴ is hydrogen and R¹⁵ is Ar² or lower cycloalkyl (optionally substituted independently with one or more R⁷ or Ar² groups), e.g., a group of formula Ar^{2a} or a cycloalkyl group substituted with a group of formula Ar^{2a}.

Optionally, a compound of formula (I) wherein Z is CO₂R⁵, and in particular wherein R⁵ is CH₃, may be reduced with a reducing agent such as diisobutylaluminum hydride and then reoxidized with Collins' reagent (CrO₃·2 pyridine) or another mild oxidant to produce a compound of formula (I) wherein Z is CHO, which may be treated with R⁵M (wherein M is a metal such as magnesium or lithium) and R⁵ is Alk¹-H (optionally substituted independently with one or more -CO₂H, CO₂R⁷, Ar² or Ar³ groups), lower cycloalkyl (optionally substituted independently with one or more -Alk¹-H groups), adamantyl, Ar², Ar³, -(lower cycloalkyl)ₙ-Ar² or -(lower cycloalkyl)ₙ-Ar³ to give, after oxidation with pyridinium dichromate, a compound of formula (I) wherein Z is COR⁵.

The above product of diisobutylaluminum hydride reduction may also be selectively oxidized at the 3-position by treatment with manganese (II) oxide to give a compound of formula (I) wherein the substitutent on C-17, that is, Z, is CH₂-OH, i.e., a (Alk²)alkanol. This alcohol may then be converted to an amine by standard methods, e.g., conversion to a mesylate, displacement with a metal azide such as sodium azide, and reduction with hydrogen in the presence of a catalyst such as palladium on carbon. The alcohol or amine may then be reacted with acid chlorides, chloroformates, isocyanates or isothiocyanates to give compounds of formula (I) wherein Z is -(Alk²)-OCOR⁵, -(Alk²)-OCO₂R⁵, -(Alk²)-NR^{5'}COR⁵, -(Alk²)-NR^{5'}-CO₂R⁵, -(Alk²)-NR⁵CONR¹⁴R¹⁵, -(Alk²)-NR⁵CSNR¹⁴R¹⁵ or -(Alk²)-OCO-NR¹⁴R¹⁵.

Further, these compounds of formula (I), wherein Z is -CHO, may be treated with a Wittig reagent, such as Et₂OPOCH₂COR⁵ Et₂OPOCH₂CO₂R⁵ or Et₂OPOCH₂CONR¹⁴R¹⁵ to give a compounds of formula (I) wherein Z is -CH=CH-COR⁵, CH=CH-CO₂R⁵ or CH=CH-CONR¹⁴R¹⁵ respectively.

It will be appreciated by those skilled in this art that for certain cases of R³ and Z some Steps in the procedures shown in Scheme I and Scheme II are incompatible with survival of the functional groups of interest. In these cases, R³ or Z is either introduced subsequent to the incompatible Step or is present in a protected form. An example of the former is the case where R³ is halogen, in which case the halogen is introduced by reaction of a compound of formula (I) with a halogenated succinimide, such as N-bromosuccinimide. An example of the latter is the use of an ester or ether to protect a carboxylic acid or alcohol, respectively.

Thus, according to another general process (D), a compound of formula (I) according to the invention, or a salt thereof may be prepared by subjecting a protected derivative of formula (I) or a salt thereof to a reaction to remove the protecting group or groups.

Thus, at an earlier stage in the preparation of a compound of formula (I) or a salt thereof it may have been necessary and/or desirable to protect one or more sensitive groups in the molecule to prevent undesirable side reactions.

The protecting groups used in the preparation of compounds of formula (I) may be used in a conventional manner. See for example Protective Groups in Organic Chemistry, Ed. J.F.W. McOmie, Plenum Press, London (1973) or Protective Groups in Organic Synthesis, Theodora Green, John Wiley and Sons, New York (1981).

Conventional amino protecting groups may include, for example, arylalkyl groups, such as benzyl, diphenylmethyl or triphenylmethyl groups; and acyl groups, such as N-benzyloxycarbonyl or t-butoxycarbonyl. Thus, compounds of formula (I) when R⁴ represents hydrogen may be prepared by deprotection of a corresponding protected compound.

Hydroxy groups may be protected, for example, by benzyl, diphenylmethyl or triphenylmethyl groups, acyl groups, such as acetyl, silicon protecting groups, such as triisopropysilyl or t-butyldimethylsilyl groups, or as tetrahydropyran derivatives.

Removal of any protecting groups present may be achieved by conventional procedures. An arylalkyl group such as benzyl, may be cleaved by hydrogenolysis in the presence of a catalyst, e.g., palladium on charcoal; an acyl group such as N-benzyloxycarbonyl may be removed by hydrolysis with, for example, hydrogen bromide in acetic acid or by reduction, for example by catalytic hydrogenation; silicon protecting groups may be removed, for example, by treatment with fluoride ion or by hydrolysis under acidic conditions; tetrahydropyran groups may be cleaved by hydrolysis under acidic conditions.

As will be appreciated, in any of the general processes (A) to (C) described above it may be desirable or even necessary to protect any sensitive groups in the molecule as just described. Thus, a reaction step involving deprotection of a protected derivative of general formula (I) or a salt thereof may be carried out subsequent to any of the above described processes (A) to (C).

Thus, according to a further aspect of the invention, the following reactions may, if necessary and/or desired be carried out in any appropriate sequence subsequent to any of the processes (A) to (C):
(i) removal of any protecting groups; and
(ii) conversion of a compound of formula (I) or a salt thereof into a pharmaceutical acceptable salt or solvate thereof.

Where it is desired to isolate a compound of the invention as a salt, for example as an acid addition salt, this may be achieved by treating the free base of formula (I) with an appropriate acid, particularly with an equivalent amount, or with creatinine sulfate in a polar, protic solvent, e.g., aqueous ethanol.

As well as being employed as the last main step in the preparative sequence, the general methods indicated above for the preparation of the compounds of the invention may also be used for the introduction of the desired groups at an intermediate stage in the preparation of the required compound. It should therefore be appreciated that in such multi-stage processes, the sequence of reactions should be chosen in order that the reaction conditions do not affect groups present in the molecule which are desired in the final product.

The compound of formula (I) and the intermediate compounds, (II)-(XIV), shown in Schemes I and II may be purified by convenient methods of the art, e.g., chromatography or crystallization.

### Steroid 5α-Reductases

### In Vitro Assay

Enzyme activies may be determined using microsomes derived from: 1) prostate tissue from benign prostatic hyperplasia (BPH) patients; 2) recombinant baculovirus infected SF9 cells that express human type 1 5α-reductase; 3) prostate tissue from the rat; or 4) recombinant baculovirus infected SF9 cells that express human type 2 5α-reductase. Microsomes were prepared by homogenization of the tissue or cells, followed by differential centrifugation of the homogenate. Microsome extracts were incubated with varying concentrations of [1,2,6,7-3H]-testosterone, 1 mM NADPH, and varying amounts of the compounds of Formula I, i.e. a test compound, in buffer containing a NADPH regenerating system capable of maintaining NADPH concentrations for a period of time within the range 0.5-240 minutes. Corresponding incubations were carried out with no test compound as a control study. For type 1 IC₅₀ measurements, assay components except testosterone were preincubated for 10 minutes at pH 7.0, and following the addition of 100nM testosterone the assays were allowed to proceed for 10-120 minutes.

For type 2 IC₅₀ measurements, assay components except testosterone were preincubated for 20 minutes at pH 6.0, and following the addition of 8nM testosterone the assays were allowed to proceed for 20-40 minutes. The percentage of conversion of testosterone to DHT in the presence of test compounds compared to the corresponding conversion in the control study was estimated using high pressure liquid chromatography (HPLC) with radiochemical detection. The results of these assays appear as IC₅₀'s reported in Table 1.

**TABLE 1**

| **5-REDUCTASE *in vitro* INHIBITORY ACTIVITY** | | | |
|---|---|---|---|
| Compound/ Example | IC₅₀ Human Type 1 | IC₅₀ Human Type 2 | IC₅₀ Rat Prostatic |
| 1 | + | +++ | ++ |
| 2 | +++ | +++ | +++ |
| 3 | ++ | ++ | +++ |
| 4 | + | +++ | + |
| 5 | + | ++ | ++ |
| 6 | + | ++ | ++ |
| 7 | + | +++ | ++ |
| 9 | ++ | +++ | +++ |
| 10 | +++ | +++ | ++ |
| 11 | ++ | +++ | +++ |
| 12 | ++ | +++ | +++ |
| 13 | ++ | +++ | +++ |
| 14 | ++ | +++ | +++ |
| 15 | + | +++ | +++ |
| 16 | ++ | +++ | +++ |
| 17 | ++ | +++ | +++ |
| 18 | ++ | +++ | +++ |
| 19 | + | ++ | ++ |
| 20 | + | ++ | ++ |
| 21 | + | ++ | + |
| 22 | ++ | +++ | +++ |
| 23 | ++ | +++ | nt |
| 24 | ++ | +++ | +++ |
| 25 | ++ | +++ | +++ |
| 26 | ++ | +++ | nt |
| 27 | +++ | +++ | ++ |
| 28 | ++ | ++ | +++ |
| 29 | + | ++ | ++ |
| 30 | ++ | +++ | ++ |
| 31 | ++ | +++ | +++ |
| 32 | ++ | +++ | +++ |
| 33 | + | ++ | ++ |
| 34 | ++ | +++ | +++ |
| 35 | + | +++ | +++ |
| 36 | + | +++ | +++ |
| 37 | ++ | +++ | nt |
| 38 | ++ | +++ | + |
| 39 | ++ | +++ | ++ |
| 40 | ++ | +++ | ++ |
| 41 | ++ | +++ | + |
| 42 | ++ | +++ | +++ |
| 43 | +++ | +++ | + |
| 44 | ++ | +++ | + |
| 45 | + | +++ | ++ |
| 46 | ++ | +++ | +++ |
| 47 | ++ | +++ | +++ |
| 48 | +++ | +++ | ++ |
| 49 | +++ | +++ | +++ |
| 50 | +++ | +++ | ++ |
| 51 | + | ++ | + |
| 52 | ++ | +++ | ++ |
| 53 | ++ | +++ | +++ |
| 54 | ++ | +++ | nt |
| 55 | ++ | +++ | nt |
| 56 | ++ | +++ | nt |
| 57 | ++ | ++ | nt |
| 58 | ++ | +++ | nt |
| 59 | + | ++ | nt |
| 60 | + | ++ | nt |
| 61 | ++ | +++ | nt |
| 62 | + | +++ | nt |
| 63 | + | +++ | nt |
| 64 | +++ | +++ | nt |
| 65 | ++ | +++ | nt |
| 66 | + | +++ | nt |
| 67 | + | +++ | nt |
| 68 | ++ | +++ | nt |
| 69 | ++ | +++ | nt |
| 70 | ++ | +++ | nt |
| 71 | ++ | +++ | nt |
| 72 | ++ | +++ | nt |
| 73 | +++ | +++ | nt |
| 74 | +++ | +++ | nt |
| 75 | +++ | +++ | nt |
| 76 | ++ | +++ | nt |
| 77 | +++ | +++ | nt |
| 78 | ++ | +++ | nt |
| 79 | ++ | +++ | nt |
| 80 | ++ | +++ | nt |
| 81 | ++ | +++ | nt |
| 82 | +++ | +++ | nt |
| 83 | ++ | +++ | nt |
| 84 | ++ | +++ | nt |
| 85 | +++ | +++ | nt |
| 86 | +++ | +++ | nt |
| 87 | +++ | +++ | nt |
| 88 | +++ | +++ | nt |
| 89 | ++ | +++ | nt |
| 90 | +++ | +++ | nt |
| 91 | ++ | +++ | ++ |
| 92 | ++ | +++ | nt |
| 93 | ++ | +++ | + |
| 94 | ++ | +++ | nt |
| 95 | + | ++ | nt |
| 96 | ++ | +++ | nt |
| 97 | + | ++ | nt |
| 98 | +++ | +++ | nt |
| 99 | +++ | +++ | nt |
| 100 | +++ | +++ | nt |
| 101 | +++ | +++ | nt |
| 102 | +++ | +++ | nt |
| 103 | ++ | +++ | nt |
| 104 | ++ | +++ | nt |
| 105 | +++ | +++ | nt |
| 106 | +++ | +++ | nt |
| 107 | + | +++ | nt |
| 108 | ++ | +++ | nt |
| 109 | +++ | +++ | nt |
| 110 | ++ | +++ | nt |
| 111 | +++ | +++ | nt |
| 112 | ++ | +++ | nt |
| 113 | ++ | +++ | nt |
| 114 | ++ | +++ | nt |
| 115 | +++ | +++ | nt |
| 116 | +++ | +++ | nt |
| 117 | +++ | +++ | nt |
| 118 | +++ | +++ | nt |
| 119 | ++ | +++ | nt |
| 120 | +++ | +++ | nt |
| 121 | +++ | +++ | nt |
| 122 | +++ | +++ | nt |
| 123 | ++ | +++ | nt |
| 124 | ++ | +++ | nt |
| 125 | +++ | +++ | nt |
| 126 | +++ | +++ | nt |
| 127 | +++ | +++ | nt |
| 128 | +++ | +++ | nt |
| 129 | ++ | +++ | nt |
| 130 | +++ | +++ | nt |
| 131 | +++ | +++ | nt |
| 132 | ++ | +++ | nt |
| 133 | + | +++ | nt |
| 134 | ++ | +++ | nt |
| 135 | + | +++ | nt |
| 136 | +++ | +++ | nt |
| 137 | + | +++ | nt |
| 138 | ++ | +++ | nt |
| 139 | +++ | +++ | nt |
| 140 | +++ | +++ | nt |
| 141 | + | +++ | nt |
| 142 | + | +++ | nt |
| 143 | +++ | +++ | nt |
| 144 | +++ | +++ | nt |
| 145 | +++ | +++ | nt |
| 146 | +++ | +++ | nt |
| 147 | +++ | +++ | nt |
| 148 | nt | nt | nt |
| +++ =< 10nM | | | |
| ++ = 10 - 100 nM | | | |
| + = >100 nM | | | |
| nt = not tested | | | |

### In Vivo Evaluation of Steroid 5α-Reductase Inhibitors

The in vivo activity of steroid 5α-reductase inhibitors may be determined in both acute and chronic rat models. The acute model utilizes castrated male rats that receive testosterone (1 mg) subcutaneously and test compound (10 mg/kg) p.o., at 0.5 hr. and 4.5 hr. prior to sacrifice, respectively. Levels of DHT in the serum and prostate indicate the ability of the test compound to inhibit steroid 5α-reductase in an acute rat model. Known steroid 5α-reductase inhibitors were tested in parallel to ensure consistency of the assay method.

The chronic model also utilizes castrated male rats that are dosed daily with testosterone (20 µg/rat) subcutaneously and with test compound (0.01-10 mg/kg) p.o. for 7 days. The animals are then sacrificed and their prostates weighed. Reduction in the size of testosterone-stimulated prostate weight demonstrated activity of the test compound. Known steroid 5a-reductase inhibitors were tested in parallel to ensure consistency of the assay method.

### Utility

The steroid 5α-reductase inhibitors of the present invention are useful in the treatment of androgen responsive diseases, e.g., benign and malignant diseases of the prostate, especially benign prostatic hyperplasia. For correlation of in vitro, rat in vivo and human clinical data relating to an inhibitor of 5α-reductase, see Stoner, J. Steroid Biochem. Molec. Biol., **37**, 375 (1990); Brooks, et al., Steroids, **47**, 1 (1986) and Rasmusson, J. Med. Chem., **29**, 2298 (1986)). They are also useful in the treatment of prostatitis, prostate cancer, androgen mediated diseases of the skin, such as acne, hirsutism and male pattern baldness. Other hormone related diseases, e.g., polycystic ovary disease, would be expected to respond to treatment with these inhibitors.

The amount of compound of formula (I) required to be effective as an 5α-reductase inhibitor will, of course, vary with the individual mammal being treated and is ultimately at the discretion of the medical or veterinary practitioner. The factors to be considered include the condition being treated, the route of administration, the nature of the formulation, the mammal's body weight, surface area, age and general condition, and the particular compound to be administered. However, a suitable effective 5α-reductase inhibitory dose is in the range of about 0.01 to about 5 mg/kg body weight per day, preferably in the range of about 0.05 to about 2 mg/kg per day.

The total daily dose may be given as a single dose, multiple doses, e.g., two to six times per day, or by intravenous infusion for a selected duration. Dosages above or below the range cited above are within the scope of the present invention and may be administered to the individual patient if desired and necessary. For example, for a 75 kg mammal, a dose range would be about 5 to about 150 mg per day, and a typical dose would be about 20 mg per day. If discrete multiple doses are indicated, treatment might typically be 5 mg of a compound of formula (I) given 4 times per day.

The compounds of formula (I) may also be administered in a topical formulation, e.g., ointment, cream, get, or lotion, in cases of dermatological disorders such as acne vulgaris. An effective topical formulation contains from about 0.25% to about 10% by weight, of a compound of formula (I) which is applied at the rate of about 0.1 g to about 1.0 g per square centimeter of infected skin area. Typically a dose is about 1 gram of a 1% ointment, cream, get, or lotion of a compound of formula (I) gently rubbed onto the square centimeter of skin in need of treatment.

### Formulations

Formulations of the present invention for medical use comprise an active compound, i.e., a compound of formula (I), together with an acceptable carrier thereof and optionally other therapeutically active ingredients. The carrier must be pharmaceutically acceptable in the sense of being compatible with the other ingredients of the formulation and not deleterious to the recipient thereof.

The present invention, therefore, further provides a pharmaceutical formulation comprising a compound of formula (I) together with a pharmaceutically acceptable carrier thereof.

The formulations include those suitable for oral, rectal, topical or parenteral (including subcutaneous, intramuscular and intravenous) administration. Preferred are those suitable for oral or parenteral administration.

The formulations may conveniently be presented in unit dosage form and may be prepared by any of the methods well known in the art of pharmacy. All methods include the step of bringing the active compound into association with a carrier which constitutes one or more accessory ingredients. In general, the formulations are prepared by uniformly and intimately bringing the active compound into association with a liquid carrier or a finely divided solid carrier and then, if necessary, shaping the product into desired unit dosage form.

Formulations of the present invention suitable for oral administration may be presented as discrete units such as capsules, cachets, tablets or lozenges, each containing a predetermined amount of the active compound; as a powder or granules; or a suspension or solution in an aqueous liquid or non-aqueous liquid, e.g., a syrup, an elixir, an emulsion or a draught.

A tablet may be made by compression or molding, optionally with one or more accessory ingredients. Compressed tablets may be prepared by compressing in a suitable machine the active compound in a free-flowing form, e.g., a powder or granules, optionally mixed with accessory ingredients, e.g., binders, lubricants, inert diluents, surface active or dispersing agents. Molded tablets may be made by molding in a suitable machine, a mixture of the powdered active compound with any suitable carrier.

A syrup or suspension may be made by adding the active compound to a concentrated, aqueous solution of a sugar, e.g., sucrose, to which may also be added any accessory ingredients. Such accessory ingredient(s) may include flavoring, an agent to retard crystallization of the sugar or an agent to increase the solubility of any other ingredient, e.g., as a polyhydric alcohol, for example, glycerol or sorbitol.

Formulations for rectal administration may be presented as a suppository with a conventional carrier, e.g., cocoa butter or Witepsol S55 (trademark of Dynamite Nobel Chemical, Germany), for a suppository base.

Formulations suitable for parenteral administration conveniently comprise a sterile aqueous preparation of the active compound which is preferably isotonic with the blood of the recipient. Such formulations suitably comprise a solution or suspension of a pharmaceutically and pharmacologically acceptable acid addition salt of a compound of the formula (I) that is isotonic with the blood of the recipient.

Thus, such formulations may conveniently contain distilled water, 5% dextrose in distilled water or saline and a pharmaceutically and pharmacologically acceptable acid addition salt of a compound of the formula (I) that has an appropriate solubility in these solvents, for example the hydrochloride, isothionate and methanesulfonate salts, preferably the latter. Useful formulations also comprise concentrated solutions or solids containing the compound of formula (I) which upon dilution with an appropriate solvent give a solution suitable for parental administration above.

Topical formulations include ointments, creams, gels and lotions which may be prepared by conventional methods known in the art of pharmacy. In addition to the ointment, cream, get, or lotion base and the active ingredient, such topical formulation my also contain preservatives, perfumes, and additional active pharmaceutical agents.

In addition to the aforementioned ingredients, the formulations of this invention may further include one or more optional accessory ingredient(s) utilized in the art of pharmaceutical formulations, e.g., diluents, buffers, flavoring agents, binders, surface active agents, thickeners, lubricants, suspending agents, preservatives (including antioxidants) and the like.

### EXAMPLES

The following examples illustrate aspects of this invention but should not be construed as limitations. The symbols and conventions used in these examples are consistent with those used in the contemporary chemical literature, for example, the Journal of the American Chemical Society.

### Example 1

### 17β-N-9-Fluorenyl-carbamoyl-6-azaandrost-4-en-3-one (Compound 1)

A. 3β-Triisopropylsilyloxyetienic acid methyl ester
   A suspension of 3β-hydroxyetienic acid methyl ester (*J*. *Med. Chem.* **27**, 1690) (516 g, 1.55 mol) in DMF (800 mL) is heated to 55°C, imidazole (264 g, 3.88 mol) added with vigorous mechanical stirring, followed by dropwise addition of triisopropylsilylchloride (360 g, 1.87 mol). The reaction becomes homogeneous after about half of the triisopropylsilylchloride is added and the reaction temperature increases to ca. 70°C. The reaction is complete by TLC (35% ethyl acetate/hexanes) after 1.5 hrs and a thick slurry forms. The reaction is cooled to 0°C, 1 L of ice water added with stirring, the solid collected by filtration and washed with water (500 mL) and methanol (500 mL). The resulting tan solid is suspended in methanol (1 L) and allowed to stir overnight to give, on filtration, 3β-triisopropylsilyloxyetienic acid methyl ester as a tan solid of sufficient purity to carry on to the following steps; yield: 667 g (88%); m. p. 116-118°C. Recrystallization from hexane gives an analytical sample as a white crystalline solid: m.p. 124-125°C. Anal. Calcd. for C₃₀H₅₂O₃Si; C, 73.71; H, 10.72. Found: C, 73.79; H, 10.74.
B. A solution of 3β-triisopropylsilyloxyetienic acid methyl ester (166 g, 0.34 mol), from part A, in methylene chloride (2 L) and methanol (800 mL) is cooled to -78°C and treated with ozone until a blue color persists. At this stage, the peroxy compound of formula (V) may be isolated and recrystallized from hexanes to give an analytical sample; m. p. 119-121°C. Anal. Calcd. for C₃₁H₅₆O₇Si; C, 65.45; H, 9.92. Found: C, 65.37; H, 9.86. However, more conveniently, the reaction is allowed to warm to -50°C under a stream of nitrogen, zinc dust added (89 g, 1.36 mol), followed by glacial acetic acid (150 mL). The reaction is then allowed to warm to room temperature with stirring, filtered to remove zinc, the solution washed with water, saturated aqueous NaCI, saturated aqueous bicarbonate, dried over MgSO₄ and concentrated by rotary evaporation to give crude keto-aldehyde of formula (VI) as a white foam; yield: 176 g (99%).
C. The compound prepared in part B above (176 g, 0.34 mol) is dissolved in acetone, cooled to 0°C and treated with Jones reagent (1.05 eq.) by dropwise addition over 15 min. After 10 min isopropanol is added, the reaction is allowed to stir for 10 min, is filtered, concentrated, dissolved in ethyl acetate (400 mL), washed with saturated NaCI, dried (MgSO₄) and concentrated to a green oil. The oil is dissolved in methylene chloride (150 mL), silica get added (50 g), the mixture filtered through silica get (200 g) and eluted with hexane/methylene chloride/ethyl acetate/ methanol (8:8:4:1) to give on concentration the corresponding keto-acid of formula (VII) as an off-white solid; yield: 163 g (89%). Recrystallization from ethyl acetate/hexanes gives a white crystalline solid; m. p. 143-145°C. Anal. Calcd. for C₃₀H₅₂O₆Si; C, 67.12; H, 9.76. Found: C, 67.21; H, 9.80.
D. 17β-Carbomethoxy-3β-triisopropylsilyloxy-6-azaandrost-5-ene
   A portion of the keto-acid of formula (VII) prepared above (110 g, 0.205 mol) is dissolved in methylene chloride (120 mL) and pyridine (44.7 mL) and is added dropwise to a solution of oxalyl chloride (48.3 mL, 0.553 mol) in methylene chloride (770 mL) at 0°C. After stirring 30 min, the reaction is poured into a mixture of saturated aqueous NaCI and ice (700 mL), the layers separated, the methylene chloride is washed with ice-cold 0.5M HCI (2X800 mL), ice-cold saturated NaHCO₃ (3X700 mL), dried (Na₂SO₄) and concentrated to give the acid chloride as a white foam; yield: 110 g (96%). This material is then dissolved in acetone (1 L) and pentane (55 mL), cooled to 0°C, and treated with an aqueous solution of sodium azide (53 g, 0.82 mol, 164 mL). After 30 min the reaction is poured into a mixture of saturated NaCI (300 mL), saturated NaHCO₃ (300 mL) and ice (300 mL). Toluene is added (1 L), the layers separated, toluene layer washed with ice-cold saturated NaHCO₃ (3X400 mL), dried (Na₂SO₄), filtered and heated to 110°C to distill down to one-half the original volume. The solution is cooled to 38°C and treated with silica get (212 g). The reaction is allowed to stir overnight, the silica removed by filtration and washed with 4:1 ethyl acetate/methanol (770 mL) to give 17β-carbomethoxy-3β-triisopropylsilyloxy-6-azaandrost-5-ene (a compound of formula ((IX)) as a white foam; yield: 106 g (94%). Flash chromatography on silica get (30% ethyl acetate/hexanes) gives an analytical sample as a white foam. Anal. Calcd. for C₂₉H₅₁NO₃Si; C, 71.11; H, 10.49; N, 2.86. Found: C, 71.04; H, 10.51; N, 2.80.
E. 17β-Carbomethoxy-6-*t*-butoxycarbonyl-6-azaandrost-4-en-3-one
   A portion of the crude product from above, 17β-carbomethoxy-3β-triisopropylsilyloxy-6-azaandrost-5-ene (66 g, 0.135 mol) is dissolved in pyridine (500 mL), treated with di-*t*-butyldicarbonate (150 g, 0.69 mol) and allowed to stir overnight. The pyridine is removed by rotary evaporation and tetrabutylammonium fluoride (500 mL, 1M, 0.5 mol) in tetrahydrofuran (THF) added carefully and the reaction heated to reflux for 5 min. The THF is removed by rotary evaporation, the residue dissolved in ethyl acetate (500 mL), washed cautiously with water, saturated aqueous NaCI, dried with MgSO₄ and concentrated. This material is dissolved in DMF (500 mL), is treated with pyridinium dichromate (153 g, 0.41 mol) and allowed to stir overnight. The reaction is poured into water (700 mL) and extracted with ethyl acetate (2X500 mL). The combined extracts are washed with water, 5% aqueous CuSO₄, saturated aqueous NaCI, dried over MgSO₄, concentrated and flash chromatographed (0-60%, diethyl ether/hexanes) to give 17β-carbomethoxy-6-*t*-butoxycarbonyl-6-azaandrost-4-en-3-one as an off-white foam; yield: 37.5 g (64%); FAB mass spec. MH⁺ 432.
F. 17β-Carboxy-6-*t*-butoxycarbonyl-6-azaandrost-4-en-3-one
   A solution of 17β-carbomethoxy-6-*t*-butoxycarbonyl-6-azaandrost-4-en-3-one (15.4 g, 36 mmol), from part E, in dioxane (150 mL) and water (100 mL) is treated with LiOH•H₂O (3.31 g, 79 mmol) and stirred overnight in a water bath. The reaction is poured into saturated aqueous NaHSO₄ (150 mL), extracted with methylene chloride (3X100 mL), extracts washed with saturated aqueous NaCI, dried over MgSO₄ and concentrated to a volume of 100 mL. At this point crystals begin to form and 2:1 hexanes/ethyl acetate (50 mL) is added, the mixture triturated, cooled to room temperature and 17β-carboxy-6-*t*-butoxycarbonyl-6-azaandrost-4-en-3-one collected as a fluffy white powder; yield: 9.44 g (63%); m. p. 215-216°C. Anal. Calcd. for C₂₄H₃₅NO₅•¹/₄H₂O ; C, 68.30; H, 8.48; N, 3.32. Found: C, 68.45; H, 8.41; N, 3.28.
   The mother liquor is diluted with methylene chloride (100 mL), filtered through silica get, silica washed with 1:1 diethyl ether/hexanes and the eluant concentrated to give recovered 17β-carbomethoxy-6-*t*-butoxycarbonyl-6-azaandrost-4-en-3-one; yield: 2.63 g (17%). The silica pad is then washed with 1:9 methanol/methylene chloride (250 mL), the eluant concentrated, the resulting solid triturated with 2:1 hexanes/ethyl acetate (50 mL), cooled to 0°C and 17β-carboxy-6-*t*-butoxycarbonyl-6-azaandrost-4-en-3-one collected as a white powder; yield: 2.25 g (15%). The combined yield based on recovered starting material is 94%.
G. 17β-N-9-Fluorenyl-carbamoyl-6-azaandrost-4-en-3-one
   A sample of 17β-carboxy-6-*t*-butoxycarbonyl-6-azaandrost-4-en-3-one (502 mg, 1.20 mmol), from part F, is suspended in toluene (15 mL), treated with pyridine (0.15 mL, 1.5 eq.), cooled to 0°C and thionyl chloride added (0.13 mL, 1.5 eq.). After 1 hr at 0°C the reaction is concentrated to a yellow solid, this crude acid chloride is dissolved in methylene chloride (15 mL) and added to a biphasic mixture of methylene chloride (5 mL) and saturated aqueous NaHCO₃ (5 mL) which contains 9-fluorenyl amine hydrochloride (2.5 eq.). After stirring 45 min, ethyl acetate (50 mL) is added, the layers separated, the ethyl acetate layer washed with saturated aqueous NaHSO₄, saturated aqueous NaCI, dried over MgSO₄, concentrated and chromatographed on silica get (35-50% ethyl acetate/hexanes) to give 17β-N-9-fluorenyl-carbamoyl-6-*t*-butoxycarbonyl-6-azaandrost-4-en-3-one as a yellow oil; yield: 558 mg (80%). This material is dissolved in methylene chloride (15 mL) and treated with trifluoroacetic acid (2 mL) at room temperature. After 3 hrs the reaction is concentrated, methylene chloride (50 mL) and saturated aqueous bicarbonate (50 mL) added, the layers separated, methylene chloride washed with saturated aqueous NaCI, dried over MgSO₄, concentrated and crystallized from acetonitrile to give 17β-N-9-fluorenyl-carbamoyl-6-azaandrost-4-en-3-one as a white crystalline solid; yield: 325 mg (56%); m. p. 227-230°C. Anal. Calcd. for C₃₂H₃₆N₂O₂•¹/₂H₂O ; C, 78.49; H, 7.62; N, 5.72. Found: C, 78.25; H, 7.67; N, 5.65.

### Example 2

### 17β-(1-Oxo-2-cyclohexylethyl)-6-azaandrost-4-en-3-one (Compound 2)

A solution of 17β-carboxy-6-*t*-butoxycarbonyl-6-azaandrost-4-en-3-one (260 mg, 0.62 mmol), example 1, part F, is dissolved in toluene (10 mL) and treated with pyridine (3 eq) and catalytic dimethylformamide, cooled to 0°C, and thionyl chloride added (80 mL, 1.10 mmol). The reaction is then allowed to warm to room temperature and stir for 1 hr. The solids are then removed by filtration, the solution concentrated, the resulting crude acid chloride dissolved in THF (6 mL), Cut added (120 mg, 0.62 mmol), cooled to -78°C and treated with methylenecyclo-hexylmagnesium bromide (2.0M in diethyl ether, 0.5 mL, 1 mmol). The reaction is allowed to warm to room temperature, stir for 30 min, is quenched with saturated NaHSO₄, extracted with ethyl acetate (2X25 mL), dried over MgSO₄, concentrated and chromatographed on silica get (25% ethyl acetate/hexane) to give 17β-(1-oxo-2-(cyclohexylethyl))-6-*t*-butoxycarbonyl-6-azaan-drost-4-en-3-one; yield: 302 mg (98%). This material may be deprotected as described in example 1, part G above to give, on crystallization from ether, 17β-(1-oxo-2-(cyclohexylethyl)-6-azaandrost-4-en-3-one; yield: 121 mg (50%); m. p. 214-216°C. Anal. Calcd. for C₂₆H₃₉NO₂•¹/₂H₂O ; C, 76.80; H, 9.92; N, 3.44. Found: C, 76.61; H, 9.89; N, 3.49.

### Example 3

### 17β-Hydroxymethyl-(trimethylacetate)-6-azaandrost-4-en-3-one (Compound 3)

A. 17β-Hydroxymethyl-3β-hydroxy-6-*t*-butoxycarbonyl-6-azaandrost-4-ene
   A solution of 17β-carbomethoxy-6-*t*-butoxycarbonyl-6-azaandrost-4-en-3-one (2.30 g, 5.33 mmol), example 1, part E, in methylene chloride (70 mL) at -78°C is treated with diisobutylaluminum hydride (1.5M in toluene, 15 mL, 22.5 mmol). After 20 minutes the reaction is quenched with methanol (4 mL), methylene chloride added (150 mL), washed with 2N NaOH and water, dried over MgSO₄ and concentrated to give crude 17β-hydroxymethyl-3β-hydroxy-6-*t*-butoxycarbonyl-6-azaandrost-4-ene of sufficient purity to carry on to the following steps; yield: 2.16 g (99%).
B. 17β-Hydroxymethyl-6-*t*-butoxycarbonyl-6-azaandrost-4-en-3-one
   A solution of 17β-Hydroxymethyl-3b-hydroxy-6-*t*-butoxycarbonyl-6-azaandrost-4-ene (prepared as described in Example 5 above), in chloroform (250 mL) is treated with activated manganese(lV) oxide. After stirring 3.5 h, the manganese salts are removed by filtration through Celite. The solution is concentrated and chromatographed on silica get (50% ethyl acetate/hexanes) to give 17β-hydroxymethyl-6-*t*-butoxycarbonyl-6-azaandrost-4-en-3-one of sufficient purity to carry on to subsequent steps; yield 9.75 g (98%).
C. 17β-Hydroxymethyl-(trimethylacetate)-6-azaandrost-4-en-3-one
   A solution of 17β-hydroxymethyl-6-*t*-butoxycarbonyl-6-azaandrost-4-en-3-one (1.28 g, 3.17 mmol) in dichloromethane (32 mL) is cooled to 0°C and treated with triethylamine (0.67 mL, 4.8 mmol), 4-dimethylaminopyridine (58.4 mg, 0.48 mmol), and trimethylacetyl chloride (0.6 mL, 4.8 mmol). After stirring 1 h at 0°C and 3 h at room temperature, the reaction is diluted with dichloromethane, washed with saturated aqueous NaHSO₄, saturated aqueous NaHCO₃, and saturated aqueous NaCI, dried over MgSO₄, filtered, concentrated, and chromatographed on silica get (25% ethyl acetate/hexanes) to give the crude ester. This material is treated with trifluoroacetic acid as described in Example 4 above to give, after recrystallization from dichloromethane/acetonitrile, 17β-Hydroxymethyl-(trimethylacetate)-6-azaandrost-4-en-3-one as a white powder; yield: 843 mg (69%); m.p. 239-241°C. Anal. Calcd. for C₂₄H₃₇NO₃: C, 74.38; H, 9.62; N, 3.61. Found: C, 74.17; H, 9.64; N, 3.58.

### Examples 4-86

Following the general procedure of example 1, part G, wherein the hydrochloride salt of the amine is reacted with the acid chloride in the presence of sodium bicarbonate or by reaction with the free base or metal salt of the amine, for preparation of amides as the Z substituent in compounds of formula (I); the general procedure of example 2 for preparation of ketones as the Z substituent in compounds of formula (I); and the general procedure of example 3 for preparation of compounds where Z = CH₂OCOR⁵ and Z = CH₂OCO₂R⁵ in compounds of formula (I) the following compounds were prepared:

### Example 4

### 17β-N-5-Indanylcarbamoyl-6-azaandrost-4-en-3-one (Compound 4)

Melting Point: >270°C.

| | | | |
|---|---|---|---|
| Anal. Calcd. for C₂₈H₃₆N₂O₂•³/₄H₂O; | C, 75.39; | H, 8.47; | N, 6.30. |
| Found: | C, 75.29; | H, 8.42; | N, 6.20. |

### Example 5

### 17β-N-Phenyl-(4-morpholino)-carbamoyl-6-azaandrost-4-en-3-one (Compound 5)

Melting Point: >250°C.

| | | | |
|---|---|---|---|
| Anal. Calcd. for C₂₉H₃₉N₃O₃•¹/₄H₂O; | C, 72.24; | H, 8.26; | N, 8.72. |
| Found: | C, 72.26; | H, 8.26; | N, 8.73. |

### Example 6

### 17β-N-4-Benzodioxan-carbamoyl-6-azaandrost-4-en-3-one (Compound 6)

Melting Point: >260°C.

| | | | |
|---|---|---|---|
| Anal. Calcd. for C₂₇H₃₄N₂O₄; | C, 71.97; | H, 7.61; | N, 6.22. |
| Found: | C, 71.70; | H, 7.68; | N, 6.18. |

### Example 7

### 17β-N-(3,4-Methylenedioxy)phenyl-carbamoyl-6-azaandrost-4-en-3-one (Compound 7)

Melting Point: >264°C.

| | | | |
|---|---|---|---|
| Anal. Calcd. for C₂₆H₃₂N₂O₄•H₂O; | C, 68.70; | H, 7.54; | N, 6.16. |
| Found: | C, 68.78; | H, 7.60; | N, 5.96. |

### Example 8

### 17β-N-2-(3-Indolyl)ethyl-carbamoyl-6-azaandrost-4-en-3-one (Compound 8)

Meltina Point: 196-202°C.

| | | | |
|---|---|---|---|
| Anal. Calcd. for C₂₉H₃₇N₃O₂•¹/₂H₂O; | C, 74.33; | H, 8.17; | N, 8.97. |
| Found: | C, 74.31; | H, 8.15; | N, 8.87. |

### Example 9

### 17β-N-(2-Trifluoromethyl)phenyl-carbamoyl-6-azaandrost-4-en-3-one (Compound 9)

Melting Point: 249-253°C.

| | | | |
|---|---|---|---|
| Anal. Calcd. for C₂₆H₃₁N₂O₂F₃•¹/₃H₂O; | C, 66.93; | H, 6.84; | N, 6.00. |
| Found: | C, 67.02; | H, 7.00; | N, 5.71. |

### Example 10

### 17β-N-(2,5-Di-t-butyl)phenyl-carbamoyl-6-azaandrost-4-en-3-one (Compound 10)

Melting Point: 178-186°C.

| | | | |
|---|---|---|---|
| Anal. Calcd. for C₃₃H₄₈N₂O₂•¹/₄H₂O; | C, 77.83; | H, 9.60; | N, 5.50. |
| Found: | C, 77.72; | H, 9.61; | N, 5.49. |

### Example 11

### 17β-N-(4-Chlorophenyl)(phenyl)methyl-carbamoyl-6-azaandrost-4-en-3-one (Compound 11)

Melting Point: 245-250°C (decomp.).

| | | | | |
|---|---|---|---|---|
| Anal. Calcd. for C₃₂H₃₇N₂O₂Cl•³/₈H₂O; | C,73.37; | H,7.26; | N,5.35; | Cl,6.78. |
| Found: | C,73.24; | H,7.23; | N,5.38; | Cl,6.76. |

### Example 12

### 17β-N-(4-Methyl)phenethyl-carbamoyl-6-azaandrost-4-en-3-one (Compound 12)

Melting Point: 244-246°C.

| | | | |
|---|---|---|---|
| Anal. Calcd. for C₂₈H₃₈N₂O₂•¹/₄H₂O; | C, 76.59; | H, 8.84; | N, 6.38. |
| Found: | C, 76.97; | H, 8.93; | N, 6.40. |

### Example 13

### 17β-N-(α-Methyl)phenethyl-carbamoyl-6-azaandrost-4-en-3-one (Compound 13)

Melting Point: 188-189°C.

| | | | |
|---|---|---|---|
| Anal. Calcd. for C₂₈H₃₈N₂O₂•¹/₄H₂O; | C, 76.59; | H, 8.84; | N, 6.38. |
| Found: | C, 76.32; | H, 9.00; | N, 6.29. |

### Example 14

### 17β-N-(2,6-Di-i-propyl)phenyl-carbamoyl-6-azaandrost-4-en-3-one (Compound 14)

Melting Point: 198-200°C.

| | | | |
|---|---|---|---|
| Anal. Calcd. for C₃₁H₄₄N₂O₂•¹/₂H₂O; | C, 76.66; | H, 9.33; | N, 5.76. |
| Found: | C, 76.77; | H, 9.09; | N, 5.77. |

### Example 15

### 17β-N-1-Fluorenyl-carbamoyl-6-azaandrost-4-en-3-one (Compound 15)

Melting Point: 215-230°C (decomp.).

| | | | |
|---|---|---|---|
| Anal. Calcd. for C₃₂H₃₆N₂O₂•³/₄H₂O; | C, 77.78; | H, 7.65; | N, 5.67. |
| Found: | C, 77.91; | H, 7.56; | N, 5.76. |

### Example 16

### 17β-N-(3,3-Diphenyl)propyl-carbamoyl-6-azaandrost-4-en-3-one (Compound 16)

Melting Point: 136-141°C.

| | | | |
|---|---|---|---|
| Anal. Calcd. for C34H42N2O2•1/2H2O; | C, 78.57; | H, 8.34; | N, 5.39. |
| Found: | C, 78.86; | H, 8.29; | N, 5.40. |

### Example 17

### 17β-N-(α-Phenyl)phenethyl-carbamoyl-6-azaandrost-4-en-3-one (Compound 17)

Melting Point: >250°C.

| | | | |
|---|---|---|---|
| Anal. Calcd. for C₃₃H₄₀N₂O₂; | C, 79.80; | H, 8.12; | N, 5.64. |
| Found: | C, 79.74; | H, 8.11; | N, 5.60. |

### Example 18

### 17β-N-(4-Fluorophenyl)(2-thiophene)methyl-carbamoyl-6-azaandrost-4-en-3-one (Compound 18)

Melting Point: 172-180°C.

| | | | | |
|---|---|---|---|---|
| Anal. Calcd. for C₃₀H₃₅N₂O₂S•³/₈H₂O; | C,70.16; | H,7.02; | N,5.46; | S,6.25. |
| Found: | C,70.18; | H,6.99; | N,5.50; | S,6.24. |

### Example 19

### 17β-N-(2-Furanyl)methyl-carbamoyl-6-azaandrost-4-en-3-one (Compound 19)

Melting Point: 156-158°C.

| | | | |
|---|---|---|---|
| Anal. Calcd. for C₂₄H₃₂N₂O₃•1¹/₄H₂O; | C, 68.79; | H, 8.30; | N, 6.68. |
| Found: | C, 68.59; | H, 8.18; | N, 6.59. |

### Example 20

### 17β-N-2-(2-Pyridyl)ethyl-carbamoyl-6-azaandrost-4-en-3-one (Compound 20)

Melting Point: 132-135°C.

| | | | |
|---|---|---|---|
| Anal. Calcd. for C₂₆H₃₅N₃O₂•H₂O; | C, 71.04; | H, 8.48; | N, 9.55. |
| Found: | C, 70.99; | H, 8.46; | N, 9.12. |

### Example 21

### 17β-N-(1,1-Dimethyl)-2-hydroxyethyl-carbamoyl-6-azaandrost-4-en-3-one (Compound 21)

Melting Point: 170°C (decomp.).

| | |
|---|---|
| FAB mass spec. for C₂₃H₃₆N₂O₃; | 388.54 |
| Found: | 389 MH⁺ |

### Example 22

### 17β-N-(2,2-Diphenyl)ethyl-carbamoyl-6-azaandrost-4-en-3-one (Compound 22)

Melting Point: >250°C.

| | |
|---|---|
| FAB mass spec. for C₃₃H₄₀N₂O₂; | 496.7 |
| Found: | 497 MH⁺ |

### Example 23

### 17β-N-2-(Phenylthio)ethyl-carbamoyl-6-azaandrost-4-en-3-one (Compound 23)

Melting Point: 130-131°C.

| | | | |
|---|---|---|---|
| Anal. Calcd. for C₂₇H₃₆N₂O₂S•H₂O; | C, 68.90; | H, 8.13; | N, 5.95. |
| Found: | C, 68.59; | H, 7.76; | N, 5.94. |

### Example 24

### 17β-N-Bis-(4-fluorophenyl)methyl-carbamoyl-6-azaandrost-4-en-3-one (Compound 24)

Melting Point: 178-184°C.

| | | | |
|---|---|---|---|
| Anal. Calcd. for C₃₂H₃₆N₂O₂F₂•¹/₄H₂O; | C, 73.47; | H, 7.03; | N, 5.36. |
| Found: | C, 73.55; | H, 7.04; | N, 5.33. |

### Example 25

### 17β-N-(2,2-Diphenyl)-hydrazidyl-6-azaandrost-4-en-3-one (Compound 25)

Melting Point: 194-196°C.

| | | | |
|---|---|---|---|
| Anal. Calcd. for C₃₁H₃₇N₃O₂•¹/₂H₂O; | C,75.55; | H,7.78; | N,8.53. |
| Found: | C, 75.44; | H, 7.82; | N, 8.50. |

### Example 26

### 17β-(1-Oxo-1-(2,4-difluorophenyl)methyl)-6-azaandrost-4-en-3-one (Compound 26)

Melting Point: 238°C (decomp.).

| | | | |
|---|---|---|---|
| Anal. Calcd. for C₂₅H₂₉NO₂F₂•¹/₄H₂O; | C,71.83; | H,7.11; | N,3.35. |
| Found: | C, 72.07; | H, 6.97; | N, 3.64. |

### Example 27

### 17β-Hydroxymethyl-(3-methylbutyrate)-6-azaandrost-4-en-3-one (Compound 27)

Melting Point: 137-138°C.

| | | | |
|---|---|---|---|
| Anal. Calcd. for C₂₄H₃₇NO₃•H₂O; | C, 71.07; | H, 9.69; | N, 3.45. |
| Found: | C, 70.82; | H, 9.69; | N, 3.46. |

### Example 28

### 17β-Hydroxymethyl-(2,2-diphenylacetate)-6-azaandrost-4-en-3-one (Compound 28)

Melting Point: 200-201°C.

| | | | |
|---|---|---|---|
| Anal. Calcd. for C₃₃H₃₉NO₃•¹/₄H₂O; | C, 78.93; | H, 7.93; | N, 2.79. |
| Found: | C, 79.14; | H, 7.97; | N, 2.79. |

### Example 29

### 17β-N-2-(3-N-Methylpyrrole)ethyl-carbamoyl-6-azaandrost-4-en-3-one (Compound 29)

Melting Point: 134-136°C.

| | | | |
|---|---|---|---|
| Anal. Calcd. for C₂₆H₃₇N₃O₂•¹/₂H₂O; | C, 72.19; | H, 8.85; | N, 9.71. |
| Found: | C, 71.88; | H, 8.77; | N, 9.60. |

### Example 30

### 17β-N-(4-Trifluoromethylphenyl)methyl-carbamoyl-6-azaandrost-4-en-3-one (Compound 30)

Melting Point: 160-168°C.

| | | | |
|---|---|---|---|
| Anal. Calcd. for C₂₇H₃₃N₂O₂F₃•¹/₄H₂O; | C, 67.69; | H, 7.05; | N, 5.85. |
| Found: | C, 67.75; | H, 7.09; | N, 5.86. |

### Example 31

### 17β-methylenecyclohexyl-carbamoyl-6-azaandrost-4-en-3-one (Compound 31)

Melting Point: 172-174°C.

| | | | |
|---|---|---|---|
| Anal. Calcd. for C₂₆H₄₀N₂O₂•³/₄H₂O; | C, 73.28; | H, 9.82; | N, 6.57. |
| Found: | C, 73.03; | H, 9.88; | N, 6.54. |

### Example 32

### 17β-N-(1,1-Dimethyl)-2-(4-fluorophenyl)ethyl-carbamoyl-6-azaandrost-4-en-3-one (Compound 32)

Melting Point: 144-148°C.

| | | | |
|---|---|---|---|
| Anal. Calcd. for C₂₉H₃₉N₂O₂F; | C, 74.63; | H, 8.43; | N, 6.01. |
| Found: | C, 74.66; | H, 8.48; | N, 5.92. |

### Example 33

### 17β-(1-Oxo-1 (4-isopropoxyphenyl)methyl)-6-azaandrost-4-en-3-one (Compound 33)

Melting Point: >260°C.

| | | | |
|---|---|---|---|
| Anal. Calcd. for C₂₈H₃₇NO₃•²/₃H₂O; | C, 75.13; | H, 8.63; | N, 3.13. |
| Found: | C, 75.24; | H, 8.57; | N, 3.13. |

### Example 34

### 17β-N-(Dicyclohexyl)methyl-carbamoyl-6-azaandrost-4-en-3-one (Compound 34)

Melting Point: 245-246°C.

| | | | |
|---|---|---|---|
| Anal. Calcd. for C₃₂H₅₀N₂O₂; | C, 77.68; | H, 10.19; | N, 5.66. |
| Found: | C, 77.54; | H, 10.20; | N, 5.60. |

### Example 35

### 17β-N-(1-Phenyl)ethyl-carbamoyl-6-azaandrost-4-en-3-one (Compound 35)

Melting Point: 160-177°C.

| | | | |
|---|---|---|---|
| Anal. Calcd. for C₂₇H₃₆N₂O₂•H₂O; | C, 73.94; | H, 8.73; | N, 6.39. |
| Found: | C, 73.95; | H, 8,72; | N, 6.37. |

### Example 36

### 17β-N-(4-Decyl)phenyl-carbamoyl-6-azaandrost-4-en-3-one (Compound 36)

Melting Point: 151-154°C.

| | | | |
|---|---|---|---|
| Anal. Calcd. for C₃₅H₅₂N₂O₂•H₂O; | C, 76.32; | H, 9.88; | N, 5.09. |
| Found: | C, 75.99; | H, 9.62; | N, 5.06. |

### Example 37

### 17β-N-(4-Benzyl)piperidyl-carbamoyl-6-azaandrost-4-en-3-one (Compound 37)

Melting Point: 250-251°C.

| | | | |
|---|---|---|---|
| Anal. Calcd. for C₃₁H₄₂N₂O₂•¹/₄H₂O; | C, 77.70; | H, 8.94; | N, 5.85. |
| Found: | C, 77.39; | H, 8.88; | N, 6.00. |

### Example 38

### 17β-Hydroxymethyl-(1-adamantylcarboxylate)-6-azaandrost-4-en-3-one (Compound 38)

Melting Point: >275°C.

| | | | |
|---|---|---|---|
| Anal. Calcd. for C₃₀H₄₃NO₃; | C, 77.38; | H, 9.31; | N, 3.01. |
| Found: | C, 77.05; | H, 9.20; | N, 3.07. |

### Example 39

### 17β-N-Myrantyl-carbamoyl-6-azaandrost-4-en-3-one (Compound 39)

Melting Point: 182-185°C (foams).

| | | | |
|---|---|---|---|
| Anal. Calcd. for C₂₉H₄₄N₂O₂•³/₄H₂O; | C, 74.71; | H, 9.84; | N, 6.01. |
| Found: | C, 74.69; | H, 9.78; | N, 6.00. |

### Example 40

### 17β-N-(2,4,6-Trimethyl)phenyl-carbamoyl-6-azaandrost-4-en-3-one (Compound 40)

Melting Point: 243-251°C.

| | | | |
|---|---|---|---|
| Anal. Calcd. for C₂₈H₃₈N₂O₂•2TFA; | C, 58.00; | H, 6.08; | N, 4.23. |
| Found: | C, 59.62; | H, 6.73; | N, 4.73. |

### Example 41

### 17β-N-Hydroxy-N-t-butyl-carbamoyl-6-azaandrost-4-en-3-one (Compound 41)

Melting Point: 252-254°C (decomp.).

| | | | |
|---|---|---|---|
| Anal. Calcd. for C₂₃H₃₆N₂O₃; | C, 71.10; | H, 9.34; | N, 7.21. |
| Found: | C, 71.03; | H, 9.39; | N, 7.20. |

### Example 42

### 17β-N-(2,4-Difluoro)benzyl-carbamoyl-6-azaandrost-4-en-3-one (Compound 42)

Melting Point: 134-137°C.

| | | | |
|---|---|---|---|
| Anal. Calcd. for C₂₆H₃₂N₂O₂F₂•H₂O; | C, 67.80; | H, 7.44; | N, 6.08. |
| Found: | C, 67.70; | H, 7.25; | N, 6.09. |

### Example 43

### 17β-(1-Oxo-3.3-diphenylpropyl)-6-azaandrost-4-en-3-one (Compound 43)

Melting Point: 106°C (shrinks).

| | |
|---|---|
| FAB mass spec. for C₃₃H₃₉NO₂; | 481.68 |
| Found: | 482 MH⁺ |

### Example 44

### 17β-N-(2-Chloro-5-trifluoromethyl)-phenyl-carbamoyl-6-azaandrost-4-en-3-one (Compound 44)

Melting Point: 173-174°C.

| | | | |
|---|---|---|---|
| Anal. Calcd. for C₂₆H₃₀N₂O₂ClF₃; | C, 63.09; | H, 6.11; | N, 5.66. |
| Found: | C, 62.88; | H, 6.48; | N, 5.62. |

### Example 45

### 17β-N-(2,2-Diphenyl-1-methyl)ethyl-carbamoyl-6-azaandrost-4-en-3-one (Compound 45)

Melting Point: >240°C.

| | | | |
|---|---|---|---|
| Anal. Calcd. for C₃₄H₄₂N₂O₂•¹/₄H₂O; | C, 79.26; | H, 8.31; | N, 5.44. |
| Found: | C, 79.17; | H, 8.35; | N, 5.46. |

### Example 46

### 17β-N-(2,6-Dimethyl-4-bromo)phenyl-carbamoyl-6-azaandrost-4-en-3-one (Compound 46)

Melting Point: >256°C.

| | | | |
|---|---|---|---|
| Anal. Calcd. for C₂₇H₃₅N₂O₂Br•¹/₃H₂O; | C, 64.15; | H, 7.11; | N, 5.54. |
| Found: | C, 64.30; | H, 7.10; | N, 5.53. |

### Example 47

### 17β-N-Bis-(4-chlorophenyflmethyl-carbamoyl-6-azaandrost-4-en-3-one (Compound 47)

Melting Point: 188-194°C.

| | | | |
|---|---|---|---|
| Anal. Calcd. for C₃₂H₃₆N₂O₂Cl₂•¹/₄H₂O; | C, 69.67; | H, 6.58; | N, 5.08. |
| Found: | C, 69.51; | H, 6.56; | N, 5.02. |

### Example 48

### 17β-N-(2,6-Dimethyl-4-t-butyl)phenyl-carbamoyl-6-azaandrost-4-en-3-one (Compound 48)

Melting Point: 203°C (shrinks).

| | | | |
|---|---|---|---|
| Anal. Calcd. for C₃₁H₄₄N₂O₂•⁵/₄H₂O; | C, 74.58; | H, 9.39; | N, 5.61. |
| Found: | C, 74.59; | H, 9.30; | N, 5.62. |

### Example 49

### 17β-N-(2,6-Dibromo-4-isopropyl)phenyl-carbamoyl-6-azaandrost-4-en-3-one (Compound 49)

Melting Point: 182°C (foams).

| | | | |
|---|---|---|---|
| Anal. Calcd. for C₂₈H₃₆N₂O₂Br₂•¹/₃H₂O; | C, 56.20; | H, 6.18; | N, 4.68. |
| Found: | C, 56.08; | H, 6.24; | N, 4.61. |

### Example 50

### 17β-N-(2,5-Ditrifluoromethyl)phenyl-carbamoyl-6-azaandrost-4-en-3-one (Compound 50)

Melting Point: 144°C (shrinks).

| | | | |
|---|---|---|---|
| Anal. Calcd. for C₂₇H₃₀N₂O₂F₆•²/₃H₂O; | C, 59.99; | H, 5.84; | N, 5.18. |
| Found: | C, 60.06; | H, 6.10; | N, 5.01. |

### Example 51

### 17β-N-(4-t-Butyl)cyclohexyl-carbamoyl-6-azaandrost-4-en-3-one (Compound 51)

Melting Point: 229-231°C.

| | | | |
|---|---|---|---|
| Anal. Calcd. for C₂₉H₄₆N₂O₂•¹/₄H₂O; | C, 75.85; | H, 10.21; | N, 6.10. |
| Found: | C, 75.72; | H, 10.22; | N, 6.08. |

### Example 52

### 17β-N-(2-Bromo)phenyl-carbamoyl-6-azaandrost-4-en-3-one (Compound 52)

Melting Point: >280°C.

| | | | |
|---|---|---|---|
| Anal. Calcd. for C₂₅H₃₁N₂O₂Br; | C, 63.69; | H, 6.63; | N, 5.94. |
| Found: | C, 63.56; | H, 6.67; | N, 5.90. |

### Example 53

### 17β-N-(2-Phenyl)phenyl-carbamoyl-6-azaandrost-4-en-3-one (Compound 53)

Melting Point: >280°C.

| | | | |
|---|---|---|---|
| Anal. Calcd. for C₃₁H₃₆N₂O₂•2H₂O; | C, 73.78; | H, 7.99; | N, 5.55. |
| Found: | C, 73.62; | H, 7.17; | N, 5.47. |

### Example 54

### 17β-N-(2,6-Diethyl-3,5-dichloro)phenyl-carbamoyl-6-azaandrost-4-en-3-one (Compound 54)

Melting Point: >250°C.

| | | | |
|---|---|---|---|
| Anal. Calcd. for C₂₉H₃₈N₂O₂Cl₂•H₂O; | C, 65.04; | H, 7.53; | N, 5.23. |
| Found: | C, 65.38; | H, 7.49; | N, 5.25. |

### Example 55

### 17β-N-(2,6-Diethyl-3-chloro)phenyl-carbamoyl-6-azaandrost-4-en-3-one (Compound 55)

Melting Point: 230-232°C.

| | | | |
|---|---|---|---|
| Anal. Calcd. for C₂₉H₃₉N₂O₂Cl•¹/₄H₂O; | C, 71.44; | H, 8.17; | N, 5.75. |
| Found: | C, 71.41; | H, 8.20; | N, 5.75. |

### Example 56

### 17β-N-(2-t-Butyl)phenyl-carbamoyl-6-azaandrost-4-en-3-one (Compound 56)

Melting Point: 174-180°C.

| | | | |
|---|---|---|---|
| Anal. Calcd. for C₂₉H₄₀N₂O₂•¹/₂H₂O; | C, 76.11; | H, 9.03; | N, 6.12. |
| Found: | C, 76.12; | H, 9.02; | N, 6.14. |

### Example 57

### 17β-N-(3,5- Ditrifluoromethyl)phenyl-carbamoyl-6-azaandrost-4-en-3-one (Compound 57)

Melting Point: 194°C (shrinks).

| | | | |
|---|---|---|---|
| Anal. Calcd. for C₂₇H₃₀N₂O₂F₆•¹/₃H₂O; | C, 60.67; | H, 5.78; | N, 5.24. |
| Found: | C, 60.72; | H, 5.82; | N, 5.03. |

### Example 58

### 17β-N-(2,4,6-Trichloro)phenyl-carbamoyl-6-azaandrost-4-en-3-one (Compound 58)

Melting Point: >250°C.

| | | | |
|---|---|---|---|
| Anal. Calcd. for C₂₅H₂₉N₂O₂Cl₃•¹/₃H₂O; | C, 60.55; | H, 5.89; | N, 5.65. |
| Found: | C, 60.52; | H, 5.93; | N, 5.65. |

### Example 59

### 17β-N-(3,5-Tetramethyl)cyclohexyl-carbamoyl-6-azaandrost-4-en-3-one (Compound 59)

Melting Point: 193-195°C.

| | | | |
|---|---|---|---|
| Anal. Calcd. for C₂₉H₄₆N₂O₂Br₂•H₂O; | C, 73.68; | H, 10.24; | N, 5.93. |
| Found: | C, 73.72; | H, 10.34; | N, 5.99. |

### Example 60

### 17β-(1-Oxo-1-((2,4,6-tri-i-propyl)phenyl)methyl)-6-azaandrost-4-en-3-one (Compound 60)

Melting Point: >230°C.

| | | | |
|---|---|---|---|
| Anal. Calcd. for C₃₄H₄₉NO₂•¹/₂H₂O; | C, 79.64; | H, 9.82; | N, 2.73. |
| Found: | C, 79.53; | H, 9.57; | N, 2.73. |

### Example 61

### 17β-N-(2-Bromo-5-trifluoromethyl)phenyl-carbamoyl-6-azaandrost-4-en-3-one (Compound 61)

Melting Point:170-190°C (decomp.).

| | | | |
|---|---|---|---|
| Anal. Calcd. for C₂₆H₃₀N₂O₂BrF₃; | C, 57.89; | H, 5.61; | N, 5.19. |
| Found: | C, 57.83; | H, 5.63; | N, 5.15. |

### Example 62

### 17β-N-(2-t-Butyl-6-methyl)phenyl-carbamoyl-6-azaandrost-4-en-3-one (Compound 62)

Melting Point: 190-220°C (decomp.).

| | |
|---|---|
| FAB mass spec. for C₃₀H₄₂N₂O₂; | 462.68 |
| Found: | 463 MH⁺ |

### Example 63

### 17β-N-(1-Methyl-1-phenyl)ethyl-carbamoyl-6-azaandrost-4-en-3-one (Compound 63)

Melting Point: 156°C (shrinks).

| | | | |
|---|---|---|---|
| Anal. Calcd. for C₂₈H₃₈N₂O₂•¹/₂H₂O; | C, 75.81; | H, 8.86; | N, 6.31. |
| Found: | C, 75.90; | H, 8.84; | N, 6.30. |

### Example 64

### 17β-N-1-(4-Chlorophenyl)cyclopentyl-carbamoyl-6-azaandrost-4-en-3-one (Compound 64)

Melting Point: 186-189°C.

| | | | |
|---|---|---|---|
| Anal. Calcd. for C₃₀H₃₉N₂O₂Cl; | C, 72.77; | H, 7.94; | N, 5.66. |
| Found: | C, 72.54; | H, 7.92; | N, 5.63. |

### Example 65

### 17β-N-(1-(4-Chlorophenyl)-2-methyl)propyl-carbamoyl-6-azaandrost-4-en-3-one (Compound 65)

Melting Point: 177-179°C.

| | | | |
|---|---|---|---|
| Anal. Calcd. for C₂₉H₃₉N₂O₂Cl•¹/₄H₂O; | C, 71.43; | H, 8.17; | N, 5.75. |
| Found: | C, 71.43; | H, 8.20; | N, 5.79. |

### Example 66

### 17β-N-(1,1-Di-4-chlorophenyl)ethyl-carbamoyl-6-azaandrost-4-en-3-one (Compound 66)

Melting Point: 186-194°C.

| | | | | |
|---|---|---|---|---|
| Anal. Calcd. for C₃₃H₃₈N₂O₂Cl₂; | C, 70.00; | H, 6.78; | N, 4.96; | Cl, 12.54. |
| Found: | C, 69.89; | H, 6.84; | N, 4.92; | Cl, 12.41. |

### Example 67

### 17β-N-(1 -Phenyl-1-(4-t-butyl)phenyl)methyl-carbamoyl-6-azaandrost-4-en-3-one (Compound 67)

Melting Point: 188-192°C.

| | | | |
|---|---|---|---|
| Anal. Calcd. for C₃₆H₄₆N₂O₂•¹/₄H₂O; | C, 79.59; | H, 8.63; | N, 5.16. |
| Found: | C, 79.52; | H, 8.67; | N, 5.10. |

### Example 68

### 17β-(1-Oxo-1-(2-norbornyl)methyl)-6-azaandrost-4-en-3-one (Compound 68)

Melting Point: 128°C (shrinks).

| | |
|---|---|
| High res. mass spec. for C₂₆H₃₇NO₂; | 396.2903 |
| Found: | 396.2911 MH⁺ |

### Example 69

### 17β-N-(2,6-Dibromo-4-chloro)phenyl-carbamoyl-6-azaandrost-4-en-3-one (Compound 69)

Melting Point: 240-244°C (decomp.).

| | | | |
|---|---|---|---|
| Anal. Calcd. for C₂₅H₂₉N₂O₂Br₂Cl•¹/₂H₂O; | C, 50.57; | H, 5.09; | N, 4.72. |
| Found: | C, 50.41; | H, 4.94; | N, 4.62. |

### Example 70

### 17β-N-(2,6-Diethyl-4-bromo)phenyl-carbamoyl-6-azaandrost-4-en-3-one (Compound 70 )

Melting Point: 212-216°C.

| | | | | |
|---|---|---|---|---|
| Anal. Calcd. for C₂₉H₃₉N₂O₂Br; | C, 66.01; | H, 7.46; | N, 5.31; | Br, 15.16. |
| Found: | C, 65.94; | H, 7.46; | N, 5.29; | Br, 15.24. |

### Example 71

### 17β-N-(2-Bromo-4-t-butyl)phenyl-carbamoyl-6-azaandrost-4-en-3-one (Compound 71)

Melting Point: >250°C.

| | | | | |
|---|---|---|---|---|
| Anal. Calcd. for C₂₉H₃₉N₂O₂Br; | C, 66.01; | H, 7.46; | N, 5.31; | Br, 15.16. |
| Found: | C, 65.91; | H, 7.42; | N, 5.31; | Br, 15.09. |

### Example 72

### 17β-N-(2-Chloro-4-t-butyl)phenyl-carbamoyl-6-azaandrost-4-en-3-one (Compound 72)

Melting Point: 235-246°C (decomp.).

| | | | | |
|---|---|---|---|---|
| Anal. Calcd. for C₂₉H₃₉N₂O₂Cl; | C, 71.44; | H, 8.16; | N, 5.74; | Cl, 7.27. |
| Found: | C, 71.52; | H, 8.19; | N, 5.75; | Cl, 7.26. |

### Example 73

### 17β-N-(1-t-Butyl-1-(4-t-butylphenyl))methyl-carbamoyl-6-azaandrost-4-en-3-one (Compound 73)

Melting Point: 198-204°C.

| | | | |
|---|---|---|---|
| Anal. Calcd. for C₃₄H₅₀N₂O₂Br₂; | C, 76.72; | H, 9.75; | N, 5.26. |
| Found: | C, 76.81; | H, 9.65; | N, 5.26. |

### Example 74

### 17β-N-(5-Bromo-2-t-butyl)phenyl-carbamoyl-6-azaandrost-4-en-3-one (Compound 74)

Melting Point: 192-195°C.

| | | | |
|---|---|---|---|
| Anal. Calcd. for C₂₉H₃₉N₂O₂Br•H₂O; | C, 63.85; | H, 7.58; | N, 5.14. |
| Found: | C, 63.75; | H, 7.58; | N, 5.16. |

### Example 75

### 17β-N-(5-Chloro-2-t-butyl)phenyl-carbamoyl-6-azaandrost-4-en-3-one (Compound 75)

Melting Point: 235-246°C (decomp.).

| | | | |
|---|---|---|---|
| Anal. Calcd. for C₂₉H₃₉N₂O₂Cl•H₂O; | C, 69.51; | H, 8.25; | N, 5.59. |
| Found: | C, 69.34; | H, 8.22; | N, 5.98. |

### Example 76

### 17β-N-(2,6-Diethyl)phenyl-carbamoyl-6-azaandrost-4-en-3-one (Compound 76)

Melting Point: 190-196°C.

| | | | |
|---|---|---|---|
| Anal. Calcd. for C₂₉H₄₀N₂O₂•³/₈H₂O; | C, 76.49; | H, 9.02; | N, 6.15. |
| Found: | C, 76.59; | H, 9.01; | N, 6.10. |

### Example 77

### 17β-N-(4-Bromo-2-t-butyl)phenyl-carbamoyl-6-azaandrost-4-en-3-one (Compound 77)

Melting Point: 162°C (foams).

| | | | |
|---|---|---|---|
| Anal. Calcd. for C₂₉H₃₉N₂O₂Br; | C, 66.01; | H, 7.46; | N, 5.31. |
| Found: | C, 65.94; | H, 7.60; | N, 5.20. |

### Example 78

### 17β-N-(2-t-Butyl-5-cyano)phenyl-carbamoyl-6-azaandrost-4-en-3-one (Compound 78)

Melting Point: >300°C.

| | | | |
|---|---|---|---|
| Anal. Calcd. for C₃₀H₃₉N₃O₂•³/₄H₂O; | C, 73.96; | H, 8.38; | N, 8.63. |
| Found: | C, 74.11; | H, 8.48; | N. 8.68. |

### Example 79

### 17β-N-(2-(O-4-Tolyl)-5-trifluoromethyl)phenyl-carbamoyl-6-azaandrost-4-en-3-one (Compound 79)

Melting Point: 134-142°C.

| | | | |
|---|---|---|---|
| Anal. Calcd. for C₃₃H₃₇N₂O₃F₃•¹/₃*i*-propanol; | C, 69.95; | H, 6.58; | N, 4.94. |
| Found: | C, 69.61; | H, 6.86; | N, 4.73. |

### Example 80

### 17β-N-(2-(O-2-Methoxyphenyl)-5-trifluoromethyl))phenyl-carbamoyl-6-azaandrost-4-en-3-one (Compound 80)

Melting Point: 132-142°C.

| | |
|---|---|
| FAB mass spec. for C₃₃H₃₇N₂O₄F₃; | 582.67 |
| Found: | 583 MH⁺ |

### Example 81

### 17β-N-(2-(O-4-Chlorophenyl)-5-trifluoromethyl)phenyl-carbamoyl-6-azaandrost-4-en-3-one (Compound 81)

Melting Point: 132-142°C.

| | | | |
|---|---|---|---|
| Anal. Calcd. for C₃₂H₃₄N₂O₃ClF₃•¹/₃*i*-propanol•¹/₃H₂O; | C, 64.65; | H, 6.14; | N, 4.57. |
| Found: | C,64.74; | H,6.23; | N,4.58. |

### Example 82

### 17β-N-(2-Nitro-4-t-butyl)phenyl-carbamoyl-6-azaandrost-4-en-3-one (Compound 82)

Melting Point: >265°C.

| | | | |
|---|---|---|---|
| Anal. Calcd. for C₂₉H₃₉N₃O₄; | C, 70.56; | H, 7.96; | N, 8.51.. |
| Found: | C, 70.42; | H, 7.98; | N, 8.42. |

### Example 83

### 17β-N-(2-(O-Phenyl)-5-(1,1-dimethyl)propyl)phenyl-carbamoyl-6-azaandrost-4-en-3-one (Compound 83)

Melting Point: 147-152°C.

| | | | |
|---|---|---|---|
| Anal. Calcd. for C₃₆H₄₆N₂O₃•¹/₂H₂O; | C, 76.70; | H, 8.40; | N, 4.97.. |
| Found: | C, 76.55; | H, 8.39; | N, 4.95. |

### Example 84

### 17β-N-(2-Ethyl-4-cyano)phenyl-carbamoyl-6-azaandrost-4-en-3-one (Compound 84)

Melting Point: >250°C.

| | | | |
|---|---|---|---|
| Anal. Calcd. for C₂₈H₃₅N₃O₂; | C, 75.47; | H, 7.92; | N, 9.43. |
| Found: | C, 75.26; | H, 7.95; | N, 9.35. |

### Example 85

### 17β-N-(2-Ethylsulfonyl-5-trifluoromethyl)phenyl-carbamoyl-6-azaandrost-4-en-3-one (Compound 85)

Melting Point: >250°C.

| | | | |
|---|---|---|---|
| Anal. Calcd. for C₂₈H₃₅N₂O₄F₃S•H₂O; | C, 58.93; | H, 6.54; | N, 4.91. |
| Found: | C, 58.87; | H, 6.54; | N, 4.89. |

### Example 86

### 17β-N-(3,5-Di-t-butyl)phenyl-carbamoyl-6-azaandrost-4-en-3-one (Compound 86)

Melting Point: 212-216°C.

| | | | |
|---|---|---|---|
| Anal. Calcd. for C₃₃H₄₈N₂O₂; | C, 77.49; | H, 9.61; | N, 5.48. |
| Found: | C, 77.59; | H, 9.57; | N, 5.53. |

### Example 87

### 17β-N-(2-t-Butyl-5-trifluoromethyl)phenyl-carbamoyl-6-azaandrost-4-en-3-one (Compound 87)

A. 4-Trifluoromethyl-α,α-dimethylbenzyl alcohol
To magnesium turnings (30.2 g, 1.24 mol) in THF (30 mL) is added 1,2-dibromoethane (0.7 mL) followed by dropwise addition, over 1 h, of 4-bromotrifluoromethylbenzene (200 g, 0.89 mol) dissolved in THF (700 mL). The reaction is heated occasionally to maintain a gentle reflux and is stirred 45 min after the addition is complete. The dark reaction mixture is then cooled in an ice bath and acetone (103 g, 1.78 mol) added dropwise. After stirring an additional 1.5 h the reaction is carefully quenched with aqueous saturated NaHSO₄, ethyl acetate added, the organics dried over MgSO₄, concentrated and purified by silica get chromatography (9:1 to 3:2, hexane to ethyl acetate) to give 4-trifluoromethyl-α,α-dimethylbenzyl alcohol of sufficient purity to carry to the next step; yield: 114 g (63%).
B. 4-Trifluoromethyl-α,α-dimethylbenzyl chloride
The alcohol from step A above (54 g, 0.27 mol) is treated with ethereal HCI (1.3 L, 1 M) followed by anhydrous zinc chloride (53 mL, 1 M in ether) and the reaction allowed to stir for 22 h at room temperature. The reaction is then washed with water, aqueous 2 N NaOH, dried over MgSO₄, concentrated and purified by silica get chromatography (8:1 hexane to ethyl acetate) to give 4-trifluoromethyl-α,α-dimethylbenzyl chloride of sufficient purity to carry to the next step; yield: 50.5 g (85%).
C. 4-Trifluoromethyl-t-butylbenzene
To a toluene solution of dimethylzinc (150 mL, 2.0 M, 0.30 mol) at -78°C is added a CH₂Cl₂ solution of titanium tetrachloride (150 mL, 1.0 M, 0.15 mol) and after stirring for 30 min 4-trifluoromethyl-α,α-dimethylbenzyl chloride (83.3 g, 0.373 mol) in CH₂Cl₂ (100 mL) is added dropwise over 20 min. After the addition is complete the reaction is allowed to warm to -40°C over 1.5 h. After an additional 2 h at -40°C the brown reaction mixture is carefully poured onto crushed ice, extracted with CH₂Cl₂ (2X500 mL), the combined extracts dried over MgSO₄ and concentrated to give 4-trifluoromethyl-t-butylbenzene; yield: 60.3 g (80%). Anal. Calcd. for C₁₁H₁₃F₃: C, 65.32; H, 6.48. Found: C, 65.11; H, 6.38.
D. 2-t-Butyl-5-trifluoromethylnitrobenzene
To a solution of 4-trifluoromethyl-t-butylbenzene (25.6 g, 126 mmol) in H₂SO₄ (143 mL) at 0°C is added a mixture of HNO₃ (90%, 42 mL) and H₂SO₄ (87 mL). After 30 min the ice bath is removed and the reaction stirred for 2.5 h further. The yellow mixture is then poured onto crushed ice and extracted with ethyl acetate, the ethyl acetate washed with 2N NaOH and water, dried (MgSO₄) and concentrated to a dark oil. Purification by silica get chromatography (14:1 hexane to ethyl acetate) gives 2-*t*-butyl-5-trifluoromethylnitrobenzene of sufficient purity to carry to the next step; yield: 18.0 g (58%).
E. 2-*t*-Butyl-5-trifluoromethylaniline
A solution of 2-*t*-butyl-5-trifluoromethylnitrobenzene (26.0 g, 105 mmol) in ethanol (95%, 100 mL) is treated with 10% Pd/C (2 g) and placed under a hydrogen atmosphere (40 psi) on a Parr apparatus for 1.5 h. The mixture is filtered, concentrated and purified by silica get chromatography (7:1 hexane to ethyl acetate) to give 2-*t*-butyl-5-trifluoromethylaniline as an oil; yield: 20.2 g (89%). Anal. Calcd. for C₁₁H₁₄F₃N: C, 60.80; H, 6.50; N, 6.45. Found: C, 60.89; H, 6.50; N, 6.51.
F. 17β-N-(2-*t*-Butyl-5-trifluoromethyl)phenyl-carbamoyl-6-azaandrost-4-en-3-one
The aniline prepared above is reacted with the acid chloride of 17β-carboxy-6-*t*-butoxycarbonyl-6-azaandrost-4-en-3-one (prepared as in example 1, part G) and deprotected as in example 1, part G to give: 7β-N-(2-*t*-butyl-5-trifluoro)phenyl-carbamoyl-6-azaandrost-4-en-3-one.
Melting Point: 186-190°C.

| | | | |
|---|---|---|---|
| Anal. Calcd. for C₃₀H₃₉N₂O₂F₃ | C,69.73; | H,7.61; | N,5.42. |
| Found | C, 69.45; | H, 7.66; | N, 5.38. |

### Examples 88-92

Following the general procedure of example 1, part G, wherein the hydrochloride salt of the amine is reacted with the acid chloride in the presence of sodium bicarbonate or by reaction with the free base or metal salt of the amine, for preparation of amides as the Z substituent in compounds of formula (I); the general procedure of example 2 for preparation of ketones as the Z substituent in compounds of formula (I); and the general procedure of example 3 for preparation of compounds where Z = CH₂OCOR⁵ and Z = CH₂OCO₂R⁵ in compounds of formula (I) the following compounds were prepared:

### Example 88

### 17β-N-(2-t-Butyl-5-phenyl)phenyl-carbamoyl-6-azaandrost-4-en-3-one (Compound 88)

Melting Point: 207-209°C.

| | | | |
|---|---|---|---|
| Anal. Calcd. for C₃₅H₄₄N₂O₂•¹/₄H₂O; | C, 79.43; | H, 8.48; | N, 5.29. |
| Found: | C, 79.53; | H, 8.54; | N, 5.31. |

### Example 89

### 17β-Hydroxymethyl-(menthylcarbonate)-6-azaandrost-4-en-3-one (Compound 89)

Melting Point: 152-155°C.

| | | | |
|---|---|---|---|
| Anal. Calcd. for C₃₀H₄₇NO₄•H₂O; | C, 71.53; | H, 9.81; | N, 2.78. |
| Found: | C, 71.63; | H, 9.79; | N, 2.80. |

### Example 90

### 17β-Hydroxymethyl-(phenylcarbonate)-6-azaandrost-4-en-3-one (Compound 90)

Melting Point: 247-249°C (decomp.).

| | | | |
|---|---|---|---|
| Anal. Calcd. for C₂₆H₃₃NO₄•¹/₈CH₂Cl₂; | C, 72.27; | H, 7.72; | N, 3.23. |
| Found: | C, 71.82; | H, 7.77; | N, 3.25. |

### Example 91

### 17β-N-(2-t-Butyl-5-(N-methyl)methylsulfonamide)phenyl-6-azaandrost-4-en-3-one (Compound 91)

Melting Point: 290-292°C (decomp.).

| | | | |
|---|---|---|---|
| Anal. Calcd. for C₃₁H₄₅N₃O₄S; | C, 65.40; | H, 8.23; | N, 7.38. |
| Found: | C, 65.41; | H, 8.23; | N, 7.42. |

### Example 92

### 17β-N-(3-Nitro-4-t-butyl)phenyl-carbamoyl-6-azaandrost-4-en-3-one (Compound 92)

Melting Point: 214-217°C.

| | | | |
|---|---|---|---|
| Anal. Calcd. for C₂₉H₃₉N₃O₄; | C, 69.30; | H, 8.02; | N, 8.36. |
| Found: | C, 69.11; | H, 8.09; | N, 8.12. |

### Example 93

### 17β-N-(2,6-Di-i-propyl)phenyl-carbamoyl-6-azaandrost-1,4-dien-3-one (Compound 93)

A. 17β-Carbomethoxy-6-*t*-butoxycarbonyl-6-azaandrost-1,4-dien-3-one
   A solution of 17β-carbomethoxy-6-*t*-butoxycarbonyl-6-azaandrost-4-en-3-one (2.00 g, 4.63 mmol), prepared in example 1, part E, in dioxane (50 mL) is treated with 2,3-dichloro-5,6-dicyano-1,4-benzoquinone (DDQ, 1.37 g, 6.02 mmol) and *p*-nitrophenol (10 mg). The reaction is heated to reflux for 2 hrs, poured into ice water (150 mL), extracted with ethyl acetate (3X100 mL), extracts washed with saturated aqueous NaHSO₃, 2N NaOH, saturated aqueous NaCI, dried over MgSO₄, concentrated and chromatographed on silica get (40% ethyl acetate/hexanes) to give crude 17β-carbomethoxy-6-*t*-butoxycarbonyl-6-azaandrost-1,4-dien-3-one as a tan solid of sufficient purity to carry on to the following steps; yield: 1.53 g (76%).
B. 17β-N-(2,6-Di-*i*-propyl)phenyl-carbamoyl-6-*t*-butoxycarbonyl-6-azaandrost-1,4-dien-3-one
   A sample of crude 17β-carbomethoxy-6-*t*-butoxycarbonyl-6-azaandrost-1,4-dien-3-one (0.90 g, 2.20 mmol), prepared in part A, is demethylated as in example 1, part F and then coupled via the acid chloride (described in example 1, part G) with the lithium anion of 2,6-di-*i*-propylaniline to give after chromatography (30-50% ethyl acetate/hexanes), 17β-N-(2,6-di-*i*-propyl)phenyl-carbamoyl-6-*t*-butoxycarbonyl-6-azaandrost-1,4-dien-3-one as a white foam; yield: 0.210 g (16%).
C. 17β-N-(2,6-Di-*i*-propyl)phenyl-carbamoyl-6-azaandrost-1,4-dien-3-one
   A sample of 17βN-(2,6-di-*i*-propyl)phenyl-carbamoyl-6-*t*-butoxycarbonyl-6-azaandrost-1,4-dien-3-one (188 mg, 0.320 mmol), prepared in part B above, is treated with trifluoroacetic acid as described in example 1, part G above to give, after chromatography (50% ethyl acetate/hexanes to 5% methanol/chloroform), 17β-N-(2,6-di-*i*-propyl)phenyl-carbamoyl-6-azaandrost-1,4-dien-3-one as a white solid; yield: 98 mg (62%); m. p. 273-276°C. Anal. Calcd. for C₃₁H₄₂N₂O₂•H₂O ; C, 75.73; H, 8.81; N, 5.69. Found: C, 75.84; H, 8.95; N, 5.68.

### Example 94

### 17β-N-(2,6-Di-i-propyl)phenyl-carbamoyl-6-azaandrost-4-methyl-1,4-dien-3-one (Compound 94)

A. 17β-Carbomethoxy-6-*t*-butoxycarbonyl-6-azaandrost-4-methyl-1,4-dien-3-one
   A solution of 17β-carbomethoxy-6-*t*-butoxycarbonyl-6-azaandrost-1,4-dien-3-one (2.57 g, 5.98 mmol), prepared in example 93, part A in methylene chloride (60 mL) containing anhydrous K₂CO₃ (8.03 g, 58 mmol) at 0°C is treated with bromine (6.1 mmol) dissolved in methylene chloride (12 mL). After 30 min the mixture is filtered, washed with 10% aqueous NaHSO₃, dried over Na₂SO₄ and concentrated to a foam. This material is dissolved in dimethylformamide (20 mL), treated with phenyltrimethyltin (2.0 mL, 11.4 mmol) and lithium chloride (180 mg, 4.3 mmol), heated to 130°C and treated with PdCl₂(PPh₃)₂ (400 mg, 0.57 mmol). After 1.5 hr the reaction is allowed to cool to room temperature, filtered through Celite, poured into water, the water extracted with ethyl acetate (3X30 mL), extracts washed with water (3X100 mL) and 1% aqueous ammonia (2X100 mL), dried over Na₂SO₄, concentrated and chromatographed (5-10% ethyl acetate/methylene chloride) to give 17β-carbomethoxy-6-*t*-butoxycarbonyl-6-azaandrost-4-methyl-1,4-dien-3-one; yield: 1.25 g (47%).
B. 17β-N-(2,6-Di-*i*-propyl)phenyl-carbamoyl-6-azaandrost-4-methyl-1,4-dien-3-one
   A sample of crude 17β-carbomethoxy-6-*t*-butoxycarbonyl-6-azaandrost-4-methyl-1,4-dien-3-one (0.45 g, 1.01 mmol), prepared in part A, is hydrolized as in example 1, part F and then coupled with 2,6-di-*i*-propylaniline as described in example 93, part B, deprotected as in example 1, part G, to give after chromatography (2.5-10% methanol/methylene chloride), 17β-N-(2,6-di-*i*-propyl)phenyl-carbamoyl-6-azaandrost-4-methyl-1,4-dien-3-one as a white powder; yield: 70 mg (14%); m. p. 181-184°C. Anal. Calcd. for C₃₂H₄₄N₂O₂• ¹/₄H₂O ; C, 77.93; H, 9.09; N, 5.67. Found: C, 77.81; H, 9.12; N, 5.58.

### Example 95

### 17β-(N-i-Propyl-N-(N-i-propylcarbamoyl))-carbamoyl-6-azaandrost-4-en-3-one (Compound 95)

A solution of 17β-carboxy-6-*t*-butoxycarbonyl-6-azaandrost-4-en-3-one (500 mg, 1.2 mmol), example 1, part F, is dissolved in methylene chloride (70 mL), treated with diisopropyl carbodiimide (0.21 mL, 1.3 mmol) and stirred at room temperature for 20 hrs. The reaction mixture is then concentrated, the solid dissolved in ethyl acetate (70 mL), the ethyl acetate washed with saturated aqueous NaHCO₃ (2X70 mL), 1N HCl (3X70 mL), saturated aqueous NaCI (70 mL), dried over Na₂SO₄, and concentrated to a yellow foam. Chromatography on silica get (50% ethyl acetate/hexanes) gives 17β-(N-*i*-propyl-N-(N-*i*-propylcarbamoyl))-carbamoyl-6-*t*-butoxycarbonyl-6-azaandrost-4-en-3-one as a white foam; yield: 600 mg (65%). FAB mass spec. for C₃₁H₄₉N₃O₅; 543.75 Found: 544 MH⁺ This material is deprotected with TFA as in example 1, part G to give, after crystallization from acetonitrile, 17β-(N-*i*-propyl-N-(N-*i*-propylcarbamoyl))-carbamoyl-6-azaandrost-4-en-3-one as a white solid; yield: 220 mg (64%); m. p. 162°C (decomp.). Anal. Calcd. for C₂₆H₄₁N₃O₃•H₂O ; C, 67.64; H, 9.39 N, 9.10. Found: C, 67.58; H, 9.42; N, 9.09.

### Example 96

### 17β-(N-cyclohexyl-N-(N-cyclohexylcarbamoyl))-carbamoyl-6-azaandrost-4-en-3-one (Compound 96)

Prepared as described in example 95.
Melting Point: 186-187°C.

| | | | |
|---|---|---|---|
| Anal. Calcd. for C₃₂H₄₉N₃O₃•¹/₄H₂O; | C, 72.76; | H, 9.44; | N, 7.95. |
| Found: | C, 72.74; | H, 9.45; | N, 7.68. |

### Example 97

### 17β-Aminomethyl-(acetamide)-6-azaandrost-4-en-3-one (Compound 97)

A. 17β-Azidomethyl-6-*t*-butoxycarbonyl-6-azaandrost-4-en-3-one
   A solution of 17β-hydroxymethyl-6-*t*-butoxycarbonyl-6-azaandrost-4-en-3-one (2.03 g, 5.04 mmol) prepared in example 3, part 15, in dichloromethane (50 mL) is treated with triethylamine (0.88 mL, 6.3 mmol), DMAP (0.155 g, 1.27 mmol) and methanesulfonyl chloride (0.43 mL, 5.6 mmol). After 3 hrs, the reaction is diluted with dichloromethane (50 mL), washed with 1N HCl, saturated aqueous NaCI and dried over MgSO₄. Concentration gives a white foam which is dissolved in dimethylformamide (50 mL), treated with sodium azide (0.94 g, 14.5 mmol) and heated to 75°C for 16 hrs. The reaction is poured into ethyl acetate, washed with water (5X100 mL), dried over MgSO₄, concentrated and chromatographed on silica get (20% ethyl acetate/hexane) to give 17β-azidomethyl-6-*t*-butoxycarbonyl-6-azaandrost-4-en-3-one as a white foam of sufficient purity to carry on to the next step.
B. 17β-Aminomethyl-(acetamide)-6-azaandrost-4-en-3-one
   A sample of the azide from step A above (20 mg, 50 mmol) is dissolved in 1:1 ethanol/tetrahydrofuran (2 mL), treated with 10% Pd(C) (3.2 mg) and placed under a hydrogen atmosphere for 10 min. The reaction is filtered, concentrated, dissolved in dichloromethane (2 mL) and treated with pyridine (0.1 mL) and acetic anhydride (0.06 mL). After 64 hrs the reaction is diluted with dichloromethane, dried over Na₂SO₄, concentrated and chromatographed on silica get (ethyl acetate) to give 17β-aminomethyl-(acetamide)-6-*t*-butoxycarbonyl-6-azaandrost-4-en-3-one which is deprotected with TFA as in example 1, part G to give 17β-aminomethyl-(acetamide)-6-azaandrost-4-en-3-one as a white solid; yield: 3 mg (17%). FAB mass spec. for C₂₁H₃₂N₂O₂; 344.5. Found: 345 MH⁺.

### Example 98

### 17β-Aminomethyl-(1-adamantylurea)-6-azaandrost-4-en-3-one (Compound 98)

A sample of the azide from example 97, part A (100 mg, 230 mmol) is dissolved in 1:2 ethanol/tetrahydrofuran (6 mL), treated with 10% Pd(C) (60 mg) and placed under a hydrogen atmosphere for 1 hr. The reaction is filtered, concentrated, dissolved in tetrahydrofuran and treated with 1-adamantylisocyanate at room temperature for 6 days. The reaction is concentrated and chromatographed on silica get (40-70% ethyl acetate/hexanes) to give 17β-aminomethyl-(1-adamantylurea)-6-*t*-butoxycarbonyl-6-azaandrost-4-en-3-one which is deprotected with TFA as in example 1, part G to give 17β-aminomethyl-(1-adamantylurea)-6-azaandrost-4-en-3-one as a white solid; yield: 47 mg (35%); m. p. 227-229°C (foams.). Anal. Calcd. for C₃₀H₄₅N₃O₂•H₂O ; C, 72.40; H, 9.52; N, 8.44. Found: C, 72.37; H, 9.42; N, 8.46.

### Examples 99-148

Following the general procedure of example 1, part G, wherein the hydrochloride salt of the amine is reacted with the acid chloride in the presence of sodium bicarbonate or by reaction with the free base or metal salt of the amine, for preparation of amides as the Z substituent in compounds of formula (I); the general procedure of example 3 for preparation of compounds where Z = CH₂OCONR¹⁴R¹⁵ in compounds of formula (I); the general procedure of example 8 and 11 in WO 93/13124 for preparation of compounds of formula (I), where R³ = Cl and Me respectively, the following compounds were prepared. Novel amines used to prepare compounds of formula (I) where the Z substituent is an amide were prepared as described in example 87 above or by the method of Stille, J.K. *Pure Appl. Chem.,* **57**, 1771 (1985). When either R¹⁴ or R¹⁵ is an aryl substituted cycloalkyl residue the amine may be prepared by Curtius rearrangement of the corresponding acid, where available, or by the method of He, X. *et al., J. Med. Chem.,* **36**, 1188 (1993), *i.e.* by reacting the corresponding cycloalkanone with the appropriate aryl Grignard reagent followed by conversion of the resulting alcohol to the amine by treatment with sodium azide and trifluoroacetic acid followed by reduction of the azide with lithium aluminum hydride. Aryl substituted cyclopropylamines are prepared by rodium catalyzed insertion of the appropriate aryl-α-diazo-ester (prepared by the method of Baum, J.S. *et al., Synthetic Comm.,* **17**, 1709 (1987)) into the appropriate olefin (as described by Davies, H.W. *et al., Tetrahedron Lett.,* **30**, 5057 (1989)) followed by saponification of the ester and Curtius rearrangement of the acid to give the desired amine.

### Example 99

### 17β-Hydroxymethyl-(2,6-diisopropylphenylcarbamate)-6-azaandrost-4-en-3-one (Compound 99)

Melting Point: 161-163°C.

| | | | |
|---|---|---|---|
| Anal. Calcd. for C₃₂H₄₆N₂O₃•H₂O; | C, 73.25; | H, 9.22; | N, 5.34. |
| Found: | C, 73.08; | H, 9.19; | N, 5.29. |

### Example 100

### 17β-N-(4-t-Butyl-2-trifluoromethyl)phenyl-carbamoyl-6-azaandrost-4-en-3-one (Compound 100)

Melting Point: 186-190°C.

| | | | |
|---|---|---|---|
| Anal. Calcd. for C₃₀H₃₉N₂O₂F₃•¹/₄H₂O; | C, 69.14; | H, 7.64; | N, 5.38. |
| Found: | C, 69.12; | H, 7.65; | N, 5.35. |

### Example 101

### 17β-N-1-(2,4-Dichlorophenyl)cyclopropyl-carbamoyl-6-azaandrost-4-en-3-one (Compound 101)

Melting Point: 160-180°C (decomp.).

| | | | |
|---|---|---|---|
| Anal. Calcd. for C₂₈H₃₄N₂O₂Cl₂•¹/₂H₂O; | C, 65.86; | H, 6.91; | N, 5.49. |
| Found: | C, 69.64; | H, 6.89; | N, 5.47. |

### Example 102

### 17β-N-1-(3-Trifluoromethylphenyl)cyclopentyl-carbamoyl-6-azaandrost-4-en-3-one (Compound 102)

Melting Point: 156-159°C.

| | | | |
|---|---|---|---|
| Anal. Calcd. for C₃₁H₃₉N₂O₂F₃•H₂O; | C, 68.11; | H, 7.56; | N, 5.12. |
| Found: | C, 67.79; | H, 7.48; | N, 5.02. |

### Example 103

### 17β-N-(1-Ethyl-1-phenyl)propyl-carbamoyl-6-azaandrost-4-en-3-one (Compound 103)

Melting Point: 157-160°C.

| | | | |
|---|---|---|---|
| Anal. Calcd. for C₃₀H₄₂N₂O₂•³/₄H₂O; | C, 75.67; | H, 9.20; | N, 5.88. |
| Found: | C, 75.80; | H, 9.09; | N, 5.78. |

### Example 104

### 17β-N-1-(4-Fluorophenyl)cyclopentyl-carbamoyl-6-azaandrost-4-en-3-one (Compound 104)

Melting Point: 220-223°C.

| | | | |
|---|---|---|---|
| Anal. Calcd. for C₃₀H₃₉N₂O₂F•¹/₂H₂O; | C, 73.71; | H, 8.17; | N, 5.75. |
| Found: | C, 73.89; | H, 8.27; | N, 5.74. |

1-Amino-1-(4-fluorophenyl)cyclopentane: ¹³C NMR (CDCl₃) δ 162.8, 159.6, 145.2, 127.0, 126.8, 114.8, 114.5, 63.5, 41.5, 23.4.

### Example 105

### 17β-N-(2-t-Butyl-5-N,N-diethylcarbamoyl)phenyl-carbamoyl-6-azaandrost-4-en-3-one (Compound 105)

Melting Point: 167-169°C.

| | | | |
|---|---|---|---|
| Anal. Calcd. for C₃₄H₄₉N₃O₃•¹/₂H₂O; | C, 73.34; | H, 9.05; | N, 7.55. |
| Found: | C, 73.41; | H, 9.10; | N, 7.42. |

### Example 106

### 17β-N-1-(4-Trifluoromethylphenyl)cyclopentyl-carbamoyl-6-azaandrost-4-en-3-one (Compound 106)

Melting Point: 201-203°C.

| | | | |
|---|---|---|---|
| Anal. Calcd. for C₃₁H₃₉N₂O₂F₃•¹/₂H₂O; | C, 69.51; | H, 7.55; | N, 5.09. |
| Found: | C, 69.25; | H, 7.50; | N, 5.21. |

1-Amino-1-(4-trifluoromethylphenyl)cyclopentane: ¹³C NMR (CDCl₃) δ 153.6, 153.5, 128.5, 128.1, 126.0, 125.7, 125.1, 125.1, 125.0, 125.0, 122.4, 63.8, 41.7, 23.5.

### Example 107

### 17β-N-(3-Cyano)phenyl-carbamoyl-6-azaandrost-4-en-3-one (Compound 107)

Melting Point: 202-208°C.

| | | | |
|---|---|---|---|
| Anal. Calcd. for C₂₆H₃₁N₃O₂•H₂O; | C, 71.70; | H, 7.64; | N, 9.65. |
| Found: | C, 71.88; | H, 7.69; | N, 9.48. |

### Example 108

### 17β-N-(4-t-Butyl)phenyl-carbamoyl-6-azaandrost-4-en-3-one (Compound 108)

Melting Point: 304-307°C.

| | | | |
|---|---|---|---|
| Anal. Calcd. for C₂₉H₄₀N₂O₂•¹/₄H₂O; | C, 76.86; | H, 9.00; | N, 6.18. |
| Found: | C, 76.83; | H, 8.92; | N, 6.54. |

### Example 109

### 17β-N-1-(4-Fluorophenyl)cyclohexyl-carbamoyl-6-azaandrost-4-en-3-one (Compound 109)

Melting Point: 167-169°C.

| | | | |
|---|---|---|---|
| Anal. Calcd. for C₃₁H₄₁N₂O₂F•¹/₃H₂O; | C, 74.66; | H, 8.42; | N, 5.61. |
| Found: | C, 74.72; | H, 8.43; | N, 5.53. |

### Example 110

### 17β-N-1-(2,2-Dimethyl)indanyl-carbamoyl-6-azaandrost-4-en-3-one (Compound 110)

Melting Point: 178-182°C.

| | | | |
|---|---|---|---|
| Anal. Calcd. for C₃₀H₄₀N₂O₂; | C, 75.99; | H, 8.82; | N, 5.91. |
| Found: | C, 75.64; | H, 8.84; | N, 5.88. |

### Example 111

### 17β-N-1-(4-Methoxyphenyl)cyclohexyl-carbamoyl-6-azaandrost-4-en-3-one (Compound 111)

Melting Point: 167-169°C.

| | | | |
|---|---|---|---|
| Anal. Calcd. for C₃₂H₄₄N₂O₃•³/₄H₂O; | C, 74.17; | H, 8.85; | N, 5.41. |
| Found: | C, 73.95; | H, 8.92; | N, 5.38. |

### Example 112

### 17β-N-(2-Phenyl-5-trifluoromethyl)phenyl-carbamoyl-6-azaandrost-4-en-3-one (Compound 112)

Melting Point: 304-306°C.

| | | | |
|---|---|---|---|
| Anal. Calcd. for C₃₂H₃₅N₂O₂F₃; | C, 71.62; | H, 6.57; | N, 5.22. |
| Found: | C, 71.56; | H, 6.56; | N, 5.27. |

### Example 113

### 17β-N-(3-Methoxy-5-chloro)phenyl-carbamoyl-6-azaandrost-4-en-3-one (Compound 113)

Melting Point: 237-238°C.

| | | | |
|---|---|---|---|
| Anal. Calcd. for C₂₆H₃₃N₂O₃Cl•³/₄H₂O; | C, 66.37; | H, 7.39; | N, 5.95. |
| Found: | C, 66.17; | H, 7.31; | N, 5.96. |

### Example 114

### 17β-N-(2-Carboethoxy)phenyl-carbamoyl-6-azaandrost-4-en-3-one (Compound 114)

Melting Point: 282-286°C.

| | | | |
|---|---|---|---|
| Anal. Calcd. for C₂₈H₃₆N₂O₄; | C, 72.39; | H, 7.81; | N, 6.03. |
| Found: | C, 72.15; | H, 7.88; | N, 6.10. |

### Example 115

### 17β-N-1-(4-Methoxyphenyl)cyclopentyl-carbamoyl-6-azaandrost-4-en-3-one (Compound 115)

Melting Point: 160°C.

| | | | |
|---|---|---|---|
| Anal. Calcd. for C₃₁H₄₂N₂O₃•H₂O; | C, 73.19; | H, 8.72; | N, 5.51. |
| Found: | C, 73.26; | H, 8.67; | N, 5.46. |

### Example 116

### 17β-N-(2,5-bis(Trifluoromethyl))phenyl-carbamoyl-6-azaandrost-4-methyl-4-en-3-one (Compound 116)

Melting Point: 142-146°C.

| | | | |
|---|---|---|---|
| Anal. Calcd. for C₂₈H₃₂N₂O₂F₆; | C, 61.96; | H, 5.95; | N, 5.16. |
| Found: | C, 61.70; | H, 5.99; | N, 5.07. |

### Example 117

### 17β-N-(2-t-Butyl-5-trifluoromethyl)phenyl-carbamoyl-6-azaandrost-4-methyl-4-en-3-one (Compound 117)

Melting Point: 188°C.

| | | | |
|---|---|---|---|
| Anal. Calcd. for C₃₁H₄₁N₂O₂F₃•³/₄H₂O; | C, 68.42; | H, 7.87; | N, 5.15. |
| Found: | C, 68.31; | H, 7.71; | N, 5.08. |

### Example 118

### 17β-N-(2,5-Di-t-butyl)phenyl-carbamoyl-6-azaandrost-4-methyl-4-en-3-one (Compound 118)

Melting Point: 168-176°C.

| | | | |
|---|---|---|---|
| Anal. Calcd. for C₃₄H₅₀N₂O₂•¹/₂H₂O; | C, 77.37; | H, 9.74; | N, 5.31. |
| Found: | C, 77.32; | H, 9.74; | N, 5.28. |

### Example 119

### 17β-N-(2,5-Diethoxy)phenyl-carbamoyl-6-azaandrost-4-en-3-one (Compound 117)

Melting Point: 277-279°C.

| | | | |
|---|---|---|---|
| Anal. Calcd. for C₂₉H₄₀N₂O₄•¹/₄H₂O; | C, 71.80; | H, 8.41; | N, 5.77. |
| Found: | C, 71.50; | H, 8.45; | N, 5.74. |

### Example 120

### 17β-N-(2-t-Butyl-5-(4-chlorophenyl))phenyl-carbamoyl-6-azaandrost-4-en-3-one (Compound 120)

Melting Point: 214-216°C.

| | | | |
|---|---|---|---|
| Anal. Calcd. for C₃₅H₄₃N₂O₂Cl•1¹/₂H₂O; | C, 71.71; | H, 7.91; | N, 4.78. |
| Found: | C, 71.46; | H, 7.88; | N, 4.81. |

### Example 121

### 17β-N-1-(4-t-Butylphenyl)cyclopentyl-carbamoyl-6-azaandrost-4-en-3-one (Compound 121)

Melting Point: 222-232°C.

| | | | |
|---|---|---|---|
| Anal. Calcd. for C₃₄H₄₈N₂O₂•³/₄H₂O; | C, 77.01; | H, 9.41; | N, 5.28. |
| Found: | C, 77.05; | H, 9.46; | N, 5.25. |

1-Amino-1-(4-*t*-butylphenyl)cyclopentane: ¹³C NMR (CDCl₃) δ 148.9, 146.4, 125.1, 125.0, 124.9, 63.7, 41.2, 34.3, 31.4, 31.3, 23.6.

### Example 122

### 17β-N-1-(4-t-Butylphenyl)cyclohexyl-carbamoyl-6-azaandrost-4-en-3-one (Compound 122)

Melting Point: 203°C.

| | | | |
|---|---|---|---|
| Anal. Calcd. for C₃₅H₅₀N₂O₂; | C, 76.60; | H, 9.55; | N, 5.10. |
| Found: | C, 76.43; | H, 9.59; | N, 5.05. |

1-Amino-1-(4-*t*-butylphenyl)cyclohexane: ¹³C NMR (CDCl₃) δ 148.8, 146.8, 125.0, 124.6, 53.4, 39.2, 34.2, 31.2, 25.7, 22.4.

### Example 123

### 17β-N-(2-Benzoyl)phenyl-carbamoyl-6-azaandrost-4-en-3-one (Compound 123)

Melting Point: 290-294°C.

| | | | |
|---|---|---|---|
| Anal. Calcd. for C₃₂H₃₆N₂O₃•¹/₂H₂O; | C, 76.01; | H, 7.38; | N, 5.54. |
| Found: | C, 76.19; | H, 7.35; | N, 5.47. |

### Example 124

### 17β-N-1-(3,4-Methylenedioxyphenyl)cyclohexyl-carbamoyl-6-azaandrost-4-en-3-one (Compound 124)

Melting Point: 192°C.

| | | | |
|---|---|---|---|
| Anal. Calcd. for C₃₂H₄₂N₂O₄•³/₄H₂O; | C, 72.22; | H, 8.24; | N, 5.26. |
| Found: | C, 72.01; | H, 8.25; | N, 5.14. |

1-Amino-1-(3,4-methylenedioxyphenyl)cyclohexane: ¹³C NMR (CDCl₃) δ 147.5, 145.6, 144.1, 117.9, 107.6, 106.1, 100.7, 53.6, 39.5, 25.6, 22.4.

### Example 125

### 17β-N-1-(4-Chlorophenyl)cyclopentyl-carbamovl-6-azaandrost-4-methyl-4-en-3-one (Compound 125)

Melting Point: 166°C.

| | |
|---|---|
| High res. mass spec. for C₃₁H₄₁N₂O₂Cl | 509.2935. |
| Found | 509.2941. |

### Example 126

### 17β-N-(2,5-bis(Trifluoromethyl))phenyl-carbamoyl-6-azaandrost-4-chloro-4-en-3-one (Compound 126)

Melting Point: 148-154°C.

| | | | |
|---|---|---|---|
| Anal. Calcd. for C₂₇H₂₉N₂O₂ClF₆; | C, 57.48; | H, 5.19; | N, 4.98. |
| Found: | C, 57.43; | H, 5.21; | N, 4.91. |

### Example 127

### 17β-N-(2-t-Butyl-5-trifluoromethyl)phenyl-carbamoyl-6-azaandrost-4-chloro-4-en-3-one (Compound 127)

Melting Point: 174-178°C.

| | | | |
|---|---|---|---|
| Anal. Calcd. for C₃₀H₃₈N₂O₂ClF₃; | C, 65.37; | H, 6.95; | N, 5.08. |
| Found: | C, 65.49; | H, 7.01; | N, 5.05. |

### Example 128

### 17β-N-(2-Methoxy-5-t-butyl)phenyl-carbamoyl-6-azaandrost-4-en-3-one (Compound 128)

Melting Point: 170-175°C.

| | | | |
|---|---|---|---|
| Anal. Calcd. for C₃₀H₄₂N₂O₃•H₂O; | C, 72.55; | H, 8.93; | N, 5.64. |
| Found: | C, 72.50; | H, 8.94; | N, 5.69. |

### Example 129

### 17β-N-(2-Fluoro-5-trifluoromethyl-phenyl-carbamoyl-6-azaandrost-4-en-3-one (Compound 129)

Melting Point: 167-170°C.

| | | | |
|---|---|---|---|
| Anal. Calcd. for C₂₆H₃₀N₂O₂F₄•³/₄H₂O; | C, 63.47; | H, 6.45; | N, 5.69. |
| Found: | C, 63.46; | H, 6.26; | N, 5.76. |

### Example 130

### 17β-N-(1-(4-Biphenyl)-2,2-diethyl)cyclopropyl-carbamoyl-6-azaandrost-4-en-3-one (Compound 130)

Melting Point: 178-182°C.

| | | | |
|---|---|---|---|
| Anal. Calcd. for C₃₈H₄₈N₂O₂; | C, 79.54; | H, 8.61; | N, 4.88. |
| Found: | C, 79.68; | H, 8.55; | N, 4.86. |

1-Amino-1-(1-(4-biphenyl)-2,2-diethyl)cyclopropane: ¹³C NMR (CDCl₃) δ 144.6, 140.9, 139.1, 128.8, 128.7, 127.1, 127.0, 127.0, 46.9, 33.2, 25.6, 24.1, 22.3, 11.6, 10.5.

### Example 131

### 17β-N-(1-(4-Trifluoromethylphenyl)-2,2-diethyl)cyclopropyl-carbamoyl-6-azaandrost-4-en-3-one (Compound 131)

Melting Point: 176-182°C.

| | | | |
|---|---|---|---|
| Anal. Calcd. for C₃₃H₄₃N₂O₂F₃•¹/₃H₂O; | C, 70.44; | H, 7.82; | N, 4.98. |
| Found: | C, 70.44; | H, 7.83; | N, 4.97. |

1-Amino-1-(1-(4-trifluoromethylphenyl)-2,2-diethyl)cyclopropane: ¹³C NMR (CDCl₃) δ 149.5, 149.5, 129.7, 128.8, 128.4, 126.1, 126.0, 125.5, 125.5, 125.4, 125.3, 122.5, 46.9, 33.4, 25.6, 24.3, 22.2, 11.5, 10.4.

### Example 132

### 17β-N-3-(2-Carbomethoxy-5-t-butyl)thiophenyl-carbamoyl-6-azaandrost-4-en-3-one (Compound 132)

Melting Point: 273-275°C.

| | | | |
|---|---|---|---|
| Anal. Calcd. for C₂₉H₄₀N₂O₄S; | C, 67.94; | H, 7.86; | N, 5.46. |
| Found: | C, 67.67; | H, 7.90; | N, 5.42. |

### Example 133

### 17β-N-(2-Phenylsulfonyl)phenyl-carbamoyl-6-azaandrost-4-en-3-one (Compound 133)

Melting Point: 175-180°C.

| | | | |
|---|---|---|---|
| Anal. Calcd. for C₃₁H₃₆N₂O₄S•¹/₄H₂O; | C, 69.31; | H, 6.84; | N, 5.22. |
| Found: | C, 69.18; | H, 7.01; | N, 5.15. |

### Example 134

### 17β-N-(4-Carboethoxy)phenyl-carbamoyl-6-azaandrost-4-en-3-one (Compound 134)

Melting Point: 299-301°C.

| | | | |
|---|---|---|---|
| Anal. Calcd. for C₂₈H₃₆N₂O₄•¹/₄H₂O; | C, 71.69; | H, 7.84; | N, 5.97. |
| Found: | C, 71.74; | H, 7.83; | N, 5.98. |

### Example 135

### 17β-N-(2-(4-t-(-Butyl)phenyl-5-trifluoromethyl)phenyl-carbamoyl-6-azaandrost-4-en-3-one (Compound 135)

Melting Point: 185-189°C.

| | | | |
|---|---|---|---|
| Anal. Calcd. for C₃₆H₄₃N₂O₂F₃; | C, 72.95; | H, 7.31; | N, 4.73. |
| Found: | C, 72.84; | H, 7.36; | N, 4.65. |

### Example 136

### 17β-N-1-(4-t-Butylphenyl)cycloheptyl-carbamoyl-6-azaandrost-4-en-3-one (Compound 136)

Melting Point: 190-194°C.

| | | | |
|---|---|---|---|
| Anal. Calcd. for C₃₆H₅₂N₂O₂•³/₄H₂O; | C, 77.27; | H, 9.65; | N, 5.00. |
| Found: | C, 77.44; | H, 9.66; | N, 5.01. |

1-Amino-1-(4-*t*-butylphenyl)cycloheptane: ¹³C NMR (CDCl₃) δ 148.7, 148.2, 125.0, 124.7, 57.3, 42.9, 34.2, 31.2, 29.7, 23.1.

### Example 137

### 17β-N-(3-Carboxy)phenyl-carbamoyl-6-azaandrost-4-en-3-one (Compound 137)

Melting Point: 258-260°C.

| | | | |
|---|---|---|---|
| Anal. Calcd. for C₂₆H₃₂N₂O₄•HCl•1¹/₄H₂O; | C, 63.02; | H, 7.22; | N, 5.65. |
| Found: | C, 63.02; | H, 7.22; | N, 5.62. |

### Example 138

### 17β-N-1-(9-Fluorenonyl)-carbamoyl-6-azaandrost-4-en-3-one (Compound 138)

Melting Point: 260-264°C.

| | | | |
|---|---|---|---|
| Anal. Calcd. for C₃₂H₃₄N₂O₃•1¹/₄H₂O; | C, 74.32; | H, 7.11; | N, 5.42. |
| Found: | C, 74.49; | H, 7.12; | N, 5.38. |

### Example 139

### 17β-N-1-(4-t-Butylphenyl)cyclohexyl-carbamoyl-6-azaandrost-4-chloro-4-en-3-one (Compound 139)

Melting Point: 158°C.

| | |
|---|---|
| FAB mass spec. for C₃₅H₄₉N₂O₂Cl; | 565.2. |
| Found: | 565.2 M⁺. |

### Example 140

### 17β-N-(2,6-Diethyl-4-(4-chlorophenyl))phenyl-carbamoyl-6-azaandrost-4-en-3-one (Compound 140)

Melting Point: >300°C.

| | | | |
|---|---|---|---|
| Anal. Calcd. for C₃₅H₄₃N₂O₂Cl•¹/₄H₂O; | C, 74.58; | H, 7.78; | N, 4.97. |
| Found: | C, 74.27; | H, 7.80; | N, 4.97. |

### Example 141

### 17β-N-(2-Phenyl)-hydrazidyl-carbamoyl-6-azaandrost-4-en-3-one (Compound 141)

Melting Point: 193-195°C.

| | | | |
|---|---|---|---|
| Anal. Calcd. for C₂₅H₃₃N₃O₂•H₂O; | C, 70.56; | H, 8.29; | N, 9.87. |
| Found: | C, 70.17; | H, 8.26; | N, 9.87. |

### Example 142

### 17β-N-(2-t-Butylcarbamoyl-5-trifluoromethyl)phenyl-carbamoyl-6-azaandrost-4-en-3-one (Compound 142)

Melting Point: 198-200°C.

| | | | |
|---|---|---|---|
| Anal. Calcd. for C₃₁H₄₀N₃O₃F₃; | C, 66.53; | H, 7.20; | N, 7.51. |
| Found: | C, 66.41; | H, 7.32; | N, 7.36. |

### Example 143

### 17β-N-4-(4-t-Butylphenyl)tetrahydrothiopyranyl-carbamoyl-6-azaandrost-4-en-3-one (Compound 143)

Melting Point: 197°C.

| | | | |
|---|---|---|---|
| Anal. Calcd. for C₃₄H₄₈N₂O₂S•³/₄H₂O; | C, 72.62; | H, 8.87; | N, 4.98. |
| Found: | C, 72.46; | H, 8.97; | N, 4.99. |

4-Amino-4-(4-t-butylphenyl)tetrahydrothiopyran: ¹³C NMR (CDCl₃) δ 149.3, 146.7, 125.3, 124.3. 52.6, 39.8, 34.2, 31.3, 24.3.

### Example 144

### 17β-N-9-(4-t-Butylphenyl)bicyclo[3.3.1]nonyl-carbamoyl-6-azaandrost-4-en-3-one (Compound 144)

Melting Point: >260°C.

| | | | |
|---|---|---|---|
| Anal. Calcd. for C₃₈H₅₄N₂O₂•1¹/₄H₂O; | C, 76.92; | H, 9.60; | N, 4.72. |
| Found: | C, 76.82; | H, 9.63; | N, 4.67. |

9-Amino-9-(4-*t*-Butylphenyl)bicyclo[3.3.1]nonane: ¹³C NMR (CDCl₃) δ 148.6, 144.8, 125.4, 125.3, 124.7, 124.3, 64.1, 55.4, 38.9, 35.3, 34.2, 31.7, 31.3, 31.3, 29.1, 27.0, 24.4, 21.1, 20.5.

### Example 145

### 17β-N-4-(4-t-Butylphenyl)tetrahydropyranyl-carbamoyl-6-azaandrost-4-en-3-one (Compound 145)

Melting Point: 181°C.

| | | | |
|---|---|---|---|
| Anal. Calcd. for C₃₄H₄₈N₂O₃•³/₄H₂O; | C, 74.75; | H, 9.13; | N, 5.13. |
| Found: | C, 74.72; | H, 9.11; | N, 5.15. |

4-Amino-4-(4-*t*-butylphenyl)tetrahydropyran: ¹³C NMR (CDCl₃) δ 149.4, 146.5, 125.3, 124.3, 64.1, 51.2, 38.9, 31.3, 22.4.

### Example 146

### 17β-N-1-(4-Chlorophenyl)cyclohexyl-carbamoyl-6-azaandrost-4-en-3-one (Compound 146)

Melting Point: 184°C.

| | | | |
|---|---|---|---|
| Anal. Calcd. for C₃₁H₄₁N₂O₂Cl; | C, 73.13; | H, 8.11; | N, 5.50. |
| Found: | C, 72.74; | H, 8.39; | N, 5.38. |

### Example 147

### 17β-N-(2-t-Butyl-5-(4-t-butyl)phenyl)phenyl-carbamoyl-6-azaandrost-4-en-3-one (Compound 147)

Melting Point: >300

| | | | |
|---|---|---|---|
| Anal. Calcd. for C₃₉H₅₂N₂O₂; | C,78.22; | H,9.09; | N,4.68. |
| Found: | C,77.98; | H,8.99; | N,4.65 |

### Example 148

### 17β-N-1-(4-Chlorophenyl)cyclopentyl-carbamoyl-6-azaandrost-4-chloro-4-en-3-one (Compound 148)

| | |
|---|---|
| FAB mass spec. for C₃₀H₃₈N₂O₂Cl₂; | 529.55 |
| Found: | 529.3M⁺ |

### Example 149

### Pharmaceutical formulations

(A) Transdermal System - For 1000 Patches

| Ingredients | Amount |
|---|---|
| Active compound | 40 g |
| Silicone fluid | 450 g |
| Colloidal silicon dioxide | 25 g |

The silicon fluid and active compound are mixed together and the colloidal silicon dioxide is added to increase viscosity. The material is then dosed into a subsequently heat sealed polymeric laminate comprised of the following: polyester release liner, skin contact adhesive composed of silicone or acrylic polymers, a control membrane which is a polyolefin (e.g. polyethylene, polyvinyl acetate or polyurethane), and an impermeable backing membrane made of a polyester multilaminate. The resulting laminated sheet is then cut into 10 sq. cm patches.
(B) Oral Tablet - For 1000 Tablets

| Ingredients | Amount |
|---|---|
| Active compound | 20 g |
| Starch | 20 g |
| Magnesium Stearate | 1 g |

The active compound and the starch are granulated with water and dried. Magnesium stearate is added to the dried granules and the mixture is thoroughly blended. The blended mixture is compressed into tablets.
(C) Suppository - For 1000 Suppositories

| | Amount |
|---|---|
| Active compound | 25 g |
| Theobromine sodium salicylate | 250 g |
| Witepsol S55 | 1725 g |

The inactive ingredients are mixed and melted. The active compound is then distributed in the molten mixture, poured into molds and allowed to cool.
(D) Injection - For 1000 Ampules

| Ingredients | Amount |
|---|---|
| Active Compound | 5 g |
| Buffering Agents | q.s. |
| Propylene glycol | 400 mg |
| Water for injection | 600 mL |

The active compound and buffering agents are dissolved in the propylene glycol at about 50°C. The water for injection is then added with stirring and the resulting solution is filtered, filled into ampules, sealed and sterilized by autoclaving.
(E) Capsule - For 1000 Capsules

| Ingredients | Amount |
|---|---|
| Active Compound | 20 g |
| Lactose | 450 g |
| Magnesium stearate | 5 g |

The finely ground active compound is mixed with the lactose and stearate and packed into gelatin capsules.

## Claims

1. A compound of formula (I): 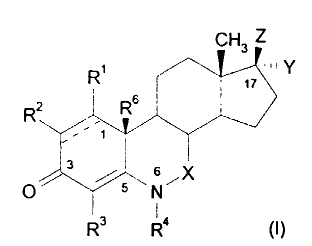 wherein R¹ and R²
i) are independently hydrogen or lower alkyl and the bond between the carbons bearing R¹ and R² is a single or a double bond,
or
ii) taken together are a -CH₂- group forming a cyclopropane ring, and the bond between the carbons bearing R¹ and R² is a single bond;
R³ is hydrogen, -Alk¹-H (optionally substituted with one or more halogens), lower cycloalkyl, lower cycloalkyl-lower alkyl, halogen, -(Alk¹)ₙ-CO₂H, -(Alk¹)ₙ-CO₂R⁷, -(Alk¹)ₙ-Ar¹, -(Alk¹)ₙ-CONR⁸R⁹, -(Alk¹)ₙ-NR⁸R⁹, -(Alk¹)ₙ-S(O)ᵣR⁷, -(Alk¹)ₙ-CN, -(Alk¹)-OH, -(Alk¹)ₙ-COR⁷; or -(Alk¹)ₙ-OR⁷;
wherein
Alk¹ is lower alkylene, lower alkenylene or lower alkynylene,
n is 0 or 1,
r is 0, 1 or 2,
R⁷ is -Alk¹-H, -(Alk¹)ₙ-Ar¹ or lower cycloalkyl,
R⁸ and R⁹ are independently hydrogen, -Alk¹-H or lower cycloalkyl,
Ar¹ is a homocyclic aryl group of 6 to 14 carbons;
R⁴ is hydrogen, -Alk¹-H, lower cycloalkyl, lower cycloalkyl-lower alkyl, -(Alk¹)ₙ-S(O)ᵣR⁷, -(Alk¹)ₙ-phthalimidyl, -(Alk¹)-CO₂H, -(Alk¹)ₙ-CO₂R⁷, -(Alk¹)ₙ-COR⁷, -(Alk¹)ₙ-Ar¹, -(Alk¹)ₙ-CONR⁸R⁹, -(Alk¹)ₙ-NR⁸R⁹, -(Alk¹)ₙ-OH or -(Alk¹)ₙ-OR⁷;
X is, 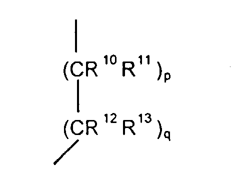 wherein
R¹⁰, R¹¹, R¹² and R¹³ are independently hydrogen or lower alkyl,
p and q are independently either 0 or 1;
and,
i) Y is hydrogen or hydroxy and
Z is -(Alk²)ₙ-COR⁵, -(Alk²)ₙ-CO₂R⁵, -(Alk²)ₙ-COSR⁵,
-(Alk²)ₙ-CONR¹⁴R¹⁵,-(Alk²)-OCO₂R⁵,-(Alk²)-OCOR⁵,
-(Alk²)-OCONR¹⁴R¹⁵, -(Alk²)-OR⁵, -(Alk²)-NR^{5'}COR⁵, -(Alk²)-NR^{5'}CO₂R⁵, -(Alk²)-NR⁵CONR¹⁴R¹⁵, -(Alk²)ₙ-CONR⁵NR¹⁴R¹⁵,
-(Alk²)ₙ-CONR⁵CONR¹⁴R¹⁵, -(Alk²)-NR⁵CSNR¹⁴R¹⁵ or -(Alk²)ₙ-CONR⁵CSNR¹⁴R¹⁵; wherein
Alk² is (C₁₋₁₂) alkylene, (C₂₋₁₂) alkenylene or (C₂₋₁₂) alkynylene, R⁵ and R^{5'} are independently hydrogen, -Alk¹-H (optionally substituted independently with one or more CO₂H, CO₂R⁷, Ar², Ar³ or cyano groups), (Alk¹)ₙ-(lower cycloalkyl (optionally substituted independently with one or more -Alk¹-H groups)), adamantyl, norbornyl, Ar², Ar³, (lower cycloalkyl)-Ar² or (lower cycloalkyl-Ar³;
wherein
Ar² is a homocyclic aromatic group of 6 to 14 carbon ring atoms (optionally substituted independently with one or more -Alk²-H (optionally substituted independently with one or more halogens), -(Alk¹)ₙCOR^{7,} -(Alk¹)ₙ-OH, -(Alk¹)ₙ-OR¹⁶, -(Alk¹)ₙ-Ar³, -(Alk¹)ₙ-CO₂H, -(Alk¹)ₙ-CO₂R⁷, S(O)ᵣR⁷, NR⁸S(O)ᵣR¹⁶, NR⁸R⁹, CONR⁸R⁹, lower cycloalkyl, lower alkoxy, -(Alk¹)ₙ-Ar¹ (optionally substituted with one or more -Alk¹-H or halogen), methylenedioxy, ethylenedioxy, morpholino, thiomorpholino, cyano, nitro or halogens);
wherein
R¹⁶ is -Alk¹-H (optionally substituted independently with one or more halogens), lower cycloalkyl (optionally substituted independently with one or more halogens, or -Alk¹-H (optionally substituted independently with one or more halogens)) or -(Alk¹)ₙ-Ar¹ (wherein Ar¹ is optionally substituted independently with one or more lower alkoxy, cyano, halogen or -Alk¹-H (optionally substituted independently with one or more halogens);
Ar³ is an aromatic group of 5 to 14 ring atoms, at least one of which is O, N or S (optionally substituted independently with one or more -Alk¹-H (optionally substituted independently with one or more halogens), lower cycloalkyl, lower alkoxy, CO₂H, CO₂R⁷, -(Alk¹)ₙ-Ar¹, cyano or halogen);
R¹⁴ and R¹⁵ are
a) independently, hydroxy, hydrogen, -Alk²-H, lower alkoxy, -(Alk¹)ₙ-adamantyl, -(Alk¹)ₙ-myrantyl, -(Alk¹)ₙ-norbornyl, -(Alk¹)ₙ-fluorenyl, -(Alk¹)ₙ-fluorenonyl, -(Alk¹)ₙ-indanyl (optionally substituted with one or more -Alk¹-H), -Alk¹-H (optionally substituted independently with one or more halogens, cyano, cycloalkyl, SR⁵, COR⁵, CONR⁵R⁷, NR^{5'}COR⁵, NR^{5'}CO₂R⁵, NR^{5'}CONHR⁵, CO₂R⁵, OR⁵, Ar² or Ar³), Ar² or Ar³ or a saturated C₄₋₁₈ bicyclic ring or C₃₋₁₁ saturated ring, optionally containing an oxygen or sulfur atom (said rings optionally substituted independently with one or more cyano, R¹⁶, Ar² or Ar³);
b) alkylene groups (optionally substituted with one or more R⁷ groups, taken together with the linking nitrogen to form a 4 to 8 atom heterocyclic group) 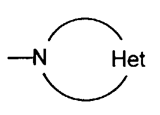 wherein;
Het represents -O-, -CH₂-, -S(O)ᵣ, -(NH)- or -(N(Alk¹-H))-;
with the proviso that
when Z is -(Alk²)ₙ-COR⁵, -(Alk²)ₙ-CO₂R⁵ or -(Alk²)ₙ-CO-thiopyridyl and R⁵ is hydrogen, -Alk¹-H, lower cycloalkyl, or adamantyl or when Z is -(Alk²)ₙ-CONR¹⁴R¹⁵ and R¹⁴ and R¹⁵ are
a) independently hydrogen, -Alk²-H, lower cycloalkyl, lower alkoxy, adamantyl, -Ar¹, benzyl, diphenylmethyl, triphenylmethyl or -(Alk¹)ₙ-norbornyl;
or
b) carbon atoms, optionally substituted with one or more lower alkyl groups, taken together with the linking nitrogen to form a 4 to 8 atom heterocylic ring as herein before defined,
Y is hydroxy; or
ii) Y is hydrogen and
Z is OR⁵, OCOR⁵, OCONR¹⁴R¹⁵, NR^{5'}COR⁵, NR^{5'}CO₂R⁵,
NR⁵CONR¹⁴R¹⁵ or NR⁵CSR¹⁴R¹⁵; and
iii) Y and Z taken together are
=O, =CH-(Alk¹)ₙ-COR⁵, =CH-(Alk¹)ₙ-CO₂R⁵ or
=CH-(Alk¹)ₙ-CONR¹⁴R¹⁵;
R⁶ is hydrogen or methyl;
or a pharmaceutically acceptable salt or solvate thereof.

2. A compound of Claim 1 which is a compound of formula (IA), (IB), (IC) or (ID)

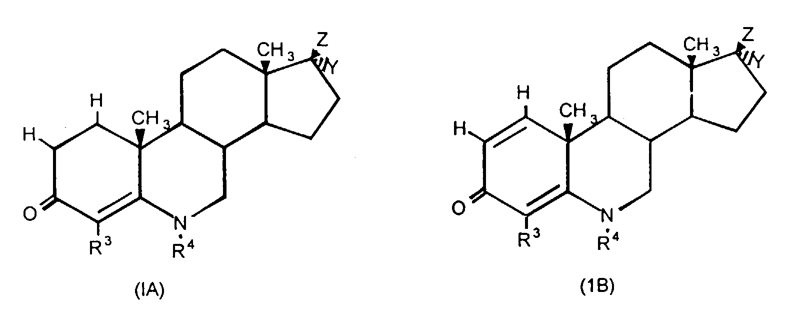

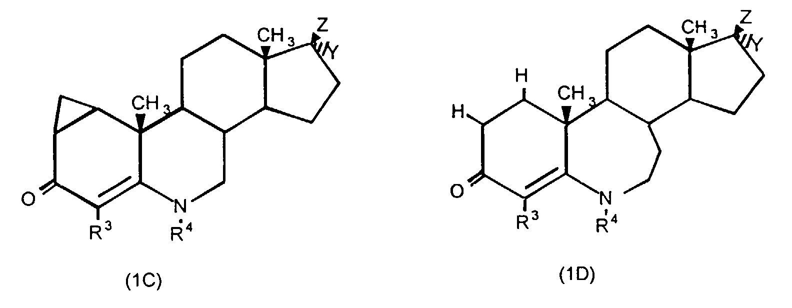

3. A compound as claimed in Claim 1 or Claim 2 wherein R³ is hydrogen, halogen, lower alkyl, lower cycloalkyl or lower cycloalkyl-lower alkyl.

4. A compound as claimed in Claim 3 wherein R³ is hydrogen, halogen or lower alkyl.

5. A compound of Claim 1 or Claim 2 wherein R³ is methyl, ethyl, cyano, iodo, bromo, chloro or dimethylaminomethyl.

6. A compound as claimed in any one of the preceding claims wherein R⁴ is hydrogen, lower alkyl, lower cycloalkyl or lower cycloalkyl-lower alkyl.

7. A compound as claimed in Claim 6 wherein R⁴ is hydrogen or lower alkyl.

8. A compound as claimed in any one of claims 1 to 5 wherein R⁴ is methyl, ethyl, propyl, *i*-propyl, butyl, *i*-butylhexyl, 3-hydroxypropyl, propenyl, methylene-cyclopropyl, benzyl, 2-methoxyethyl, 2-acetic acid, 3-proponic acid, 5-pentanoic acid, 6-hexanoic acid, methyl-5-pentanoate, ethyl-6-hexanoate, 3-phthalimidylpropyl and 4-phthalimidylpropyl.

9. A compound according to any one of the preceding claims wherein X is -CH₂-.

10. A compound as claimed in any one of the preceding claims wherein Y is hydrogen and Z is -(Alk²)ₙ-COR⁵, -(Alk²)ₙ-CONR¹⁴R¹⁵, -(Alk²)-OCO₂R⁵, -(Alk²)-OCOR⁵, -(Alk²)-NR^{5'}COR⁵, -(Alk²)ₙ-CONR⁵NR¹⁴R¹⁵, -(Alk²)-NR⁵CONR¹⁴R¹⁵, or -(Alk²)ₙ-CONR⁵CONR¹⁴R¹⁵.

11. A compound as claimed in Claim 10 wherein Z is -COR⁵, -CONR¹⁴R¹⁵, -CH₂OCO₂R⁵, -CH₂OCOR⁵, -CH₂NR^{5'}COR⁵, -CONR⁵NR¹⁴R¹⁵, -CH₂NR⁵CONR¹⁴R¹⁵ or -CONR⁵CONR¹⁴R¹⁵.

12. A compound as claimed in Claim 11 wherein Z is -CONR¹⁴R¹⁵.

13. A compound as claimed in any one of the preceding claims wherein Ar¹ is a phenyl group.

14. A compound as claimed in any one of the preceding claims wherein Ar² is a phenyl group optionally substituted independently with one or more -Alk²-H (optionally substituted independently with one or more halogens), -OR¹⁶, -S(O)ᵣR⁷, -Ar¹, methylenedioxy, ethylenedioxy, morpholino, thiomorpholino, cyano, nitro or halogen groups.

15. A compound as claimed in any one of the preceding claims wherein R¹⁴ is hydrogen or hydroxy, and R¹⁵ is hydrogen, lower cycloalkyl (optionally substituted independently with one or more R⁷ or Ar² groups), -(Alk¹)-adamantyl, -(Alk¹)ₙ-myrantyl, -(Alk¹)ₙ-norbornyl, -(Alk¹)ₙ-fluorenyl, -(Alk¹)ₙ-indanyl, -Alk¹-H (optionally substituted independently with one or more lower cycloalkyl, SR⁵, OR⁵, Ar² or Ar³ groups), Ar² or Ar³; or
R¹⁴ and R¹⁵ are carbon atoms, optionally substituted with one or more R⁷ groups, taken together with the linking nitrogen to form a 5 to 7 atom heterocyclic group 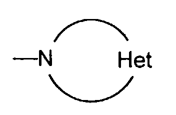 wherein Het represents -CH₂-.

16. A compound as claimed in any one of the preceding claims wherein Ar³ is an aromatic group of five or six ring atoms, at least one of which is O, N or S, optionally substituted independently with one or more Alk¹H groups.

17. A compound as claimed in Claim 16 wherein Ar³ is an optionally substituted pyrrolyl, thienyl, furyl or pyridyl group.

18. A compound as claimed in any one of the preceding claims wherein R⁵ is hydrogen, lower alkyl optionally substituted independently with one or more Ar² groups, (lower alkyl)n-lower cycloalkyl, menthyl, adamantyl, norbornyl or Ar².

19. A compound as claimed in any one of Claims 1 to 11 or 13 to 18 wherein Z is -COR⁵.

20. A compound as claimed in Claim 19 which is:
17β-(1-Oxo-2-cyclohexylethyl)-6-azaandrost-4-en-3-one;
17β-(1-Oxo-1-(2,4-difluorophenyl)methyl)-6-azaandrost-4-en-3-one;
17β-(1-Oxo-1-(4-isopropoxyphenyl)methyl)-6-azaandrost-4-en-3-one;
17β-(1-Oxo-3,3-diphenylpropyl)-6-azaandrost-4-en-3-one; or
17β-(1-Oxo-1-(2-norbornyl)methyl)-6-azaandrost-4-en-3-one or
a pharmaceutically acceptable salt or solvate thereof.

21. A compound as claimed in any one of Claims 1 to 18 wherein Z is -CONR¹⁴R¹⁵, R¹⁴ is hydrogen and R¹⁵ is Ar² or a C₃₋₁₁ saturated ring, optionally containing an oxygen or sulphur atom (optionally substituted independently with one or more R⁷ or Ar² groups).

22. A compound as claimed in any one of the preceding claims wherein R¹⁵ is a group of formula Ar^{2a} 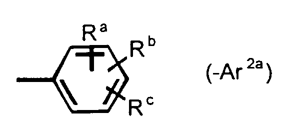 wherein R^{a} and R^{b} are independently hydrogen, lower alkyl, trifluoromethyl, halogen or phenyl (optionally substituted with one or more halogens or branched C₄₋₇alkyl) and R^{c} is hydrogen or one or more halogens, or R¹⁵ is a C₃₋₁₁ saturated ring, optionally containing an oxygen or sulphur atom substituted with a group of formula Ar^{2a}.

23. A compound as claimed in Claim 22 wherein Ar^{2a} is a group of formula Ar^{2aa} 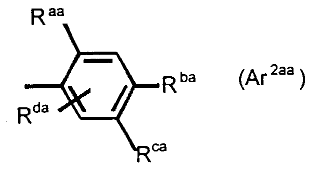 where R^{aa} is branched C₄₋₇alkyl, trifluoromethyl or phenyl optionally substituted with one or more halogens; one of R^{ba} and R^{ca} is branched C₄₋₇alkyl, trifluoromethyl, halogen or phenyl optionally substituted with one or more halogens, and the other is hydrogen or halogen; and R^{da} is hydrogen or halogen.

24. A compound as claimed in Claim 1 which is:
17β-N-((2,6-Di-*i*-propyl)phenyl)-carbamoyl-6-azaandrost-4-en-3-one
17β-N-(2,4,6-trimethyl)phenyl-carbamoyl-6-azaandrost-4-en-3-one
17β-N-(2-Chloro-5-trifluoromethyl)phenyl-carbamoyl-6-azaandrost-4-en-3-one
17β-N-(2,6-Dimethyl-4-bromo)phenyl-carbamoyl-6-azaandrost-4-en-3-one
17β-N-(2,6-Dimethyl-4-*t*-butyl)phenyl-carbamoyl-6-azaandrost-4-en-3-one
17β-N-(2,6-Dibromo-4-isopropyl)phenyl-carbamoyl-6-azaandrost-4-en-3-one
17β-N-(2,5-Ditrifluoromethyl)phenyl-carbamoyl-6-azaandrost-4-en-3-one
17β-N-(2-Phenyl)phenyl-carbamoyl-6-azaandrost-4-en-3-one
17β-N-(2,6-Diethyl-3,5-dichloro)phenyl-carbamoyl-6-azaandrost-4-en-3-one
17β-N-(2,6-Diethyl-3-chloro)phenyl-carbamoyl-6-azaandrost-4-en-3-one
17β-N-(2-*t*-Butyl)phenyl-carbamoyl-6-azaandrost-4-en-3-one
17β-N-(2,4,6-Trichloro)phenyl-carbamoyl-6-azaandrost-4-en-3-one
17β-N-(2-Bromo-5-trifluoromethyl)phenyl-carbamoyl-6-azaandrost-4-en-3-one
17β-N-(2-*t*-Butyl-6-methyl)phenyl-carbamoyl-6-azaandrost-4-en-3-one
17β-N-1-(4-Chlorophenyl)cyclopentyl-carbamoyl-6-azaandrost-4-en-3-one
17β-N-(2,6-Dibromo-4-chloro)phenyl-carbamoyl-6-azaandrost-4-en-3-one
17β-N-(2,6-Diethyl-4-bromo)phenyl-carbamoyl-6-azaandrost-4-en-3-one
17β-N-(2-Bromo-4-*t*-butyl)phenyl-carbamoyl-6-azaandrost-4-en-3-one
17β-N-(2-Chloro-4-*t*-butyl)phenyl-carbamoyl-6-azaandrost-4-en-3-one
17β-N-(5-Bromo-2-*t*-butyl)phenyl-carbamoyl-6-azaandrost-4-en-3-one
17β-N-(5-Chloro-2-*t*-butyl)phenyl-carbamoyl-6-azaandrost-4-en-3-one
17β-N-(2,6-Diethyl)phenyl-carbamoyl-6-azaandrost-4-en-3-one
17β-N-(4-Bromo-2-*t*-butyl)phenyl-carbamoyl-6-azaandrost-4-en-3-one
17β-N-(2-*t*-Butyl-5-cyano)phenyl-carbamoyl-6-azaandrost-4-en-3-one
17β-N-(2-(O-4-Tolyl)-5-trifluoromethyl)phenyl-carbamoyl-6-azaandrost-4-en-3-one
17β-N-(2-(O-4-Chlorophenyl)-5-trifluoromethyl)phenyl-carbamoyl-6-azaandrost-4-en-3-one
17β-N-(2-Nitro-4-*t*-butyl)phenyl-carbamoyl-6-azaandrost-4-en-3-one
17β-N-(2-(O-Phenyl)-5-(1,1-dimethyl)propyl)phenyl-carbamoyl-6-azaandrost-4-en-3-one
17β-N-(2-Ethylsulfonyl-5-trifluoromethyl)phenyl-carbamoyl-6-azaandrost-4-en-3-one
17β-N-(3,5-Di-*t*-butyl)phenyl-carbamoyl-6-azaandrost-4-en-3-one
17β-N-(2-*t*-Butyl-5-trifluoromethyl)phenyl-carbamoyl-6-azaandrost-4-en-3-one
17β-N-(2-*t*-Butyl-5-phenyl)phenyl-carbamoyl-6-azaandrost-4-en-3-one
17β-N-(2,6-Di-*i*-propyl)phenyl-carbamoyl-6-azaandrost-1,4-dien-3-one
17β-N-(2,6-Di-*i*-propyl)phenyl-carbamoyl-6-azaandrost-4-methyl-1,4-dien-3-one
17β-N-1-(4-Trifluoromethylphenyl)cyclopentyl-carbamoyl-6-azaandrost-4-en-3-one
17β-N-1-(4-Fluorophenyl)cyclohexyl-carbamoyl-6-azaandrost-4-en-3-one
17β-N-1-(4-Methoxyphenyl)cyclohexyl-carbamoyl-6-azaandrost-4-en-3-one
17β-N-1-(4-Methoxyphenyl)cyclopentyl-carbamoyl-6-azaandrost-4-en-3-one
17β-N-(2,5-bis(Trifluoromethyl ))phenyl-carbamoyl-6-azaandrost-4-methyl-4-en-3-one
17β-N-(2-t-Butyl-5-trifluoromethyl )phenyl-carbamoyl-6-azaandrost-4-methyl-4-en-3-one
17β-N-(2,5-Di-t-butyl )phenyl-carbamoyl-6-azaand rost-4-methyl-4-en-3-one
17β-N-(2-t-Butyl-5-(4-chlorophenyl))phenyl-carbamoyl-6-azaandrost-4-en-3-one
17β-N-1-(4-t-Butylphenyl )cyclopentyl-carbamoyl-6-azaandrost-4-en-3-one
17β-N-1-(4-t-Butyl phenyl )cyclohexyl-carbamoyl-6-azaandrost-4-en-3-one
17β-N-1-(4-Chlorophenyl)cyclopentyl-carbamoyl-6-azaandrost-4-methyl-4-en-3-one
17β-N-(2,5-bis(Trifluoromethyl))phenyl-carbamoyl-6-azaandrost-4-chloro-4-en-3-one
17β-N-(2-*t*-Butyl-5-trifluoromethyl)phenyl-carbamoyl-6-azaandrost-4-chloro-4-en-3-one
17β-N-1-(4-*t*-Butylphenyl)cycloheptyl-carbamoyl-6-azaandrost-4-en-3-one
17β-N-1-(4-*t*-Butylphenyl )cyclohexyl-carbamoyl-6-azaandrost-4-chloro-4-en-3-one
17β-N-(2,6-Diethyl-4-(4-chlorophenyl))phenyl-carbamoyl-6-azaandrost-4-en-3-one
17β-N-4-(4-*t*-Butylphenyl )tetrahydrothiopyranyl-carbamoyl-6-azaandrost-4-en-3-one
17β-N-9-(4-*t*-Butylphenyl)bicyclo[3.3.1]nonyl-carbamoyl-6-azaandrost-4-en-3-one
17β-N-4-(4-*t*-Butylphenyl)tetrahydropyranyl-carbamoyl-6-azaandrost-4-en-3-one
17β-N-1-(4-Chlorophenyl)cyclohexyl-carbamoyl-6-azaandrost-4-en-3-one
17β-N-(2-*t*-Butyl-5-(4-*t*-butyl)phenyl)phenyl-carbamoyl-6-azaand rost4-en-3-one or
17β-N-1-(4-Chlorophenyl)cyclopentyl-carbamoyl-6-azaandrost-4-chloro-4-en-3-one
or a pharmaceutically acceptable salt or solvate thereof.

25. 17β-N-(2-*t*-Butyl-5-trifluoromethyl)phenyl-carbamoyl-6-azaandrost-4-en-3-one
or a pharmaceutically acceptable salt or solvate thereof.

26. A compound of formula (I) 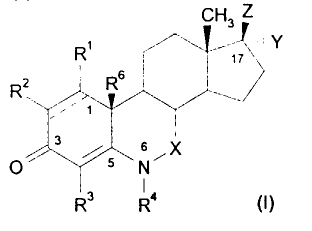
wherein R¹ and R² are,
i) independently hydrogen or lower alkyl and the bond between the carbons bearing R¹ and R² is a single or a double bond, or
ii) taken together are a -CH₂- group to form a cyclopropane ring, and the bond between the carbons bearing R¹ and R² is a single bond;
R³ is hydrogen or lower alkyl;
R⁴ is hydrogen or lower alkyl;
X is, 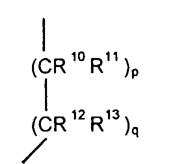 wherein
R¹⁰, R¹¹, R¹² and R¹³ are independently hydrogen or lower alkyl, p and q are independently either 0 or 1;
Y is hydrogen; and
Z is -COR⁵, -CONR¹⁴R¹⁵, -CH₂OCO₂R⁵, -CH₂OCOR⁵, -CH₂NR^{5'}COR⁵, -CONR⁵NR¹⁴R¹⁵, -CH₂NR⁵CONR¹⁴R⁵, or -CON⁵CONR¹⁴R¹⁵ wherein R⁵ and R^{5'} are independently hydrogen, lower alkyl optionally substituted independently with one or more Ar² groups (lower alkyl)ₙ-lower cycloalkyl, menthyl, adamantyl, norbornyl or Ar²;
Ar² is a phenyl group optionally substituted independently with one or more -Alk-H (optionally substituted independently with one or more halogens), -OR¹⁶, -S(O)ᵣR⁷, phenyl, methylenedioxy, ethylenedioxy, morpholino, thiomorpholino, cyano, nitro or halogen groups;
Alk is lower alkylene, lower alkenylene or lower alkynylene;
n is 0 or 1;
r is 0, 1 or 2;
R⁷ is -Alk-H, -(Alk)ₙ-phenyl or lower cycloalkyl;
R¹⁶ is -Alk-H (optionally substituted independently with one or more halogens), lower cycloalkyl (optionally substituted independently with one or more halogens or -Alk-H (optionally substituted independently with one or more halogens)) or -(Alk)ₙ-phenyl (wherein phenyl is optionally substituted independently with one or more lower alkoxy, cyano, halogen or -Alk-H groups (optionally substituted independently with one or more halogens)); R¹⁴ is hydrogen or hydroxy, and R¹⁵ is hydrogen, lower cycloalkyl (optionally substituted independently with one or more R⁷ or Ar² groups), -(Alk)-adamantyl, -(Alk)ₙ-myrantyl, -(Alk)ₙ-norbornyl, (Alk)ₙ-fluorenyl, -(Alk)ₙ-indanyl, -Alk-H (optionally substituted independently with one or more lower cycloalkyl, SR⁵, OR⁵, Ar² or Ar³ groups), Ar² or Ar³, or R¹⁴ and R¹⁵ taken together with the linking nitrogen form a pyrrolidinyl, piperidinyl or perhydroazepinyl ring optionally substituted with one or more R⁷ groups; Ar³ is a pyrrolyl, thienyl, furyl or pyridyl group optionally substituted independently with one or more Alk-H groups; with the provisos that
when Z is COR⁵, R⁵ is substituted lower alkyl, lower alkyl lower cycloalkyl, menthyl, norbornyl or Ar²;
when Z is CONR¹⁴R¹⁵ and R¹⁵ is hydrogen, -Alk-H, lower cycloalkyl, lower alkoxy, adamantyl, phenyl, benzyl, diphenylmethyl, triphenylmethyl or -(Alk)ₙ-norbornyl, R¹⁴ is hydroxy; and
when Z is CONR¹⁴R¹⁵ and R¹⁴ and R¹⁵ taken together with the linking nitrogen form a pyrrolidinyl, piperidinyl or perhydroazepinyl ring, said ring is substituted with one or more lower alkenyl, lower alkynyl, -(Alk)n-phenyl or lower cycloalkyl groups; or a pharmaceutically acceptable salt or solvate thereof.

27. A compound of formula (I) 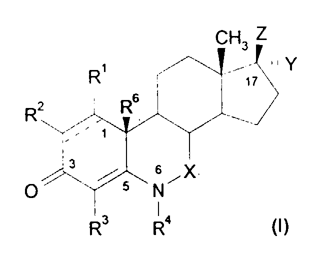 wherein:
R¹ and R² are,
i) independently hydrogen or lower alkyl and the bond between the carbons bearing R¹ and R² is a single or a double bond, or
ii) taken together are a -CH₂- group to form a cyclopropane ring, and the bond between the carbons bearing R¹ and R² is a single bond;
R³ is
hydrogen, -Alk¹-H, -Alk¹-H substituted with one or more halogens, lower cycloalkyl, lower cycloalkyl-lower alkyl, halogen,-(Alk¹)ₙ-CO₂H, -(Alk¹)ₙ-CO₂R⁷, -(Alk¹)ₙ-Ar¹, -(Alk¹)ₙ-CONR⁸R⁹, -(Alk¹)ₙ-NR⁸R⁹, -(Alk¹)ₙ-S(O)ᵣR⁷, -(Alk¹)ₙ-CN, -(Alk¹)-OH or -(Alk¹)ₙ-OR⁷; wherein
Alk¹ is lower alkylene, lower alkenylene or lower alkynylene,
n is 0 or 1,
r is 0, 1 or 2,
R⁷ is -Alk¹-H, -(Alk¹)ₙ-Ar¹ or lower cycloalkyl,
R⁸ and R⁹ are independently hydrogen, -Alk¹-H or lower cycloalkyl,
Ar¹ is a homocyclic aryl group of 6 to 14 carbons;
R⁴ is
hydrogen, -Alk¹-H, lower cycloalkyl, lower cycloalkyl-lower alkyl, -(Alk¹)ₙ-S(O)ᵣR⁷, -(Alk¹)ₙ-phthalimidyl, (-Alk¹-)CO₂H, -(Alk¹)ₙ-CO₂R⁷, -(Alk¹)ₙ-Ar¹, -(Alk¹)ₙ-CONR⁸R⁹, -(Alk¹)ₙ-NR⁸R⁹, -(Alk¹)ₙ-OH or -Alk¹)ₙ-OR⁷; X is, 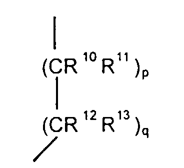 wherein
R¹⁰, R¹¹, R¹² and R¹³ are independently hydrogen or lower alkyl, p and q are independently either 0 or 1;
Y and Z are,
i) Y is hydrogen or hydroxy and
Z is -(Alk²)ₙ-COR⁵, -(Alk²)ₙ-CO₂R⁵, -(Alk²)ₙ-COSR⁵, -(Alk²)ₙ-CONR¹⁴R¹⁵, -(Alk²)-OCOR⁵, -(Alk²)-OCONR¹⁴R¹⁵, -(Alk²)-OR⁵, -(Alk²)-NR⁵COR⁵, -(Alk²)-NR⁵CO₂R⁵, -(Alk2)-NR⁵CONR¹⁴R¹⁵, -(Alk²)-ₙCONR⁵NR¹⁴R¹⁵, -(Alk²)ₙ-CONR⁵CONR¹⁴R¹⁵, (-Alk²-)NR⁵CSNR¹⁴R¹⁵ or -(Alk²)ₙ-CONR⁵CSNR¹⁴R¹⁵; wherein
Alk² is (C₁₋₁₂) alkylene, (C₂₋₁₂) alkenylene or (C₂₋₁₂) alkynylene,
R⁵ is hydrogen, -Alk¹-H, -(Alk¹)ₙ-(lower cycloalkyl), adamantyl, -(Alk¹)ₙ-Ar², -(Alk¹)ₙ-Ar³, (lower cycloalkyl)ₙAr², (lower cycloalkyl)ₙAr³ or -Alk¹- substituted independently with one or more CO₂H, CO₂R⁷, Ar² or Ar³ groups;
wherein
Ar² is an aromatic group of 6 to 14 carbon ring atoms, optionally substituted with one or more Alk¹, Alk¹ substituted with one or more halogens, -(Alk¹)ₙ-OH, -(Alk¹)ₙ-OR⁷, -(Alk¹)ₙ-CO₂H, -(Alk¹)ₙ-CO₂R⁷, S(O)ᵣR⁷ or NR⁸R⁹, lower cycloalkyl, lower alkoxy, -(Alk¹)ₙ-Ar¹, methylenedioxy, ethylenedioxy, morpholino, thiomorpholino, cyano or halogen groups;
Ar³ is an aromatic group of 5 to 14 ring atoms, at least one of which is O, N or S, optionally substituted with one or more Alk¹, lower cycloalkyl, lower alkoxy, CO₂H, CO₂R⁷, -(Alk¹)ₙ-Ar¹, cyano or halogen groups;
R¹⁴ and R¹⁵ are,
a) independently, hydrogen or -Alk²-H, lower cycloalkyl, lower alkoxy, adamantyl, -(Alk¹)ₙ-norbornyl, Ar², Ar³, -Alk¹- substituted independently with one or more SR⁵, COR⁵, CONR⁵R⁷, NR⁵COR⁵, NR⁵CO₂R⁵, NR⁵CONHR⁵, CO₂R⁵, OR⁵, Ar² or Ar³ groups, -(Alk¹)ₙ-fluorenyl, or -(Alk¹)ₙ-indanyl; or
b) carbon atoms, optionally substituted with one or more lower alkyl groups, taken together with the linking nitrogen to form a 4 to 8 atom heterocyclic group 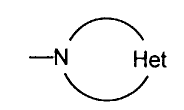 wherein;
Het represents -O-, -CH₂-, -S(O)ᵣ, -(NH)- or -(N(Alk¹))-;
with the proviso that when Z is (-Alk²-)ₙCOR⁵, (-Alk²-)ₙCO₂R⁵ or -(Alk²)ₙ-CO-thiopyridyl and R⁵ is hydrogen, -Alk¹-H, lower cycloalkyl, -(Alk¹)ₙ-Ar¹, adamantyl or
when Z is (-Alk²-)ₙCONR¹⁴R¹⁵ and R¹⁴ and R¹⁵ are
(a) independently hydrogen, -Alk²-H, lower cycloalkyl, lower alkoxy, adamantyl, -Ar¹, benzyl, diphenylmethyl, triphenylmethyl or -(Alk¹)ₙ-norbornyl; or
(b) carbon atoms, optionally substituted with one or more lower alkyl groups, taken together with the linking nitrogen to form a 4 to 8 atom heterocylic ring as herein before defined,
Y is hydroxy;
ii) Y is hydrogen and
Z is OR⁵, OCOR⁵, OCONR¹⁴R¹⁵, NR⁵COR⁵, NR⁵CO₂R⁵, NR⁵CONR¹⁴R¹⁵ or NR⁵CSR¹⁴R¹⁵;
iii) Y and Z taken together are =O, =CH-(Alk¹)ₙ-COR⁵,
=CH-(Alk¹)ₙ-CO₂R⁵ or =CH-(Alk¹)ₙ-CONR¹⁴R¹⁵;
R⁶ is hydrogen or methyl;
and pharmaceutically acceptable salts thereof.

28. A compound of formula (I): 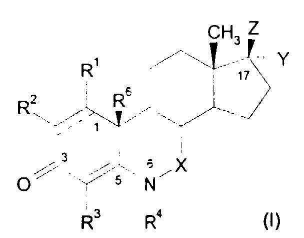 wherein
R¹ and R²
i) are independently hydrogen or lower alkyl and the bond between the carbons bearing R¹ and R² is a single or a double bond,
or
ii) taken together are a -CH₂- group forming a cyclopropane ring, and the bond between the carbons bearing R¹ and R² is a single bond;
R³ is,
hydrogen, -Alk¹-H (optionally substituted with one or more halogens), lower cycloalkyl, lower cycloalkyl-lower alkyl), halogen, -(Alk¹)ₙ-CO₂H, -(Alk¹)ₙ-CO₂R⁷, -(Alk¹)ₙ-Ar¹, -(Alk¹)ₙ-CONR⁸R⁹, -(Alk¹)ₙ-NR⁸R⁹, -(Alk¹)ₙ-S(O)ᵣR⁷, -(Alk¹)ₙ-CN, -(Alk¹)-OH, -(Alk¹)ₙ-COR⁷ or -(Alk¹)ₙ-OR⁷;
wherein
Alk¹ is lower alkylene, lower alkenylene or lower alkynylene,
n is 0 or 1,
r is 0, 1 or 2,
R⁷ is -Alk¹-H, -(Alk¹)ₙ-Ar¹ or lower cycloalkyl,
R⁸ and R⁹ are independently hydrogen, -Alk¹-H or lower cycloalkyl, Ar¹ is a homocyclic aryl group of 6 to 14 carbons;
R⁴ is,
hydrogen, -Alk¹-H, lower cycloalkyl, lower cycloalkyl-lower alkyl, -(Alk¹)ₙ-S(O)ᵣR⁷, -(Alk¹)ₙ-phthalimidyl, -(Alk¹)-CO₂H, -(Alk¹)ₙ-CO₂R⁷, -(Alk¹)ₙ-COR⁷, -(Alk¹)ₙ-Ar¹, -(Alk¹)ₙ-CONR⁸R⁹, -(Alk¹)ₙ-NR⁸R⁹, -(Alk¹)ₙ-OH or -(Alk¹)ₙ-OR⁷;
X is, 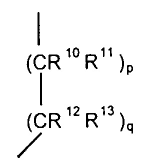 wherein
R¹⁰, R¹¹, R¹² and R¹³ are independently hydrogen or lower alkyl, p and q are independently either 0 or 1;
and,
i) Y is hydrogen or hydroxy and
Z is -(Alk²)ₙ-COR⁵, -(Alk²)ₙ-CO₂R⁵, -(Alk²)ₙ-COSR⁵, -(Alk²)ₙ-CONR¹⁴R¹⁵,-(Alk²)-OCO₂R⁵,-(Alk²)-OCOR⁵, -(Alk²)-OCONR¹⁴R¹⁵, -(Alk²)-OR⁵, -(Alk²)-NR^{5'}COR⁵, -(Alk²)-NR^{5'}CO₂R⁵, -(Alk2)-NR⁵CONR¹⁴R¹⁵, -(Alk²)ₙ-CONR⁵NR¹⁴R¹⁵, (Alk²)ₙ-CONR⁵CONR¹⁴R¹⁵, -(Alk²)-NR⁵CSNR¹⁴R¹⁵ or -(Alk²)ₙ-CONR⁵CSNR¹⁴R¹⁵;
wherein
Alk² is (C₁₋₁₂) alkylene, (C₂₋₁₂) alkenylene or (C₂₋₁₂) alkynylene, R⁵ and R^{5'} are independently hydrogen, -Alk¹-H (optionally substituted independently with one or more CO₂H, CO₂R⁷, Ar², Ar³ or cyano groups), -(Alk¹)ₙ-(lower cycloalkyl (optionally substituted independently with one or more -Alk¹-H groups)), adamantyl, norbornyl, Ar², Ar3, (lower cycloalkyl)-Ar² or (lower cycloalkyl)-Ar³;
wherein
Ar² is a homocyclic aromatic group of 6 to 14 carbon ring atoms (optionally substituted independently with one or more -Alk²-H (optionally substituted independently with one or more halogens), -(Alk¹)ₙ-OH, -(Alk¹)ₙ-OR¹⁶, -(Alk¹)ₙ-Ar³, -(Alk¹)ₙ-CO₂H, -(Alk¹)ₙ-CO₂R⁷, S(O)ᵣR⁷, NR⁸S(O)ᵣR¹⁶, NR⁸R⁹, CONR⁸R⁹, lower cycloalkyl, lower alkoxy, -(Alk¹)ₙ-Ar¹, methylenedioxy, ethylenedioxy, morpholino, thiomorpholino, cyano, nitro or halogens);
wherein
R¹⁶ is -Alk¹-H (optionally substituted independently with one or more halogens), lower cycloalkyl (optionally substituted independently with one or more halogens, or -Alk¹-H (optionally substituted independently with one or more halogens)) or -(Alk¹)ₙ-Ar¹ (wherein Ar¹ is optionally substituted independently with one or more, lower alkoxy, cyano groups, halogens or -Alk¹-H (optionally substituted independently with one or more halogens));
Ar³ is an aromatic group of 5 to 14 ring atoms, at least one of which is O, N or S, (optionally substituted independently with one or more -Alk¹-H (optionally substituted independently with one or more halogens), lower cycloalkyl, lower alkoxy, CO₂H, CO₂R⁷, -(Alk¹)ₙ-Ar¹, cyano or halogen);
R¹⁴ and R¹⁵ are,
a) independently, hydroxy, hydrogen, -Alk²-H, lower cycloalkyl (optionally substituted independently with one or more cyano, R¹⁶, Ar², Ar³), lower alkoxy, -(Alk¹)ₙ-adamantyl, -(Alk¹)ₙ-myrantyl, -(Alk¹)ₙ-norbornyl, -(Alk¹)ₙ-fluorenyl, -(Alk¹)ₙ-indanyl, -Alk¹- H (optionally substituted independently with one or more, halogens, cyano, cycloalkyl, SR⁵, COR⁵, CONR⁵R⁷, NR^{5'}COR⁵, NR^{5'}CO₂R⁵, NR^{5'}CONHR⁵, CO₂R⁵, OR⁵, Ar² or Ar³), Ar² or Ar³;
b) alkylene groups (optionally substituted with one or more R⁷ groups, taken together with the linking nitrogen to form a 4 to 8 atom heterocyclic group) 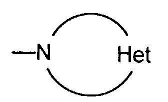 wherein;
Het represents -O-, -CH₂-, -S(O)ᵣ, -(NH)- or -(N(Alk¹-H))-; with the proviso that
when Z is
-(Alk²)ₙ-COR⁵, -(Alk²)ₙ-CO₂R⁵ or -(Alk²)ₙ-CO-thiopyridyl and
R⁵ is
hydrogen, -Alk¹-H, lower cycloalkyl, -(Alk¹)ₙ-Ar¹, adamantyl or
when Z is
-(Alk²)ₙ-CONR¹⁴R¹⁵ and
R¹⁴ and R¹⁵ are
a) independently hydrogen, -Alk²-H, lower cycloalkyl, lower alkoxy, adamantyl, -Ar¹, benzyl, diphenylmethyl, triphenylmethyl or -(Alk¹)ₙ-norbornyl;
or
b) carbon atoms, optionally substituted with one or more lower alkyl groups, taken together with the linking nitrogen to form a 4 to 8 atom heterocylic ring as herein before defined,
Y is hydroxy; or
ii) Y is hydrogen and
Z is
OR⁵, OCOR⁵, OCONR¹⁴R¹⁵, NR^{5'}COR⁵, NR^{5'}CO₂R⁵, NR⁵CONR¹⁴R¹⁵ or NR⁵CSR¹⁴R¹⁵; and
iii) Y and Z taken together are
=O, =CH-(Alk¹)ₙ-COR⁵, =CH-(Alk¹)ₙ-CO₂R⁵ or =CH-(Alk¹)ₙ-CONR¹⁴R¹⁵;
R⁶ is,
hydrogen or methyl;
and pharmaceutically acceptable salts thereof.

29. A pharmaceutical composition comprising a compound of formula (I) as defined in any one of Claims 1 to 28 together with a pharmaceutically acceptable carrier.

30. An in vitro method of inhibiting testosterone-5a-reductases comprising contacting testosterone-5α-reductases with a compound of formula (I) as defined in any one of Claims 1 to 28.

31. Use of a compound of formula (I) of any of claims 1 to 28 or a physiologically acceptable salt thereof in the manufacture of a medicament for the treatment or prevention of an androgen responsive or mediated disease.

32. Use according to claim 31 wherein the androgen responsive or mediated disease is benign prostatic hyperplasia, prostatitis, prostate cancer, acne, male pattern baldness and hiustism.

33. A compound as defined in any one of Claims 1 to 28 for use in therapy.

34. A process for preparing a compound of formula (I) as defined in Claim 1 or a pharmaceutically acceptable salt or solvate thereof which comprises reacting (A) reactina a compound of formula (IX) 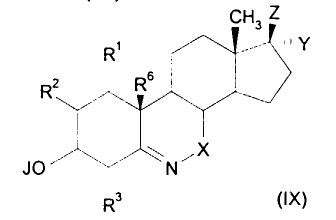 wherein JO is hydroxy or a protected hydroxy group, with an oxidising agent; or (B) for the preparation of compounds of formula (I) wherein X is -CH₂CH₂-, reacting a compound of formula (XV): 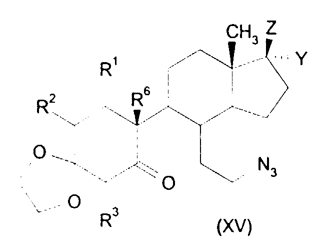 with a reducing agent, and if necessary and/or desired subjecting the compound thus obtained to one or more further reactions comprising
(i) converting the resulting compound of formula (I) or a salt or protected derivative thereof into another compound of formula (I); and/or
(ii) removing any protecting group or groups; and/or
(III) converting a compound of formula (I) or a salt thereof into a pharmaceutically acceptable salt thereof.

35. A process as claimed in Claim 34 wherein a compound of formula (IX) wherein JO is a protected hydroxy group is deprotected and then oxidised with an appropriate oxidising agent to give a compound of formula (I) where R⁴ is hydrogen.

36. A process as claimed in Claim 35 wherein the oxidising agent is Jones reagent.

37. A process as claimed in Claim 34 wherein a compound of formula (IX) wherein JO is a protected hydroxy group is treated with an acylating agent, deprotected and then oxidised with an appropriate oxidising agent to give a compound of formula (I) where R⁴ is acyl.

38. A process as claimed in Claim 37 wherein the oxidising agent is pyridinium dichromate.

39. A process as claimed in Claim 38 wherein a compound of formula (XV) is reacted with triphenylphosphine to give a compound of formula (I) wherein X is -CH₂CH₂-.

## Patentansprüche

1. Verbindung der Formel (I): 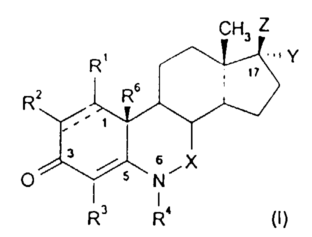 worin R¹ und R²
i) unabhängig Wasserstoff oder Niederalkyl sind und die Bindung zwischen den Kohlenstoffatomen, die R¹ und R² tragen, eine Einfach- oder eine Doppelbindung ist,
oder
ii) zusammengenommen eine -CH₂-Gruppe sind, die einen Cyclopropan-Ring bildet, und die Bindung zwischen den Kohlenstoffen, die R¹ und R² tragen, eine Einfachbindung ist;
R³ Wasserstoff, -Alk¹-H (gegebenenfalls substituiert mit einem oder mehreren Halogenen), Niedercycloalkyl, Niedercycloalkyl-niederalkyl, Halogen, -(Alk¹)ₙ-CO₂H, -(Alk¹)ₙ-CO₂R⁷, -(Alk¹)ₙ-Ar¹, -(Alk¹)ₙ-CONR⁸R⁹, -(Alk¹)ₙ-NR⁸R⁹, -(Alk¹)ₙ-S(O)ᵣR⁷, -(Alk¹)ₙ-CN, -(Alk¹)-OH, (Alk¹)ₙ-COR⁷ oder -(Alk¹)ₙ-OR⁷ ist, worin
Alk¹ Niederalkylen, Niederalkenylen oder Niederalkinylen ist,
n 0 oder 1 ist,
r 0, 1 oder 2 ist,
R⁷ -Alk¹-H, -(Alk¹)ₙ-Ar¹ oder Niedercycloalkyl ist,
R⁸ und R⁸ unabhängig Wasserstoff, -Alk¹-H oder Niedercycloalkyl sind,
Ar¹ eine homocyclische Aryl-Gruppe mit 6 bis 14 Kohlenstoffatomen ist;
R⁴ Wasserstoff, -Alk¹-H, Niedercycloalkyl, Niedercycloalkyl-niederalkyl, -(Alk¹)ₙ-S(O)ᵣR^{7,} -(Alk¹)ₙ-Phthalimidyl, -(Alk¹)-CO₂H, -(Alk¹)ₙ-CO₂R⁷, -(Alk¹)ₙ-COR⁷, -(Alk¹)ₙ-Ar¹, -(Alk¹)ₙ-CONR⁸R⁹, -(Alk¹)ₙ-NR⁸R⁹, -(Alk¹)ₙ-OH oder -(Alk¹)ₙ-OR⁷ ist;
X 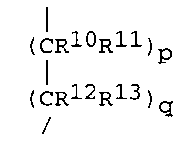 ist, wobei
R¹⁰, R¹¹, R¹² und R¹³ unabhängig Wasserstoff oder Niederalkyl sind,
p und q unabhängig entweder 0 oder 1 sind;
und
i) Y Wasserstoff oder Hydroxy ist und
Z -(Alk²)ₙ-COR⁵, -(Alk²)ₙ-CO₂R⁵, -(Alk²)ₙ-COSR⁵, -(Alk²)ₙ-CONR¹⁴R¹⁵, -(Alk²)-OCO₂R⁵, -(Alk²)-OCOR⁵, -(Alk²)-OCONR¹⁴R¹⁵, -(Alk²)-OR⁵, -(Alk²)-NR^{5'}COR⁵, -(Alk²)-NR^{5'}CO₂R⁵, -(Alk²)-NR⁵CONR¹⁴R¹⁵, -(Alk²)ₙ-CONR⁵NR¹⁴R¹⁵, -(Alk²)ₙ-CONR⁵CONR¹⁴R¹⁵, -(Alk²)-NR⁵CSNR¹⁴R¹⁵ oder -(Alk²)ₙ-CONR⁵CSNR¹⁴R¹⁵ ist;
wobei
Alk² (C₁₋₁₂)Alkylen, (C₂₋₁₂)Alkenylen oder (C₂₋₁₂)Alkinylen ist,
R⁵ und R^{5'} unabhängig Wasserstoff, -Alk¹-H (gegebenenfalls unabhängig substituiert mit einer oder mehreren CO₂H-, CO₂R⁷-, Ar²-, Ar³- oder Cyano-Gruppen), -(Alk¹)ₙ-(Niedercycloalkyl (gegebenenfalls unabhängig substituiert mit einer oder mehreren -Alk¹H-Gruppen)), Adamantyl, Norbornyl, Ar², Ar³, (Niedercycloalkyl)-Ar² oder (Niedercycloalkyl)-Ar³ ist;
wobei
Ar² eine homocyclische aromatische Gruppe mit 6 bis 14 Ringkohlenstoffatomen (gegebenenfalls unabhängig substituiert mit einem oder mehreren -Alk²-H (gegebenenfalls unabhängig mit einem oder mehreren Halogenen substituiert), -(Alk¹)ₙCOR⁷, -(Alk¹)ₙ-OH, -(Alk¹)ₙ-OR¹⁶, -(Alk¹)ₙ-Ar³, -(Alkl)n-C02H, -(Alk¹)ₙ-CO₂R⁷, S(O)ᵣR⁷, NR⁸S(O)ᵣR¹⁶, NR⁸R⁹, CONR⁸R⁹, Niedercycloalkyl, Niederalkoxy,-(Alk¹)ₙ-Ar¹ (gegebenenfalls substituiert mit einer oder mehreren -Alk¹-H-Gruppen oder Halogen), Methylendioxy, Ethylendioxy, Morpholino, Thiomorpholino, Cyano, Nitro oder Halogenen) ist;
wobei
R¹⁶ -Alk¹-H (gegebenenfalls unabhängig substituiert mit einem oder mehreren Halogenen), Niedercycloalkyl (gegebenenfalls unabhängig substituiert mit einem oder mehreren Halogenen oder -Alk¹-H (gegebenenfalls unabhängig substituiert mit einem oder mehreren Halogenen)) oder -(Alk¹)ₙ-Ar¹ ist (wobei Ar¹ gegebenenfalls unabhängig substituiert ist mit einer oder mehreren Niederalkoxy-Gruppen, Cyano-Gruppen, Halogen oder -Alk¹-H (gegebenenfalls unabhängig substituiert mit einem oder mehreren Halogenen));
Ar³ eine aromatische Gruppe mit 5 bis 14 Ringatomen ist, von denen wenigstens eines O, N oder S ist (gegebenenfalls unabhängig substituiert mit einer oder mehreren Gruppen -Alk¹-H (gegebenenfalls unabhängig substituiert mit einem oder mehreren Halogenen), Niedercycloalkyl, Niederalkoxy, CO₂H, CO₂R⁷, -(Alk¹)ₙ-Ar¹, Cyano oder Halogen);
R¹⁴ und R¹⁵ sind
a) unabhängig Hydroxy, Wasserstoff, -Alk²-H, Niederalkoxy, -(Alk¹)ₙ-adamantyl,-(Alk¹)ₙ-myrantyl, -(Alk¹)ₙ-norbornyl, -(Alk¹)ₙ-fluorenyl,-(Alk¹)ₙ-fluorenonyl, -(Alk¹)ₙ-indanyl (gegebenenfalls substituiert mit einem oder mehreren -Alk¹-H), -Alk¹-H (gegebenenfalls unabhängig substituiert mit einem oder mehreren Halogenen, Cyano, Cycloalkyl, SR⁵, COR⁵, CONR⁵R⁷, NR^{5'}COR⁵, NR^{5'}CO₂R⁵, NR^{5'}CONHR⁵, CO₂R⁵, OR⁵, Ar² oder Ar³), Ar² oder Ar³ oder ein gesättigter C₄₋₁₈-bicyclischer Ring oder ein C₃₋₁₁-gesättigter Ring sind, der gegebenenfalls ein Sauerstoff- oder Schwefelatom enthält (wobei diese Ringe gegebenenfalls unabhängig substituiert sind mit einer oder mehreren Cyano-, R¹⁶-, Ar²- oder Ar³-Gruppen);
b) Alkylen-Gruppen (gegebenenfalls substituiert mit einer oder mehreren R⁷-Gruppen, die zusammen mit dem Stickstoffatom eine 4- bis 8-atomige heterocyclische Gruppe bilden) 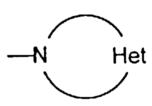 wobei
Het -O-, -CH₂-, -S(O)ᵣ-, -(NH)- oder -(N(Alk¹-H))- bedeutet;
mit der Maßgabe, daß
wenn Z -(Alk²)ₙ-COR⁵, -(Alk²)ₙ-CO₂R⁵ oder -(Alk²)ₙ-CO-thiopyridyl ist und
R⁵ Wasserstoff, -Alk¹-H, Niedercycloalkyl oder Adamantyl ist, oder
wenn Z -(Alk²)ₙ-CONR¹⁴R¹⁵ ist und
R¹⁴ und R¹⁵
a) unabhängig Wasserstoff, -Alk²-H, Niedercycloalkyl, Niederalkoxy, Adamantyl, -Ar¹, Benzyl, Diphenylmethyl, Triphenylmethyl oder-(Alk¹)ₙ-norbornyl sind; oder
b) Kohlenstoffatome, gegebenenfalls substituiert mit einer oder mehreren Niederalkyl-Gruppen sind, die zusammen mit dem verbindenden Stickstoff einen 4- bis 8-atomigen heterocyclischen Ring, wie zuvor definiert, bilden,
dann ist Y Hydroxy; oder
ii) Y ist Wasserstoff und
Z ist OR⁵, OCOR⁵, OCONR¹⁴R¹⁵, NR^{5'}COR⁵, NR^{5'}CO₂R⁵, NR⁵CONR¹⁴R¹⁵ oder NR⁵CSR¹⁴R¹⁵; und
iii) Y und Z sind zusammen:
=O, =CH-(Alk¹)ₙ-COR⁵, =CH-(Alk¹)ₙ-CO₂R⁵ oder =CH-(Alk¹)ₙ-CONR¹⁴R¹⁵;
R⁶ ist Wasserstoff oder Methyl;
oder ein pharmazeutisch akzeptables Salz oder Solvat davon.

2. Verbindung gemäß Anspruch 1, die eine Verbindung der Formel (IA), (IB), (IC) oder (ID) ist:

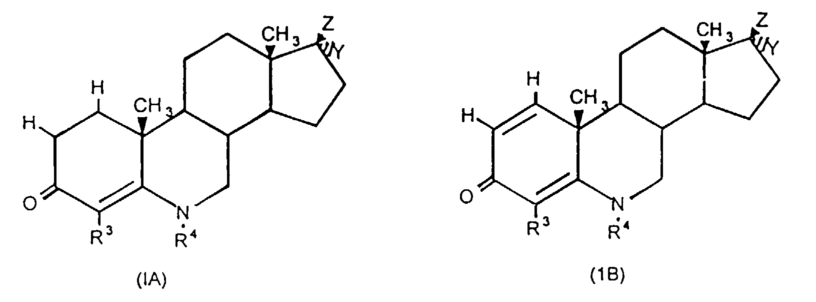

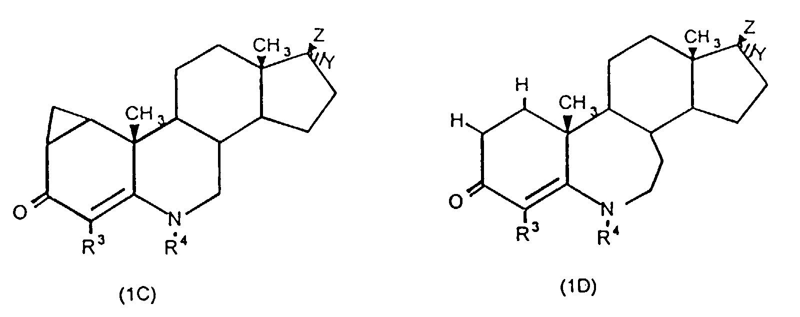

3. Verbindung gemäß Anspruch 1 oder Anspruch 2, worin R³ Wasserstoff, Halogen, Niederalkyl, Niedercycloalkyl oder Niedercycloalkyl-niederalkyl ist.

4. Verbindung gemäß Anspruch 3, worin R³ Wasserstoff, Halogen oder Niederalkyl ist.

5. Verbindung gemäß Anspruch 1 oder Anspruch 2, worin R³ Methyl, Ethyl, Cyano, Jodo, Bromo, Chloro oder Dimethylaminomethyl ist.

6. Verbindung gemäß einem der vorhergehenden Ansprüche, worin R⁴ Wasserstoff, Niederalkyl, Niedercycloalkyl oder Niedercycloalkyl-niederalkyl ist.

7. Verbindung gemäß Anspruch 6, worin R⁴ Wasserstoff oder Niederalkyl ist.

8. Verbindung gemäß einem der Ansprüche 1 bis 5, worin R⁴ Methyl, Ethyl, Propyl, i-Propyl, Butyl, i-Butylhexyl, 3-Hydroxypropyl, Propenyl, Methylencyclopropyl, Benzyl, 2-Methoxyethyl, 2-Essigsäure, 3-Propionsäure, 5-Pentansäure, 6-Hexansäure, Methyl-5-pentanoat, Ethyl-6-hexanoat, 3-Phthalimidylpropyl und 4-Phthalimidylpropyl ist.

9. Verbindung gemäß einem der vorhergehenden Ansprüche, worin X -CH₂- ist.

10. Verbindung gemäß einem der vorhergehenden Ansprüche, worin Y Wasserstoff ist und Z -(Alk²)ₙ-COR⁵, -(Alk²)ₙ-CONR¹⁴R¹⁵, -(Alk²)-OCO₂R⁵, -(Alk²)-OCOR⁵, -(Alk²)-NR^{5'}COR⁵, -(Alk²)ₙ-CONR⁵NR¹⁴R¹⁵, -(Alk²)-NR⁵CONR¹⁴R¹⁵ oder -(Alk²)ₙ-CONR⁵CONR¹⁴R¹⁵ ist.

11. Verbindung gemäß Anspruch 10, worin Z -COR⁵, -CONR¹⁴R¹⁵, -CH₂OCO₂R⁵, -CH₂OCOR⁵, -CH₂NR^{5'}COR⁵, -CONR⁵NR¹⁴R¹⁵, -CH₂NR⁵CONR¹⁴R¹⁵ oder -CONR⁵CONR¹⁴R¹⁵ ist.

12. Verbindung gemäß Anspruch 11, worin Z -CONR¹⁴R¹⁵ ist.

13. Verbindung gemäß einem der vorhergehenden Ansprüche, worin Ar¹ eine Phenyl-Gruppe ist.

14. Verbindung gemäß einem der vorhergehenden Ansprüche, worin Ar² eine Phenyl-Gruppe, gegebenenfalls unabhängig substituiert mit einer oder mehreren -Alk²-H-(gegebenenfalls unabhängig substituiert mit einem oder mehreren Halogenen), -OR¹⁶⁻, -S(O)ᵣR⁷-, Ar¹-, Methylendioxy-, Ethylendioxy-, Morpholino-, Thiomorpholino-, Cyano-, Nitro- oder Halogen-Gruppen, ist.

15. Verbindung gemäß einem der vorhergehenden Ansprüche, worin R¹⁴ Wasserstoff oder Hydroxy ist und R¹⁵ Wasserstoff, Niedercycloalkyl (gegebenenfalls unabhängig substituiert mit einer oder mehreren R⁷- oder Ar²-Gruppen),-(Alk¹)-adamantyl, -(Alk¹)ₙ-myrantyl, -(Alk¹)ₙ-norbornyl,-(Alk¹)ₙ-fluorenyl, -(Alk¹)ₙ-indanyl, -Alk¹-H (gegebenenfalls unabhängig substituiert mit einer oder mehreren Cycloalkyl-, SR⁵-, OR⁵-, Ar²- oder Ar³-Gruppen), Ar² oder Ar³ ist; oder
R¹⁴ und R¹⁵ Kohlenstoffatome sind, gegebenenfalls substituiert mit einer oder mehreren R⁷-Gruppen, die zusammen mit dem verbindenden Stickstoff eine 5- bis 7-atomige heterocyclische Gruppe 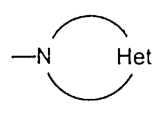 bilden, worin Het -CH₂- bedeutet.

16. Verbindung gemäß einem der vorhergehenden Ansprüche, worin Ar³ eine aromatische Gruppe mit 5 oder 6 Ringatomen ist, von denen wenigstens eines O, N oder S ist, gegebenenfalls unabhängig substituiert mit einer oder mehreren Alk¹H-Gruppen.

17. Verbindung gemäß Anspruch 16, worin Ar³ eine gegebenenfalls substituierte Pyrrolyl-, Thienyl-, Furyl- oder Pyridyl-Gruppe ist.

18. Verbindung gemäß einem der vorhergehenden Ansprüche, worin R⁵ Wasserstoff, Niederalkyl, gegebenenfalls unabhängig substituiert mit einer oder mehreren Ar²-Gruppen, (Niederalkyl)ₙ-niedercycloalkyl, Menthyl, Adamantyl, Norbornyl oder Ar² ist.

19. Verbindung gemäß einem der Ansprüche 1 bis 11 oder 13 bis 18, worin Z -COR⁵ ist.

20. Verbindung gemäß Anspruch 19, welche ist:
17β-(1-Oxo-2-cyclohexylethyl)-6-azaandrost-4-en-3-on,
17β-(1-Oxo-1-(2,4-difluorphenyl)methyl)-6-azaandrost-4-en-3-on,
17β-(1-Oxo-1-(4-isopropoxyphenyl)methyl)-6-azaandrost-4-en-3-on,
17β-(1-Oxo-3,3-diphenylpropyl)-6-azaandrost-4-en-3-on,
17β-(1-Oxo-1-(2-norbornyl)methyl)-6-azaandrost-4-en-3-on
oder ein pharmazeutisch akzeptables Salz oder Solvat davon.

21. Verbindung gemäß einem der Ansprüche 1 bis 18, worin Z -CONR¹⁴R¹⁵ ist, R¹⁴ Wasserstoff ist und R¹⁵ Ar² oder ein C₃₋₁₁-gesättigter Ring ist, der gegebenenfalls ein Sauerstoff- oder Schwefelatom enthält (gegebenenfalls unabhängig substituiert mit einer oder mehreren R⁷- oder Ar²-Gruppen) .

22. Verbindung gemäß einem der vorhergehenden Ansprüche, worin R¹⁵ ein Gruppe der Formel Ar^{2a} ist 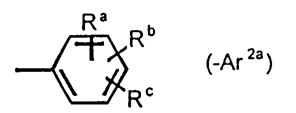 worin R^{a} und R^{b} unabhängig Wasserstoff, Niederalkyl, Trifluormethyl, Halogen oder Phenyl (gegebenenfalls substituiert mit einem oder mehreren Halogenen oder verzweigten C₄₋₇-Alkyl-Gruppen) sind und R^{c} Wasserstoff oder eine oder mehrere Halogene sind, oder R¹⁵ ein C₃₋₁₁-gesättigter Ring ist, der gegebenenfalls ein Sauerstoff- oder Schwefelatom enthält und mit einer Gruppe der Formel Ar^{2a} substituiert ist.

23. Verbindung gemäß Anspruch 22, worin Ar^{2a} eine Gruppe der Formel Ar^{2aa} ist: 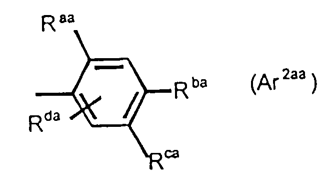 ist, worin R^{aa} verzweigtes C₄₋₇-Alkyl, Trifluormethyl oder Phenyl, gegebenenfalls substituiert mit einem oder mehreren Halogenen, ist; eine der Gruppen aus R^{ba} und R^{ca} verzweigtes C₄₋₇-Alkyl, Trifluormethyl, Halogen oder Phenyl, gegebenenfalls substituiert mit einem oder mehreren Halogenen, ist und die andere Gruppe Wasserstoff oder Halogen ist; und R^{da} Wasserstoff oder Halogen ist.

24. Verbindung gemäß Anspruch 1, und zwar:
17β-N-((2,6-Di-i-propyl)phenyl)carbamoyl-6-azaandrost-4-en-3-on,
17β-N-(2,4,6-Trimethyl)phenyl-carbamoyl-6-azaandrost-4-en-3-on,
17β-N-(2-Chloro-5-trifluormethyl)phenyl-carbamoyl-6-azaandrost-4-en-3-on,
17β-N-(2,6-Dimethyl-4-bromo)phenyl-carbamoyl-6-azaandrost-4-en-3-on,
17β-N-(2,6-Dimethyl-4-t-butyl)phenyl-carbamoyl-6-azaandrost-4-en-3-on,
17β-N-(2,6-Dibromo-4-isopropyl)phenyl-carbamoyl-6-azaandrost-4-en-3-on,
17β-N-(2,5-Ditrifluormethyl)phenyl-carbamoyl-6-azaandrost-4-en-3-on,
17β-N-(2-Phenyl)phenyl-carbamoyl-6-azaandrost-4-en-3-on,
17β-N-(2,6-Diethyl-3,5-dichloro)phenyl-carbamoyl-6-azaandrost-4-en-3-on,
17β-N-(2,6-Diethyl-3-chloro)phenyl-carbamoyl-6-azaandrost-4-en-3-on,
17β-N-(2-t-Butyl)phenyl-carbamoyl-6-azaandrost-4-en-3-on,
17β-N-(2,4,6-Trichloro)phenyl-carbamoyl-6-azaandrost-4-en-3-on,
17β-N-(2-Bromo-5-trifluormethyl)phenyl-carbamoyl-6-azaandrost-4-en-3-on,
17β-N-(2-t-Butyl-6-methyl)phenyl-carbamoyl-6-azaandrost-4-en-3-on,
17β-N-1-(4-Chlorophenyl)cyclopentyl-carbamoyl-6-azaandrost-4-en-3-on,
17β-N-(2,6-Dibromo-4-chloro)phenyl-carbamoyl-6-azaandrost-4-en-3-on,
17β-N-(2,6-Diethyl-4-bromo)phenyl-carbamoyl-6-azaandrost-4-en-3-on,
17β-N-(2-Bromo-4-t-butyl)phenyl-carbamoyl-6-azaandrost-4-en-3-on,
17β-N-(2-Chloro-4-t-butyl)phenyl-carbamoyl-6-azaandrost-4-en-3-on,
17β-N-(5-Bromo-2-t-butyl)phenyl-carbamoyl-6-azaandrost-4-en-3-on,
17β-N-(5-Chloro-2-t-butyl)phenyl-carbamoyl-6-azaandrost-4-en-3-on,
17β-N-(2,6-Diethyl)phenyl-carbamoyl-6-azaandrost-4-en-3-on,
17β-N-(4-Bromo-2-t-butyl)phenyl-carbamoyl-6-azaandrost-4-en-3-on,
17β-N-(2-t-Butyl-5-cyano)phenyl-carbamoyl-6-azaandrost-4-en-3-on,
17β-N-(2-(O-4-Tolyl)-5-trifluormethyl)phenylcarbamoyl-6-azaandrost-4-en-3-on,
17β-N-(2-(O-4-Chlorophenyl)-5-trifluormethyl)phenyl-carbamoyl-6-azaandrost-4-en-3-on,
17β-N-(2-Nitro-4-t-butyl)phenyl-carbamoyl-6-azaandrost-4-en-3-on,
17β-N-(2-(O-Phenyl)-5-(1,1-dimethyl)propyl)phenylcarbamoyl-6-azaandrost-4-en-3-on,
17β-N-(2-Ethylsulfonyl-5-trifluormethyl)phenylcarbamoyl-6-azaandrost-4-en-3-on,
17β-N-(3,5-Di-t-butyl)phenyl-carbamoyl-6-azaandrost-4-en-3-on,
17β-N-(2-t-Butyl-5-trifluormethyl)phenyl-carbamoyl-6-azaandrost-4-en-3-on,
17β-N-(2-t-Butyl-5-phenyl)phenyl-carbamoyl-6-azaandrost-4-en-3-on,
17β-N-(2,6-Di-i-propyl)phenyl-carbamoyl-6-azaandrost-1,4-dien-3-on,
17β-N-(2,6-Di-i-propyl)phenyl-carbamoyl-6-azaandrost-4-methyl-1,4-dien-3-on,
17β-N-1-(4-Trifluormethylphenyl)cyclopentyl-carbamoyl-6-azaandrost-4-en-3-on,
17β-N-l-(4-Fluorphenyl)cyclohexyl-carbamoyl-6-azaandrost-4-en-3-on,
17β-N-1-(4-Methoxyphenyl)cyclohexyl-carbamoyl-6-azaandrost-4-en-3-on,
17β-N-1-(4-Methoxyphenyl)cyclopentyl-carbamoyl-6-azaandrost-4-en-3-on,
17β-N-(2,5-Bis(trifluormethyl))phenyl-carbamoyl-6-azaandrost-4-methyl-4-en-3-on,
17β-N-(2-t-Butyl-5-trifluormethyl)phenyl-carbamoyl-6-azaandrost-4-methyl-4-en-3-on,
17β-N-(2,5-Di-t-butyl)phenyl-carbamoyl-6-azaandrost-4-methyl-4-en-3-on,
17β-N-(2-t-Butyl-5-(4-chlorophenyl))phenyl-carbamoyl-6-azaandrost-4-en-3-on,
17β-N-1-(4-t-Butylphenyl)cyclopentyl-carbamoyl-6-azaandrost-4-en-3-on,
17β-N-1-(4-t-Butylphenyl)cyclohexyl-carbamoyl-6-azaandrost-4-en-3-on,
17β-N-1-(4-Chlorophenyl)cyclopentyl-carbamoyl-6-azaandrost-4-methyl-4-en-3-on,
17β-N-(2,5-Bis(trifluormethyl))phenyl-carbamoyl-6-azaandrost-4-chloro-4-en-3-on,
17β-N-(2-t-Butyl-5-trifluormethyl)phenyl-carbamoyl-6-azaandrost-4-chloro-4-en-3-on,
17β-N-1-(4-t-Butylphenyl)cycloheptyl-carbamoyl-6-azaandrost-4-en-3-on,
17β-N-1-(4-t-Butylphenyl)cyclohexyl-carbamoyl-6-azaandrost-4-chloro-4-en-3 -on,
17β-N-(2,6-Diethyl-4-(4-chlorophenyl))phenyl-carbamoyl-6-azaandrost-4-en-3-on,
17β-N-4-(4-t-Butylphenyl)tetrahydrothiopyranyl-carbamoyl-6-azaandrost-4-en-3-on,
17β-N-9-(4-t-Butylphenyl)bicyclo[3.3.1]nonyl-carbamoyl-6-azaandrost-4-en-3-on,
17β-N-4-(4-t-Butylphenyl)tetrahydropyranyl-carbamoyl-6-azaandrost-4-en-3-on,
17β-N-1-(4-Chlorophenyl)cyclohexyl-carbamoyl-6-azaandrost-4-en-3-on,
17β-N-(2-t-Butyl-5-(4-t-butyl)phenyl)phenyl-carbamoyl-6-azaandrost-4-en-3-on, oder
17β-N-1-(4-Chlorophenyl)cyclopentyl-carbamoyl-6-azaandrost-4-chloro-4-en-3 -on
oder ein pharmazeutisch akzeptables Salz oder Solvat davon.

25. 17β-N-(2-t-Butyl-5-trifluormethyl)phenyl-carbamoyl-6-azaandrost-4-en-3-on oder ein pharmazeutisch akzeptables Salz davon.

26. Verbindung der Formel (I): 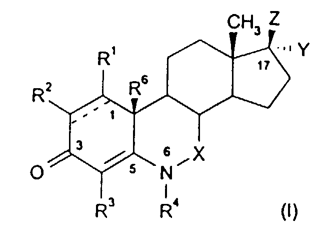 worin R¹ und R²
i) unabhängig Wasserstoff oder Niederalkyl sind und die Bindung zwischen den Kohlenstoffatomen, die R¹ und R² tragen, eine Einfach- oder eine Doppelbindung ist, oder
ii) zusammengenommen eine -CH₂-Gruppe sind, so daß ein Cyclopropan-Ring gebildet wird, und die Bindung zwischen den Kohlenstoffen, die R¹ und R² tragen, eine Einfachbindung ist;
R³ Wasserstoff oder Niederalkyl ist;
R⁴ Wasserstoff oder Niederalkyl ist;
X 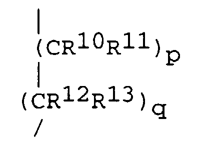 ist, wobei
R¹⁰, R¹¹, R¹² und R¹³ unabhängig Wasserstoff oder Niederalkyl sind,
p und q unabhängig entweder 0 oder 1 sind;
Y Wasserstoff ist; und
Z -COR⁵, -CONR¹⁴R¹⁵, -CH₂OCO₂R⁵, -CH₂OCOR⁵, -CH₂NR^{5'}COR⁵, -CONR⁵NR¹⁴R¹⁵, -CH₂NR⁵CONR¹⁴R⁵ oder -CON⁵CONR¹⁴R¹⁵ ist, wobei R⁵ und R^{5'} unabhängig Wasserstoff, Niederalkyl, gegebenenfalls unabhängig substituiert mit einer oder mehreren Ar²-Gruppen, (Niederalkyl)ₙ-niedercycloalkyl, Menthyl, Adamantyl, Norbornyl oder Ar² ist;
Ar² eine Phenyl-Gruppe ist, die gegebenenfalls unabhängig mit einer oder mehreren -Alk-H (gegebenenfalls unabhängig substituiert mit einem oder mehreren Halogenen), -OR¹⁶⁻, -S(O)ᵣR⁷-, Phenyl-, Methylendioxy-, Ethylendioxy-, Morpholino-, Thiomorpholino-, Cyano-, Nitro- oder Halogen-Gruppen substituiert ist;
Alk Niederalkylen, Niederalkenylen oder Niederalkinylen ist;
n 0 oder 1 ist;
r 0, 1 oder 2 ist;
R⁷ -Alk-H, -(Alk)ₙ-phenyl oder Niedercycloalkyl ist;
R¹⁶ -Alk-H (gegebenenfalls unabhängig substituiert mit einem oder mehreren Halogenen), Niedercycloalkyl (gegebenenfalls unabhängig substituiert mit einem oder mehreren Halogenen oder -Alk-H (gegebenenfalls unabhängig substituiert mit einem oder mehreren Halogenen)) oder -(Alk)ₙ-phenyl ist (worin Phenyl gegebenenfalls unabhängig mit einer oder mehreren Niederalkoxy-Gruppen, Cyano, Halogen oder -Alk-H-Gruppen (gegebenenfalls unabhängig substituiert mit einem oder mehreren Halogenen) substituiert ist); R¹⁴ Wasserstoff oder Hydroxy ist und R¹⁵ Wasserstoff, Niedercycloalkyl (gegebenenfalls unabhängig substituiert mit einer oder mehreren R⁷- oder Ar²-Gruppen), -(Alk)-adamantyl, -(Alk)ₙ-myrantyl,-(Alk)ₙ-norbornyl, -(Alk)ₙ-fluorenyl, -(Alk)ₙ-indanyl, -Alk-H (gegebenenfalls unabhängig substituiert mit einer oder mehreren Cycloalkyl-, SR⁵-, OR⁵-, Ar²- oder Ar²-Gruppen), Ar² oder Ar³ ist, oder R¹⁴ und R¹⁵ zusammen mit dem verbindenden Stickstoff einen Pyrrolidinyl-, Piperidinyl- oder Perhydroazepinyl-Ring bilden, der gegebenenfalls mit einer oder mehreren R⁷-Gruppen substituiert ist; Ar³ eine Pyrrolyl-, Thienyl-, Furyl- oder Pyridyl-Gruppe ist, die gegebenenfalls unabhängig mit einer oder mehreren Alk-H-Gruppen substituiert ist; mit den Maßgaben daß,
wenn Z COR⁵ ist, ist R⁵ eine substituierte Niederalkyl-, Niederalkylniedercycloalkyl-, Menthyl-, Norbornyl- oder Ar²-Gruppe;
wenn Z CONR¹⁴R¹⁵ ist und R¹⁵ Wasserstoff, -Alk-H, Niedercycloalkyl, Niederalkoxy, Adamantyl, Phenyl, Benzyl, Diphenylmethyl, Triphenylmethyl oder -(Alk)ₙ-norbornyl ist, ist R¹⁴ Hydroxy; und
wenn Z CONR¹⁴R¹⁵ ist und R¹⁴ und R¹⁵ zusammen mit dem Stickstoffatom einen Pyrrolidinyl-, Piperidinyl- oder Perhydroazepinyl-Ring bilden, ist dieser Ring substituiert mit einer oder mehreren Niederalkenyl-, Niederalkinyl-, - (Alk)ₙ-phenyl- oder Niedercycloalkyl-Gruppen; oder ein pharmazeutisch akzeptables Salz oder Solvat davon.

27. Verbindung der Formel (I): 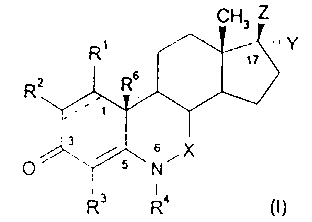 worin R¹ und R²
i) unabhängig Wasserstoff oder Niederalkyl sind und die Bindung zwischen den Kohlenstoffatomen, die R¹ und R² tragen, eine Einfach- oder eine Doppelbindung ist,
oder
ii) zusammengenommen eine -CH₂-Gruppe sind und einen Cyclopropan-Ring bilden und die Bindung zwischen den Kohlenstoffen, die R¹ und R² tragen, eine Einfachbindung ist;
R³ Wasserstoff, -Alk¹-H, -Alk¹-H substituiert mit einem oder mehreren Halogenen, Niedercycloalkyl, Niedercycloalkyl-niederalkyl, Halogen, - (Alk¹)ₙ-CO₂H, -(Alk¹)ₙ-CO₂R⁷, -(Alk¹)ₙ-Ar¹, -(Alk¹)ₙ-CONR⁸R⁹, -(Alk¹)ₙ-NR⁸R⁹, -(Alk¹)ₙ-S(O)ᵣR⁷, -(Alk¹)ₙ-CN, -(Alk¹)-OH oder -(Alk¹)ₙ-OR⁷ ist, worin
Alk¹ Niederalkylen, Niederalkenylen oder Niederalkinylen ist,
n 0 oder 1 ist,
r 0, 1 oder 2 ist,
R⁷ -Alk¹-H, -(Alk¹)ₙ-Ar¹ oder Niedercycloalkyl ist,
R⁸ und R⁹ unabhängig Wasserstoff, -Alk¹-H oder Niedercycloalkyl sind,
Ar¹ eine homocyclische Aryl-Gruppe mit 6 bis 14 Kohlenstoffatomen ist;
R⁴ Wasserstoff, -Alk¹-H, Niedercycloalkyl, Niedercycloalkyl-niederalkyl,-(Alk¹)ₙ-S(O)ᵣR⁷, -(Alk¹)ₙ-Phthalimidyl, -(Alk¹)CO₂H, -(Alk¹)ₙ-CO₂R⁷, -(Alk¹)ₙ-Ar¹, -(Alk¹)ₙ-CONR⁸R⁹, -(Alk¹)ₙ-NR⁸R⁹, -(Alk¹)ₙ-OH oder -(Alk¹)ₙ-OR⁷ ist;
X 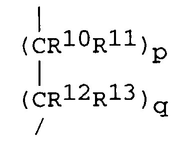 ist, wobei
R¹⁰, R¹¹, R¹² und R¹³ unabhängig Wasserstoff oder Niederalkyl sind,
p und q unabhängig entweder 0 oder 1 sind;
Y und Z die folgenden Bedeutungen haben:
i) Y ist Wasserstoff oder Hydroxy und
Z ist -(Alk²)ₙ-COR⁵, -(Alk²)ₙ-CO₂R⁵, -(Alk²)ₙ-COSR⁵, -(Alk²)ₙ-CONR¹⁴R¹⁵, -(Alk²)-OCOR⁵, -(Alk²)-OCONR¹⁴R¹⁵, -(Alk²)-OR⁵, -(Alk²)-NR⁵COR⁵, -(Alk²)-NR⁵CO₂R⁵, -(Alk²)-NR⁵CONR¹⁴R¹⁵, -(Alk²)ₙCONR⁵NR¹⁴R¹⁵, -(Alk²)ₙ-CONR⁵CONR¹⁴R¹⁵, -(Alk²)-NR⁵CSNR¹⁴R¹⁵ oder -(Alk²)ₙ-CONR⁵CSNR¹⁴R¹⁵;
wobei
Alk² (C₁₋₁₂)Alkylen, (C₂₋₁₂) Alkenylen oder (C₂₋₁₂)Alkinylen ist,
R⁵ Wasserstoff, -Alk¹-H, -(Alk¹)ₙ- (Niedercycloalkyl), Adamantyl, -(Alk¹)ₙ-Ar², -(Alk¹)ₙ-Ar³, (Niedercycloalkyl)ₙAr²,
(Niedercycloalkyl)ₙAr³ oder -Alk¹ ist, unabhängig substituiert mit einer oder mehreren CO₂H-, CO₂R⁷-, Ar²- oder Ar³-Gruppen;
wobei
Ar² eine aromatische Gruppe mit 6 bis 14 Ringkohlenstoffatomen, gegebenenfalls substituiert mit einer oder mehreren Alk¹-Gruppen, halogen-substituierte Alk¹-Gruppen, -(Alk¹)ₙ-OH, -(Alk¹)ₙ-OR⁷, -(Alk¹)ₙ-CO₂H, -(Alk¹)ₙ-CO₂R⁷, S(O)ᵣR⁷ oder NR⁸R⁹, Niedercycloalkyl-, Niederalkoxy-,-(Alk¹)ₙ-Ar¹-, Methylendioxy-, Ethylendioxy-, Morpholino-, Thiomorpholino-, Cyano- oder Halogen-Gruppen, ist;
Ar³ eine aromatische Gruppe mit 5 bis 14 Ringatomen ist, von denen wenigstens eines 0, N oder S ist, gegebenenfalls substituiert mit einer oder mehreren Alk¹-, Niedercycloalkyl-, Niederalkoxy-, CO₂H, CO₂R⁷, -(Alk¹)ₙ-Ar¹, Cyano- oder Halogen-Gruppen;
R¹⁴ und R¹⁵ sind
a) unabhängig Wasserstoff oder -Alk²-H, Niedercycloalkyl, Niederalkoxy, Adamantyl, -(Alk¹)ₙ-norbornyl, Ar², Ar³, -Alk¹-, unabhängig substituiert mit einer oder mehreren SR⁵-, COR⁵-, CONR⁵R⁷-, NR⁵COR⁵-, NR⁵CO₂R⁵-, NR⁵CONHR⁵-, CO₂R⁵-, OR⁵-, Ar²- oder Ar³-Gruppen, -(Alk¹)ₙ-fluorenyl oder -(Alk¹)ₙ-indanyl;
oder
b) Kohlenstoffatome, die gegebenenfalls mit einer oder mehreren Niederalkyl-Gruppen substituiert sind, und zusammen mit dem verbindenden Stickstoff eine 4- bis 8-atomige heterocyclische Gruppe 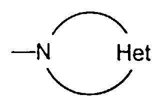 bilden, worin
Het -O-, -CH₂-, -S(O)ᵣ-, -(NH)- oder -(N(Alk¹))-bedeutet;
mit der Maßgabe, daß
wenn Z -(Alk²)ₙ-COR⁵; -(Alk²)ₙ-CO₂R⁵ oder -(Alk²)ₙ-CO-thiopyridyl ist und R⁵ Wasserstoff, -Alk¹-H, Niedercycloalkyl, -(Alk¹)ₙ-Ar¹, Adamantyl ist, oder
wenn Z -(Alk²)ₙ-CONR¹⁴R¹⁵ ist und R¹⁴ und R¹⁵
(a) unabhängig Wasserstoff, -Alk²-H, Niedercycloalkyl, Niederalkoxy, Adamantyl, -Ar¹, Benzyl, Diphenylmethyl, Triphenylmethyl oder -(Alk¹)ₙ-norbornyl sind; oder
(b) Kohlenstoffatome sind, gegebenenfalls substituiert mit einer oder mehreren Niederalkyl-Gruppen, die zusammen mit dem verbindenden Stickstoff einen 4- bis 8-atomigen heterocyclischen Ring wie zuvor definiert bilden,
dann ist Y Hydroxy; oder
ii) Y ist Wasserstoff und
Z ist OR⁵, OCOR⁵, OCONR¹⁴R¹⁵, NR⁵COR⁵, NR⁵CO₂R⁵, NR⁵CONR¹⁴R¹⁵ oder NR⁵CSR¹⁴R¹⁵;
iii) Y und Z sind zusammen:
=O, =CH-(Alk¹)ₙ-COR⁵, =CH-(Alk¹)ₙ-CO₂R⁵ oder =CH-(Alk¹)ₙ-CONR¹⁴R¹⁵;
R⁶ ist Wasserstoff oder Methyl;
und pharmazeutisch akzeptable Salze davon.

28. Verbindung der Formel (I): 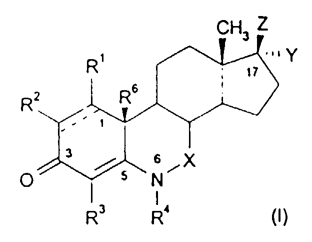 worin R¹ und R²
i) unabhängig Wasserstoff oder Niederalkyl sind und die Bindung zwischen den Kohlenstoffatomen, die R¹ und R² tragen, eine Einfach- oder eine Doppelbindung ist,
oder
ii) zusammengenommen eine -CH₂-Gruppe sind und einen Cyclopropan-Ring bilden und die Bindung zwischen den Kohlenstoffen, die R¹ und R² tragen, eine Einfachbindung ist;
R³ Wasserstoff, -Alk¹-H (gegebenenfalls substituiert mit einem oder mehreren Halogenen), Niedercycloalkyl, Niedercycloalkyl-niederalkyl), Halogen, - (Alk¹)ₙ-CO₂H, -(Alk¹)ₙ-CO₂R⁷, -(Alk¹)ₙ-Ar¹, -(Alk¹)ₙ-CONR⁸R⁹, -(Alk¹)ₙ-NR⁸R⁹, -(Alk¹)ₙ-S(O)ᵣR⁷, -(Alk¹)ₙ-CN, -(Alk¹)-OH, -(Alk¹)ₙ-COR⁷ oder -(Alk¹)ₙ-OR⁷ ist, worin
Alk¹ Niederalkylen, Niederalkenylen oder Niederalkinylen ist,
n 0 oder 1 ist,
r 0, 1 oder 2 ist,
R⁷ -Alk¹-H, -(Alk¹)ₙ-Ar¹ oder Niedercycloalkyl ist,
R⁸ und R⁹ unabhängig Wasserstoff, -Alk¹-H oder Niedercycloalkyl sind,
Ar¹ eine homocyclische Aryl-Gruppe mit 6 bis 14 Kohlenstoffatomen ist;
R⁴ Wasserstoff, -Alk¹-H, Niedercycloalkyl, Niedercycloalkyl-niederalkyl,-(Alk¹)ₙ-S(O)ᵣR⁷, -(Alk¹)ₙ-Phthalimidyl, -(Alk¹)-CO₂H, -(Alk¹)ₙ-CO₂R⁷, -(Alk¹)ₙ-COR⁷, -(Alk¹)ₙ-Ar¹, -(Alk¹)ₙ-CONR⁸R⁹, -(Alk¹)ₙ-NR⁸R⁹, -(Alk¹)ₙ-OH oder -(Alk¹)ₙ-OR⁷ ist;
X 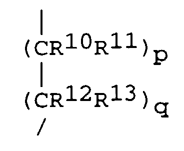 ist, wobei
R¹⁰, R¹¹, R¹² und R¹³ unabhängig Wasserstoff oder Niederalkyl sind,
p und q unabhängig entweder 0 oder 1 sind;
und
i) Y Wasserstoff oder Hydroxy ist und
Z -(Alk²)ₙ-COR⁵, -(Alk²)ₙ-CO₂R⁵, -(Alk²)ₙ-COSR⁵, -(Alk²)ₙ-CONR¹⁴R¹⁵, -(Alk²)-OCO₂R⁵, -(Alk²)-OCOR⁵, -(Alk²)-OCONR¹⁴R¹⁵, -(Alk²)-OR⁵, -(Alk²)-NR^{5'}COR⁵, -(Alk²)-NR^{5'}CO₂R⁵, -(Alk²)-NR⁵CONR¹⁴R¹⁵, -(Alk²)ₙ-CONR⁵NR¹⁴R¹⁵, (Alk²)ₙ-CONR⁵CONR¹⁴R¹⁵, -(Alk²)-NR⁵CSNR¹⁴R¹⁵ oder -(Alk²)ₙ-CONR⁵CSNR¹⁴R¹⁵ ist;
wobei
Alk² (C₁₋₁₂)Alkylen, (C₂₋₁₂)Alkenylen oder (C₂₋₁₂)Alkinylen ist,
R⁵ und R^{5'} unabhängig Wasserstoff, -Alk¹-H (gegebenenfalls unabhängig substituiert mit einer oder mehreren CO₂H-, CO₂R⁷-, Ar²-, Ar³- oder Cyano-Gruppen), -(Alk¹)ₙ-(Niedercycloalkyl (gegebenenfalls unabhängig substituiert mit einer oder mehreren -Alk¹H-Gruppen)), Adamantyl, Norbornyl, Ar², Ar³, (Niedercycloalkyl)-Ar² oder (Niedercycloalkyl)-Ar³ ist;
wobei
Ar² eine homocyclische aromatische Gruppe mit 6 bis 14 Ringkohlenstoffatomen (gegebenenfalls unabhängig substituiert mit einem oder mehreren -Alk²-H (gegebenenfalls unabhängig mit einem oder mehreren Halogenen substituiert), -(Alk¹)ₙ-OH, -(Alk¹)ₙ-OR¹⁶, -(Alk¹)ₙ-Ar³, -(Alk¹)ₙ-CO₂H, -(Alk¹)ₙ-CO₂R⁷, S(O)ᵣR⁷, NR⁸S(O)ᵣR¹⁶, NR⁸R⁹, CONR⁸R⁹, Niedercycloalkyl, Niederalkoxy, -(Alk¹)ₙ-Ar¹, Methylendioxy, Ethylendioxy, Morpholino, Thiomorpholino, Cyano, Nitro oder Halogenen) ist;
wobei
R¹⁶ -Alk¹-H (gegebenenfalls unabhängig substituiert mit einem oder mehreren Halogenen), Niedercycloalkyl (gegebenenfalls unabhängig substituiert mit einem oder mehreren Halogenen oder -Alk¹-H (gegebenenfalls unabhängig substituiert mit einem oder mehreren Halogenen)) oder -(Alk¹)ₙ-Ar¹ ist (wobei Ar¹ gegebenenfalls unabhängig substituiert ist mit einer oder mehreren Niederalkoxy-Gruppen, Cyano-Gruppen, Halogenen oder -Alk¹-H (gegebenenfalls unabhängig substituiert mit einem oder mehreren Halogenen));
Ar³ eine aromatische Gruppe mit 5 bis 14 Ringatomen ist, von denen wenigstens eines 0, N oder S ist (gegebenenfalls unabhängig substituiert mit einer oder mehreren Gruppen -Alk¹-H (gegebenenfalls unabhängig substituiert mit einem oder mehreren Halogenen), Niedercycloalkyl, Niederalkoxy, CO₂H, CO₂R⁷, -(Alk¹)ₙ-Ar¹, Cyano oder Halogen ist);
R¹⁴ und R¹⁵ sind
a) unabhängig Hydroxy, Wasserstoff, -Alk²-H, Niedercycloalkyl (gegebenenfalls unabhängig substituiert mit einer oder mehreren Cyano-, R¹⁶-, Ar²-, Ar³-Gruppen), Niederalkoxy, -(Alk¹)ₙ-adamantyl, -(Alk¹)ₙ-myrantyl, -(Alk¹)ₙ-norbornyl, -(Alk¹)ₙ-fluorenyl, -(Alk¹)ₙ-indanyl, -Alk¹-H (gegebenenfalls unabhängig substituiert mit einem oder mehreren Halogenen, Cyano, Cycloalkyl, SR⁵, COR⁵, CONR⁵R⁷, NR^{5'}COR⁵, NR^{5'}CO₂R⁵, NR^{5'}CONHR⁵, CO₂R⁵, OR⁵, Ar² oder Ar³), Ar² oder Ar³;
b) Alkylen-Gruppen (die gegebenenfalls substituiert sind mit einer oder mehreren R⁷-Gruppen und zusammen mit dem Stickstoffatom eine 4- bis 8-atomige heterocyclische Gruppe bilden) 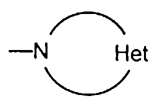 wobei
Het -O-, -CH₂-, -S(O)ᵣ-, -(NH)- oder -(N(Alk¹-H))- bedeutet;
mit der Maßgabe, daß
wenn Z -(Alk²)ₙ-COR⁵; -(Alk²)ₙ-CO₂R⁵ oder -(Alk²)ₙ-CO-thiopyridyl ist und
R⁵ Wasserstoff, -Alk¹-H, Niedercycloalkyl, -(Alk¹)ₙ-Ar¹ oder Adamantyl ist, oder
wenn Z -(Alk²)ₙ-CONR¹⁴R¹⁵ ist und
R¹⁴ und R¹⁵
a) unabhängig Wasserstoff, -Alk²-H, Niedercycloalkyl, Niederalkoxy, Adamantyl, -Ar¹, Benzyl, Diphenylmethyl, Triphenylmethyl oder-(Alk¹)ₙ-norbornyl sind; oder
b) Kohlenstoffatome, gegebenenfalls substituiert mit einer oder mehreren Niederalkyl-Gruppen sind, die zusammen mit dem verbindenden Stickstoff einen 4- bis 8-atomigen heterocyclischen Ring wie zuvor definiert bilden,
dann ist Y Hydroxy; oder
ii) Y ist Wasserstoff und
Z ist OR⁵, OCOR⁵, OCONR¹⁴R¹⁵, NR^{5'}COR⁵, NR^{5'}CO₂R⁵, NR⁵CONR¹⁴R¹⁵ oder NR⁵CSR¹⁴R¹⁵ und
iii) Y und Z sind zusammen:
=O, =CH-(Alk¹)ₙ-COR⁵, =CH-(Alk¹)ₙ-CO₂R⁵ oder =CH-(Alk¹)ₙ-CONR¹⁴R¹⁵;
R⁶ ist Wasserstoff oder Methyl;
und pharmazeutisch akzeptable Salze davon.

29. Pharmazeutische Zusammensetzung, umfassend eine Verbindung der Formel (I) gemäß einem der Ansprüche 1 bis 28 zusammen mit einem pharmazeutisch akzeptablen Träger.

30. In vitro-Verfahren zur Inhibierung von Testosteron-5α-Reduktasen, umfassend das Inkontaktbringen von Testosteron-5α-Reduktasen mit einer Verbindung der Formel (I) gemäß einem der Ansprüche 1 bis 28.

31. Verwendung einer Verbindung der Formel (I) gemäß einem der Ansprüche 1 bis 28 oder eines physiologisch akzeptablen Salzes davon bei der Herstellung eines Medikaments zur Behandlung oder Verhinderung einer Krankheit, die auf Androgene anspricht oder durch Androgene vermittelt wird.

32. Verwendung gemäß Anspruch 31, wobei die auf Androgene ansprechende oder durch Androgene vermittelte Krankheit benigne Prostatahyperplasie, Prostatitis, Prostatakrebs, Akne, männlicher Haarausfall und Hirsutismus ist.

33. Verbindung gemäß einem der Ansprüche 1 bis 28 zur Verwendung in der Therapie.

34. Verfahren zur Herstellung einer Verbindung der Formel (I) gemäß Anspruch 1 oder eines pharmazeutisch akzeptablen Salzes oder Solvats davon, umfassend (A) die Umsetzung einer Verbindung der Formel (IX): 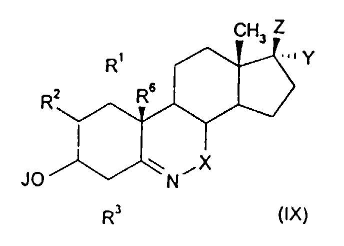 worin JO eine Hydroxy- oder geschützte Hydroxy-Gruppe ist, mit einem Oxidationsmittel; oder (B) zur Herstellung von Verbindungen der Formel (I), worin X -CH₂CH₂- ist, Umsetzung einer Verbindung der Formel (XV): 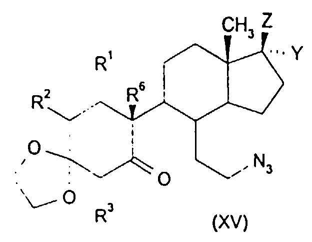 mit einem Reduktionsmittel und, falls erforderlich bzw. gewünscht, Unterwerfung der so erhaltenen
Verbindung einer oder mehrerer weiterer Reaktionen, umfassend
(i) Umwandlung der resultierenden Verbindung der Formel (I) oder eines Salzes oder geschützten Derivats davon in eine andere Verbindung der Formel (I); und/oder
(ii) Entfernung etwaiger Schutzgruppe(n); und/oder
(iii) Umwandlung einer Verbindung der Formel (I) oder eines Salzes davon in ein pharmazeutisch akzeptables Salz davon.

35. Verfahren gemäß Anspruch 34, wobei eine Verbindung der Formel (IX), worin JO eine geschützte Hydroxy-Gruppe ist, entschützt und dann mit einem geeigneten Oxidationsmittel oxidiert wird, so daß eine Verbindung der Formel (I) entsteht, in der R⁴ Wasserstoff ist.

36. Verfahren gemäß Anspruch 35, wobei das Oxidationsmittel Jones Reagens ist.

37. Verfahren gemäß Anspruch 34, wobei eine Verbindung der Formel (IX), worin JO eine geschützte Hydroxy-Gruppe ist, mit einem Acylierungsmittel behandelt, entschützt und dann mit einem geeigneten Oxidationsmittel oxidiert wird, so daß eine Verbindung der Formel (I) entsteht, in der R⁴ Acyl ist.

38. Verfahren gemäß Anspruch 37, wobei das Oxidationsmittel Pyridiniumdichromat ist.

39. Verfahren gemäß Anspruch 38, wobei eine Verbindung der Formel (XV) mit Triphenylphosphin umgesetzt wird, um eine Verbindung der Formel (I) herzustellen, in der X -CH₂CH₂- ist.

## Revendications

1. Composé de la formule (I): 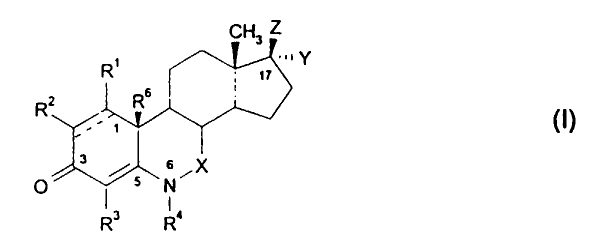 dans laquelle
R¹ et R²
i) représentent chacun indépendamment un atome d'hydrogène ou un radical alkyle inférieur et la liaison entre les carbones portant R¹ et R² est une simple liaison ou une double liaison,
ou bien
ii) considérés ensemble, ils sont un groupe -CH₂- qui forme un noyau cyclopropane et la liaison entre les carbones portant R¹ et R² est une simple liaison,
R³ est
un atome d'hydrogène, un groupe -Alk¹-H (éventuellement substitué par un ou plusieurs atomes d'halogènes), un radical cycloalkyle inférieur, cycloalkyle inférieur-alkyle inférieur, un atome d'halogène, un radical -(Alk¹)ₙ-CO₂H, -(Alk¹)ₙ-CO₂R⁷, -(Alk¹)ₙ-Ar¹, -(Alk¹)ₙ-CONR⁸R⁹, -(Alk¹)ₙ-NR⁸R⁹, -(Alk¹)ₙ-S(O)ᵣR⁷, -(Alk¹)ₙ-CN, -(Alk¹)OH, -(Alk¹)ₙ-CONR⁷ ou -(Alk¹)ₙ-OR⁷,
où
Alk¹ représente un groupe alkylène inférieur, alcénylène inférieur ou alcynylène inférieur,
n est égal à 0 ou à 1,
r est égal à 0, 1 ou 2,
R⁷ représente un groupe -Alk¹-H, -(Alk¹)ₙ-Ar¹ ou cycloalkyle inférieur,
R⁸ et R⁹ représentent chacun indépendamment un atome d'hydrogène, un groupe -Alk¹-H ou cycloalkyle inférieur,
Ar¹ représente un groupe aryle homocyclique de 6 à 14 atomes de carbone,
R⁴ est
un atome d'hydrogène, un radical -Alk¹-H, cycloalkyle inférieur, cycloalkyl inférieur-cycloalkyle inférieur, -(Alk¹)-ₙ-S(O)ᵣR⁷, -(Alk¹)ₙ-phtalimidyle, -(Alk¹)-CO₂H, -(Alk¹)ₙ-CO₂R⁷, -(Alk¹)ₙ-COR⁷, -(Alk¹)ₙ-Ar¹, -(Alk¹)ₙ-CONR⁸R⁹, -(Alk¹)ₙ-NR⁸R⁹, -(Alk¹)ₙ-OH ou -(Alk¹)ₙ-OR⁷,
X est
un groupe 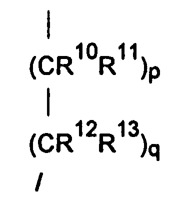 où
R¹⁰, R¹¹, R¹² et R¹³ représentent chacun indépendamment un atome d'hydrogène ou un groupe alkyle inférieur,
p et q sont chacun indépendamment égaux à 0 ou à 1,
et,
i) Y représente un atome d'hydrogène ou le radical hydroxyle et
Z est
-(Alk²)ₙ-COR⁵, -(Alk²)ₙ-CO₂R⁵, -(Alk²)ₙ-COSR⁵, -(Alk²)ₙ-CONR¹⁴R¹⁵, -(Alk²)ₙ-OCO₂R⁵, -(Alk²)OCOR⁵, -(Alk²)ₙ-OCONR¹⁴R¹⁵, -(Alk²)-OR⁵, -(Alk²)NR^{5'}COR⁵, -(Alk²)-NR^{5'}CO₂R⁵, -(Alk²)-NR⁵CONR¹⁴R¹⁵, -(Alk²)ₙ-CONR⁵NR¹⁴R¹⁵, -(Alk²)ₙ-CONR⁵CONR¹⁴R¹⁵, -(Alk²)NR⁵CSNR¹⁴R¹⁵ ou -(Alk²)ₙ-CONR⁵CSNR¹⁴R¹⁵,
où
Alk² est un groupe alkylène en (C₁ à C₁₂), alcénylène, en (C₂ à C₁₂), ou alcynylène en (C₂ à C₁₂),
R⁵ et R^{5'} représentent chacun indépendamment un atome d'hydrogène, un groupe -Alk¹-H (éventuellement substitué indépendamment par un ou plusieurs radicaux CO₂H, CO₂R⁷, Ar², Ar³ ou cyano), -(Alk¹)ₙ-(cycloalkyle inférieur) (éventuellement substitué indépendamment par un ou plusieurs radicaux -Alk¹-H)), adamantyle, norbornyle, Ar², Ar³, (cycloalkyle inférieur)-Ar² ou (cycloalkyle inférieur)-Ar³,
où
Ar² représente un radical aromatique homocyclique possédant de 6 à 14 atomes de carbone cycliques (éventuellement substitué indépendamment par un ou plusieurs radicaux -Alk²-H (éventuellement substitué indépendamment par un ou plusieurs atomes d'halogènes), un radical -(Alk¹)ₙ-COR⁷, -(Alk¹)ₙ-OH, -(Alk¹)ₙ-OR¹⁶, -(Alk¹)ₙ-Ar³, -(Alk¹)ₙ-CO₂H, -(Alk¹)ₙ-CO₂R⁷, S(O)ᵣR⁷, NR⁸S(O)ᵣR¹⁶, NR⁸R⁹, CONR⁸R⁹, cycloalkyle inférieur, alcoxy inférieur, -(Alk¹)ₙ-Ar¹ (éventuellement substitué par un ou plusieurs radicaux -Alk¹-H ou atomes d'halogènes), un radical méthylènedioxy, éthylènedioxy, morpholino, thiomorpholino, cyano, nitro ou des atomes d'halogènes),
où
R¹⁶ représente un groupe -Alk¹-H (éventuellement substitué indépendamment par un ou plusieurs atomes d'halogènes), un radical cycloalkyle inférieur (éventuellement substitué indépendamment par un ou plusieurs atomes d'halogènes, ou groupes -Alk¹-H (éventuellement substitué indépendamment par un ou plusieurs atomes d'halogènes)) ou un radical -(Alk¹)ₙ-Ar¹ (où Ar¹ est éventuellement substitué indépendamment par un ou plusieurs radicaux alcoxy inférieur, cyano, atomes d'halogènes ou -Alk¹-H (éventuellement substitué indépendamment par un ou plusieurs atomes d'halogènes)),
Ar³ représente un groupe aromatique possédant de 5 à 14 atomes cycliques, au moins l'un d'entre eux étant un atome d'oxygène, d'azote ou de soufre (éventuellement substitué indépendamment par un ou plusieurs radicaux -Alk¹-H (éventuellement substitué indépendamment par un ou plusieurs atomes d'halogènes), radicaux cycloalkyle inférieur, alcoxy inférieur, CO₂H, CO₂R⁷, -(Alk¹)ₙ-Ar¹, cyano ou atomes d'halogènes),
R¹⁴ et R¹⁵ sont
a) indépendamment chacun un groupe hydroxyle, un atome d'hydrogène, un groupe -Alk²-H, alcoxy inférieur, -(Alk¹)ₙ-adamantyle, -(Alk¹)ₙ-myrantyle, -(Alk¹)ₙ-norbornyle, -(Alk¹)ₙ-fluorényle, -(Alk¹)ₙ-fluorénonyle, -(Alk¹)ₙ-indanyle (éventuellement substitué par un ou plusieurs radicaux -Alk¹-H), -Alk¹-H (éventuellement substitué indépendamment par un ou plusieurs atomes d'halogènes, radicaux cyano, cycloalkyle, SR⁵, COR⁵, CONR⁵R⁷, NR^{5'}COR⁵, CONR^{5'}CO₂R⁵, NR^{5'}CONHR⁵, CO₂R⁵, OR⁵, Ar² ou Ar³), Ar² ou Ar³ ou un noyau bicyclique en C₄-C₁₈ saturé ou un noyau saturé en C₃ à C₁₁, contenant éventuellement un atome d'oxygène ou de soufre (lesdits noyaux étant éventuellement substitués indépendamment par un ou plusieurs radicaux cyano, R¹⁶, Ar², Ar³),
b) des radicaux alkylène (éventuellement substitués par un ou plusieurs groupes R⁷, considérés ensemble avec l'azote de liaison pour former un radical hétérocyclique à 4-8 atomes) : 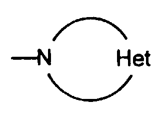 où Het représente -O-, -CH₂-, -S(O)ᵣ, (NH)- ou -(N(Alk¹-H))-,
avec la condition que
lorsque Z est -(Alk²)ₙ-COR⁵, -(Alk²)ₙ-CO₂R⁵, -(Alk²)ₙ-CO-thiopyridyle,
et
R⁵ est
hydrogène, -Alk¹-H, cycloalkyle inférieur ou adamantyle ou
lorsque Z est
-(Alk²)ₙ-CONR¹⁴R¹⁵
et
R¹⁴ et R¹⁵ sont
a) indépendamment de l'hydrogène un groupe -Alk²-H, cycloalkyle inférieur, alcoxy inférieur, adamantyle, -Ar¹, benzyle, diphénylméthyle, triphénylméthyle ou -(Alk¹)ₙ-norbornyle,
ou
b) des atomes de carbone, éventuellement substitués par un ou plusieurs radicaux alkyle inférieur, considérés ensemble avec l'azote de liaison pour former un noyau hétérocyclique à 4-8 atomes tel que précédemment défini,
Y représente le radical hydroxyle, ou
ii) Y est un atome d'hydrogène et
Z est
OR⁵, OCOR⁵, OCONR¹⁴R¹⁵, NR^{5'}COR⁵, NR^{5'}CO₂R⁵, NR⁵CON¹⁴R¹⁵ ou NR⁵CSR¹⁴R¹⁵, et
iii) Y et Z considérés ensemble sont
=0, =CH-(Alk¹)ₙ-COR⁵, =CH-(Alk¹)ₙ-CO₂R⁵ ou =CH-Alk¹)ₙ-CONR¹⁴R¹⁵,
R⁶ est
un atome d'hydrogène ou le radical méthyle,
ainsi que les sels pharmaceutiquement acceptables de ces composés.

2. Composé suivant la revendication 1, qui est un composé de la formule (IA), (IB), (IC) ou (ID):

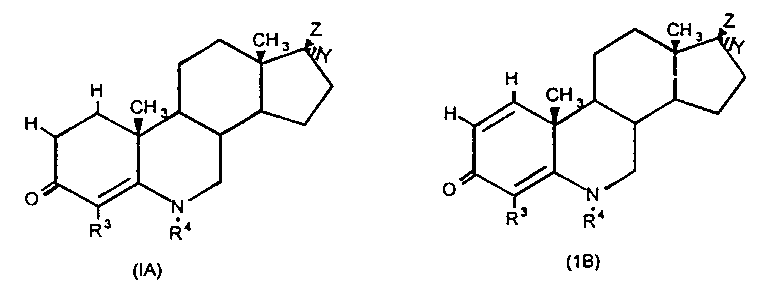

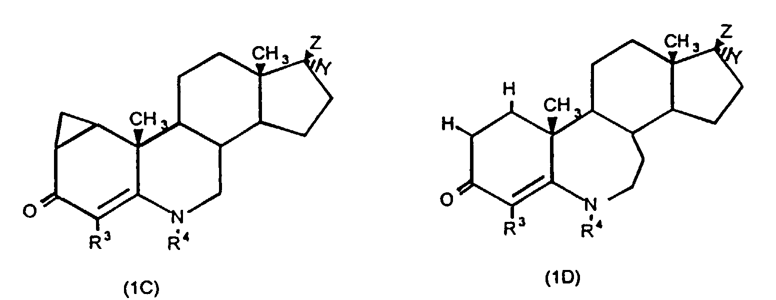

3. Composé suivant la revendication 1 ou la revendication 2, caractérisé en ce que R³ représente un atome d'hydrogène, un atome d'halogène, un radical alkyle inférieur, cycloalkyle inférieur ou cycloalkyl inférieur-alkyle inférieur.

4. Composé suivant la revendication 3, caractérisé en ce que R³ représente un atome d'hydrogène, un atome d'halogène ou un radical alkyle inférieur.

5. Composé suivant la revendication 1 ou la revendication 2, caractérisé en ce que R³ représente un atome d'iode, de brome, de chlore ou un radical méthyle, éthyle, cyano ou diméthylaminométhyle.

6. Composé suivant l'une quelconque des revendications précédentes, caractérisé en ce que R⁴ représente un atome d'hydrogène, un radical alkyle inférieur, cycloalkyle inférieur ou cycloalkyl inférieur-alkyle inférieur.

7. Composé suivant la revendication 6, caractérisé en ce que R⁴ représente un atome d'hydrogène ou un radical alkyle inférieur.

8. Composé suivant l'une quelconque des revendications 1 à 5, caractérisé en ce que R⁴ représente un radical méthyle, éthyle, propyle, i-propyle, butyle, i-butylhexyle, 3-hydroxypropyle, propényle, méthylène-cyclopropyle, benzyle, 2-méthoxyéthyle, acide 2-acétique, acide 3-propionique, acide 5-pentanoïque, acide 5-hexanoïque, 5-pentanoate de méthyle, 6-hexanoate d'éthyle, 3-phtalimidylpropyle et 4-phtalimidylpropyle.

9. Composé suivant l'une quelconque des revendications précédentes, caractérisé en ce que X est -CH₂-.

10. Composé suivant l'une quelconque des revendications précédentes, caractérisé en ce que Y représente un atome d'hydrogène et Z représente un groupe -(Alk²)ₙ-COR⁵, -(Alk²)ₙ-CONR¹⁴R¹⁵, -(Alk²)ₙ-OCO₂R⁵, -(Alk²)OCOR⁵, -(Alk²)NR^{5'}COR⁵, -(Alk²)ₙ-CONR⁵R¹⁴R¹⁵, -(Alk²)-NR⁵CONR¹⁴R¹⁵ ou -(Alk²)ₙ-CONR⁵CONR¹⁴R¹⁵.

11. Composé suivant la revendication 10, caractérisé en ce que Z représente un groupe COR⁵, -CONR¹⁴R¹⁵, -CH₂OCO₂R⁵, -CH₂OCOR⁵, -CH₂OCOR⁵, -CONR⁵NR¹⁴R¹⁵, -CH₂NR⁵CONR¹⁴R¹⁵ ou -CONR⁵CONR¹⁴R¹⁵.

12. Composé suivant la revendication 11, caractérisé en ce que Z représente le radical -CONR¹⁴R¹⁵.

13. Composé suivant l'une quelconque des revendications précédentes, caractérisé en ce que Ar¹ est un radical phényle.

14. Composé suivant l'une quelconque des revendications précédentes, caractérisé en ce que Ar² est un radical phényle éventuellement substitué indépendamment par un ou plusieurs radicaux -Alk²-H (éventuellement substitué indépendamment par un ou plusieurs atomes d'halogènes), -OR¹⁶, -S(O)ᵣR⁷, -Ar¹, méthylènedioxy, éthylènedioxy, morpholino, thiomorpholino, cyano, nitro, ou atomes d'halogènes.

15. Composé suivant l'une quelconque des revendications précédentes, caractérisé en ce que R¹⁴ est un atome d'hydrogène ou le radical hydroxyle et R¹⁵ est un atome d'hydrogène, un radical cycloalkyle inférieur (éventuellement substitué indépendamment par un ou plusieurs radicaux R⁷ ou Ar²), -(Alk¹)ₙ-adamantyle, -(Alk¹)ₙ-myrantyle, -(Alk¹)ₙ-norbornyle, -(Alk¹)ₙ-fluorényle, -(Alk¹)ₙ-indanyle, -Alk¹-H (éventuellement substitué indépendamment par un ou plusieurs radicaux cycloalkyle inférieur, SR⁵, OR⁵, Ar² ou Ar³), Ar² ou Ar³; ou bien
R¹⁴ et R¹⁵ sont des atomes de carbone, éventuellement substitués par un ou plusieurs radicaux R⁷, considérés ensemble avec l'azote de liaison pour former un groupe hétérocyclique à 5-7 atomes 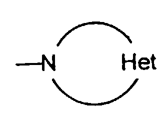 où Het représente -CH₂-.

16. Composé suivant l'une quelconque des revendications précédentes, caractérisé en ce que Ar³ représente un groupe aromatique comportant cinq ou six atomes cycliques, au moins l'un de ceux-ci étant un atome d'oxygène, d'azote ou de soufre, éventuellement substitué indépendamment par un ou plusieurs radicaux Alk¹H.

17. Composition suivant la revendication 16, caractérisé en ce que Ar³ est un groupe pyrrolyle, thiényle, furyle ou pyridyle, éventuellement substitué.

18. Composé suivant l'une quelconque des revendications précédentes, caractérisé en ce que R⁵ représente un atome d'hydrogène, un radical alkyle inférieur éventuellement substitué indépendamment par un ou plusieurs groupes Ar², (alkyl inférieur)ₙ-cycloalkyle inférieur, menthyle, adamantyle, norbornyle ou Ar².

19. Composé suivant l'une quelconque des revendications 1 à Il ou 13 à 18, caractérisé en ce que Z est un radical -COR⁵.

20. Composé suivant la revendication 19, caractérisé en ce qu'il est l'une des substances qui suivent:
17β-(1-oxo-2-cyclohexyléthyl)-6-azaandrost-4-ène-3-one
17β-(1-oxo-1-(2,4-difluorophényl)méthyl)-6-azaandrost-4-ène-3-one
17β-(1-oxo-1-(4-isopropoxyphényl)méthyl)-6-azaandrost-4-ène-3-one
17β-(1-oxo-3,3-diphénylpropyl)-6-azaandrost-4-ène-3-one, ou
17β-(1-oxo-1-(2-norbornyl)méthyl)-6-azaandrost-4-ène-3-one , ou
un sel pharmaceutiquement acceptable ou un solvate d'un tel composé.

21. Composé suivant l'une quelconque des revendications 1 à 18, caractérisé en ce que Z est -CONR¹⁴R¹⁵, R¹⁴ représente un atome d'hydrogène et R¹⁵ représente un groupe Ar² ou un noyau en C₃ à C₁₁ saturé, contenant éventuellement un atome d'oxygène ou un atome de soufre (éventuellement substitué indépendamment par un ou plusieurs radicaux R⁷ ou Ar²).

22. Composé suivant l'une quelconque des revendications précédentes, caractérisé en ce que R¹⁵ est un groupe de la formule Ar^{2a} : 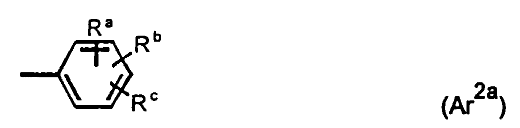 dans laquelle R^{a} et R^{b} représentent chacun indépendamment un atome d'hydrogène, un radical alkyle inférieur, trifluorométhyle, un atome d'halogène ou un radical phényle (éventuellement substitué par un ou plusieurs atomes d'halogènes ou radicaux alkyle en C₄ à C₇ ramifiés) et R^{C} représente un atome d'hydrogène ou un ou plusieurs atomes d'halogènes, ou bien R¹⁵ représente un noyau en C₃ à C₁₁ saturé, contenant éventuellement un atome d'oxygène ou un atome de soufre substitué par un groupe de la formule Ar^{2a}.

23. Composé suivant la revendication 22, caractérisé en ce que Ar^{2a} représente un groupe de la formule Ar^{2aa} : 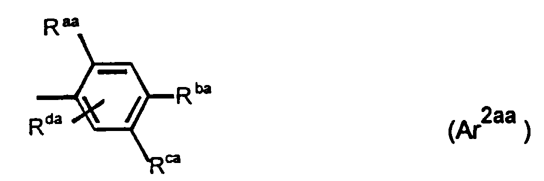 dans laquelle R^{aa} représente un radical alkyle en C₄ à C₇ ramifié, trifluorométhyle ou phényle éventuellement substitué par un ou plusieurs atomes d'halogènes; l'un des symboles R^{ba} et R^{ca} représente un radical alkyle en C₄ à C₇ ramifié, trifluorométhyle, un atome d'halogène ou un groupe phényle éventuellement substitué par un ou plusieurs atomes d'halogènes, cependant que l'autre de ces symboles représente un atome d'hydrogène ou un atome d'halogène et R^{da} représente un atome d'hydrogène ou un atome d'halogène.

24. Composé suivant la revendication 1, caractérisé en ce qu'il est l'une des substances qui suivent:
17β-N-((2,6-di-i-propyl)phényl)-carbamoyl-6-azaandrost-4-ène-3-one
17β-N-(2,4,6-triméthyl)phényl-carbamoyl-6-azaandrost-4-ène-3-one
17β-N-(2-chloro-5-trifluorométhyl)phényl-carbamoyl-6-azaandrost-4-ène-3-one
17β-N-(2,6-diméthyl-4-bromo)phényl-carbamoyl-6-azaandrost-4-ène-3-one
17β-N-(2,6-diméthyl-4-butyl)phényl-carbamoyl-6-azaandrost-4-ène-3-one
17β-N-(2,6-dibromo-4-isopropyl)phényl-carbamoyl-6-azaandrost-4-ène-3-one
17β-N-(2,5-ditrifluorométhyl)phényl-carbamoyl-6-azaandrost-4-ène-3-one
17β-N-(2-phényl)phényl-carbamoyl-4-azaandrost-4-ène-3-one
17β-N-(2,6-diéthyl-3,5-dichloro)phényl-carbamoyl-6-azaandrost-4-ène-3-one
17β-N-(2,6-diéthyl-3-chloro)phényl-carbamoyl-6-azaandrost-4-ène-3-one
17β-N-(2-t-butyl)phényl-carbamoyl-4-azaandrost-4-ène-3-one
17β-N-(2,4,6-trichloro)phényl-carbamoyl-6-azaandrost-4-ène-3-one
17β-N-(2-bromo-5-trifluorométhyl)phényl-carbamoyl-6-azaandrost-4-ène-3-one
17β-N-(2-t-butyl-6-méthyl)phényl-carbamoyl-6-azaandrost-4-ène-3-one
17β-N-1-(4-chlorophényl)cyclopentyl-carbamoyl-6-azaandrost-4-ène-3-one
17β-N-(2,6-dibromo-4-chloro)phényl-carbamoyl-6-azaandrost-4-ène-3-one
17β-N-(2,6-diéthyl-4-bromo)phényl-carbamoyl-6-azaandrost-4-ène-3-one
17β-N-(2-bromo-4-t-butyl)phényl-carbamoyl-6-azaandrost-4-ène-3-one
17β-N-(2-chloro-4-t-butyl)phényl-carbamoyl-6-azaandrost-4-ène-3-one
17β-N-(5-bromo-2-t-butyl)phényl-carbamoyl-6-azaandrost-4-ène-3-one
17β-N-(5-chloro-2-t-butyl)phényl-carbamoyl-6-azaandrost-4-ène-3-one
17β-N-(2,6-diéthyl)phényl-carbamoyl-6-azaandrost-4-ène-3-one
17β-N-(4-bromo-2-t-butyl)phényl-carbamoyl-6-azaandrost-4-ène-3-one
17β-N-(2-t-butyl-5-cyano)phényl-carbamoyl-6-azaandrost-4-ène-3-one
17β-N-(2-(O-4-tolyl)-5-trifluorométhyl)phényl-carbamoyl-6-azaandrost-4-ène-3-one
17β-N-(2-(O-4-chlorophényl)-5-trifluorométhyl)phényl-carbamoyl-6-azaandrost-4-ène-3-one
17β-N-(2-nitro-4-t-butyl)phényl-carbamoyl-6-azaandrost-4-ène-3-one
17β-N-(2-(O-phényl)-5-(1,1-diméthyl)propyl)phényl-carbamoyl-6-azaandrost-4-ène-3-one
17β-N-(2-éthylsulfonyl-5-trifluorométhyl)phényl-carbamoyl-6-azaandrost-4-ène-3-one
17β-N-(3,5-di-t-butyl)phényl-carbamoyl-6-azaandrost-4-ène-3-one
17β-N-(2-t-butyl-5-trifluorométhyl)phényl-carbamoyl-6-azaandrost-4-ène-3-one
17β-N-(2-t-butyl-5-phényl)phényl-carbamoyl-6-azaandrost-4-ène-3-one
17β-N-(2,6-di-i-propyl)phényl-carbamoyl-6-azaandrost-1,4-diène-3-one
17β-N-(2,6-di-i-propyl)phényl-carbamoyl-6-azaandrost-4-méthyl-1,4-diène-3-one
17β-N-1-(4-trifluorométhylphényl)cyclopentyl-carbamoyl-6-azaandrost-4-ène-3-one
17β-N-1-(4-fluorophényl)cyclohexyl-carbamoyl-6-azaandrost-4-ène-3-one
17β-N-1-(4-méthoxyphényl)cyclohexyl-carbamoyl-6-azaandrost-4-ène-3-one
17β-N-1-(4-méthoxyphényl)cyclopentyl-carbamoyl-6-azaandrost-4-ène-3-one
17β-N-(2,5-bis-(trifluorométhyl))phényl-carbamoyl-6-azaandrost-4-ène-3-one
17β-N-(2-t-butyl-5-trifluorométhyl)phényl-carbamoyl-6-azaandrost-4-ène-3-one
17β-N-(2,5-di-t-butyl)phényl-carbamoyl-6-azaandrost-4-ène-3-one
17β-N-(2-t-butyl-5-(4-chlorophényl))-phénylcarbamoyl-6-azaandrost-4-ène-3-one
17β-N-1-(4-t-butylphényl)cyclopentyl-carbamoyl-6-azaandrost-4-ène-3-one
17β-N-1-(4-t-butylphényl)cyclohexyl-carbamoyl-6-azaandrost-4-ène-3-one
17β-N-1(4-chlorophényl)cyclopentyl-carbamoyl-6-azaandrost-4-ène-3-one
17β-N-(2,5-bis-(trifluorométhyl))phényl-carbamoyl-6-azaandrost-4-ène-3-one
17β-N-(2-t-butyl-5-trifluorométhyl)phényl-carbamoyl-6-azaandrost-4-ène-3-one
17β-N-1-(4-t-butylphényl)cycloheptyl-carbamoyl-6-azaandrost-4-ène-3-one
17β-N-1-(4-t-butylphényl)cyclohexyl-carbamoyl-6-azaandrost-4-ène-3-one
17β-N-(2,6-diéthyl-4-(4-chlorophényl))phényl-carbamoyl-6-azaandrost-4-ène-3-one
17β-N-(4-(4-t-butylphényl)tétrahydrothiopyrannyl-carbamoyl-6-azaandrost-4-ène-3-one
17β-N-9-(4-t-butylphényl)bicyclo[3.3.1]nonyl-carbamoyl-6-azaandrost-4-ène-3-one
17β-N-4-(4-t-butylphényl)tétrahydropyrannyl-carbamoyl-6-azaandrost-4-ène-3-one
17β-N-1-(4-chlorophényl)cyclohexyl-carbamoyl-6-azaandrost-4-ène-3-one
17β-N-(2-t-butyl-5-(4-t-butyl)phényl)phényl-carbamoyl-6-azaandrost-4-ène-3-one
ou
17β-N-1-(4-chlorophényl)cyclopentyl-carbamoyl-6-azaandrost-4-ène-3-one
ou un sel pharmaceutiquement acceptable ou un solvate d'un tel composé.

25. 17β-N-(2-t-butyl-5-trifluorométhyl)phényl-carbamoyl-6-azaandrost-4-ène-3-one,
ou un sel pharmaceutiquement acceptable ou un solvate d'un tel composé.

26. Composé de la formule (I): 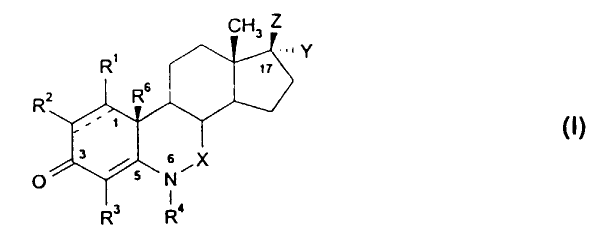 dans laquelle
R¹ et R²
i) représentent chacun indépendamment un atome d'hydrogène ou un radical alkyle inférieur et la liaison entre les carbones portant R¹ et R² est une simple liaison ou une double liaison, ou bien
ii) considérés ensemble, ils sont un groupe -CH₂- qui forme un noyau cyclopropane et la liaison entre les carbones portant R¹ et R² est une simple liaison,
R³ représente un atome d'hydrogène ou un radical alkyle inférieur;
R⁴ représente un atome d'hydrogène ou un radical alkyle inférieur;
X est un groupe 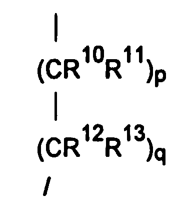 où
R¹⁰, R¹¹, R¹² et R¹³ représentent chacun indépendamment un atome d'hydrogène ou un groupe alkyle inférieur,
p et q sont chacun indépendamment égaux à 0 ou à 1;
Y représente un atome d'hydrogène, et
Z représente un groupe -COR⁵ ,OCONR¹⁴R¹⁵, -CH₂OCO₂COR⁵, -CH₂OCOR⁵, -CH₂NR^{5'}COR⁵, OCONR⁵NR¹⁴R¹⁵, -CH₂NR⁵CONR¹⁴R⁵ ou -CON⁵CONR¹⁴R¹⁵, où R⁵ et R^{5'} représentent chacun indépendamment un atome d'hydrogène, un radical alkyle inférieur éventuellement substitué indépendamment par un ou plusieurs radicaux Ar², (alkyl inférieur)ₙ-cycloalkyle inférieur, menthyle, adamantyle, norbornyle ou Ar²;
Ar² représente un groupe phényle éventuellement substitué indépendamment par un ou plusieurs radicaux -Alk-H (éventuellement substitué indépendamment par un ou plusieurs atomes d'halogènes), -OR¹⁶, -S(O)ᵣR⁷, phényle, méthylènedioxy, éthylènedioxy, morpholino, thiomorpholino, cyano, nitro ou atomes d'halogènes,
Alk représente un groupe alkylène inférieur, alcénylène inférieur ou alcynylène inférieur,
n est égal à 0 ou à 1,
r est égal à 0, 1 ou 2,
R⁷ représente un groupe -Alk-H, -(Alk)ₙ-phényle ou cycloalkyle inférieur,
R¹⁶ représente un groupe -Alk-H (éventuellement substitué indépendamment par un ou plusieurs atomes d'halogènes), cycloalkyle inférieur (éventuellement substitué indépendamment par un ou plusieurs atomes d'halogènes ou -Alk-H (éventuellement substitué indépendamment par un ou plusieurs atomes d'halogènes)) ou -(Alk)ₙ-phényle (ou le groupe phényle est éventuellement substitué indépendamment par un ou plusieurs radicaux alcoxy inférieur, cyano, atomes d'halogènes ou -Alk-H (éventuellement substitué indépendamment par un ou plusieurs atomes d'halogènes)), R¹⁴ représente un atome d'hydrogène ou le radical hydroxyle, et R¹⁵ représente un atome d'hydrogène, un groupe cycloalkyle inférieur (éventuellement substitué indépendamment par un ou plusieurs radicaux R⁷ ou Ar²), -(Alk)-adamantyle, -(Alk)ₙ-myrantyle, -(Alk)ₙ-norbornyle, -(Alk)ₙ-fluorényle, -(Alk)ₙ-indanyle, -Alk-H (éventuellement substitué indépendamment par un ou plusieurs radicaux cycloalkyle inférieur, SR⁵, OR⁵, Ar² ou Ar³), Ar² ou Ar³, ou bien R¹⁴ et R¹⁵, considérés ensemble avec l'azote de liaison, forment un cycle pyrrolidinyle, pipéridnyle ou perhydroazépinyle éventuellement substitué par un ou plusieurs radicaux R⁷, Ar³ représente un groupe pyrrolyle, thiényle, furyle ou pyridyle, éventuellement substitué indépendamment par un ou plusieurs radicaux Alk-H, avec les conditions que
lorsque Z est COR⁵, R⁵ représente un groupe alkyle inférieur substitué, alkyl inférieur-cycloalkyle inférieur, menthyle, norbornyle ou Ar²,
lorsque Z représente un groupe CONR¹⁴R¹⁵ et R¹⁵ est un atome d'hydrogène, un radical -Alk-H, cycloalkyle inférieur, alcoxy inférieur, adamantyle, phényle, benzyle, diphénylméthyle, triphénylméthyle ou -(Alk)ₙ-norbornyle, R¹⁴ représente le radical hydroxyle, et
lorsque Z représente un groupe CONR¹⁴R¹⁵ et R¹⁴ et R¹⁵ considérés ensemble avec l'azote de liaison forment un cycle pyrrolidinyle, pipéridinyle ou perhydroazépinyle, ledit cycle soit substitué par un ou plusieurs radicaux alcényle inférieur, alcynyle inférieur, -(Alk)ₙ-phényle ou cycloalkyle inférieur, ainsi que les sels pharmaceutiquement acceptables de ce composé.

27. Composé de la formule (I): 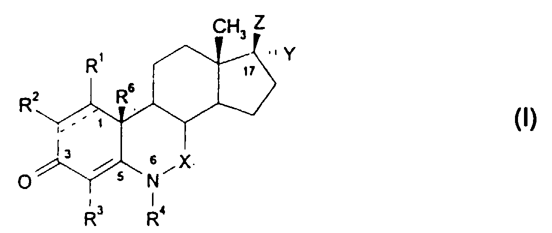
R¹ et R²
i) représentent chacun indépendamment un atome d'hydrogène ou un radical alkyle inférieur et la liaison entre les carbones portant R¹ et R² est une simple liaison ou une double liaison,
ou bien
ii) considérés ensemble, ils sont un groupe -CH₂- qui forme un noyau cyclopropane et la liaison entre les carbones portant R¹ et R² est une simple liaison,
R³ est
un atome d'hydrogène, un groupe -Alk¹-H, -Alk¹-H substitué par un ou plusieurs atomes d'halogènes, un radical cycloalkyle inférieur, cycloalkyl inférieur-alkyle inférieur, un atome d'halogène, un radical -(Alk¹)ₙ-CO₂H, -(Alk¹)ₙ-CO₂R⁷, -(Alk¹)ₙ-Ar¹, ,-(Alk¹)ₙ-CONR⁸R⁹, -(Alk¹)ₙ-NR⁸R⁹, -(Alk¹)ₙ-S(O)ᵣR⁷, -(Alk¹)ₙ-CN, -(Alk¹)-OH ou -(Alk¹)ₙ-OR⁷,
où
Alk¹ représente un groupe alkylène inférieur, alcénylène inférieur ou alcynylène inférieur,
n est égal à 0 ou à 1,
r est égal à 0, 1 ou 2,
R⁷ représente un groupe -Alk¹-H, -(Alk¹)ₙ-Ar¹ ou cycloalkyle inférieur,
R⁸ et R⁹ représentent indépendamment chacun un atome d'hydrogène, un groupe -Alk¹-H ou cycloalkyle inférieur,
Ar¹ représente un groupe aryle homocyclique de 6 à 14 atomes de carbone,
R⁴ est
un atome d'hydrogène, un radical -Alk¹-H, cycloalkyle inférieur, cycloalkyl inférieur-alkyle inférieur, -(Alk¹)-ₙ-S(O)ᵣR⁷, -(Alk¹)ₙ-phtalimidyle, -(Alk¹)-CO₂H, -(Alk¹)ₙ-CO₂R⁷, -(Alk¹)ₙ-Ar¹, -(Alk¹)ₙ-CONR⁸R⁹, -(Alk¹)ₙ-NR⁸R⁹, -(Alk¹)ₙ-OH ou -(Alk¹)ₙ-OR⁷,
X est un groupe 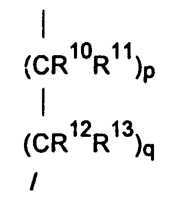 où
R¹⁰, R¹¹, R¹² et R¹³ représentent chacun indépendamment un atome d'hydrogène ou un groupe alkyle inférieur,
p et q sont chacun indépendamment égaux à 0 ou à 1,
Y et Z sont
i) Y représente un atome d'hydrogène ou le radical hydroxyle, et
Z est -(Alk²)ₙ-COR⁵, -(Alk²)ₙ-CO₂R⁵, -(Alk²)ₙ-COSR⁵, -(Alk²)ₙ-CONR¹⁴R¹⁵, -(Alk²)-OCOR⁵, -(Alk²)-OCONR¹⁴R¹⁵, -(Alk²)-OR⁵, -(Alk²)-NR^{5'}COR⁵, -(Alk²)-NR⁵CO₂R⁵, -(Alk²)-NR⁵CONR¹⁴R¹⁵, -(Alk²)ₙ-CONR⁵NR¹⁴R¹⁵, -(Alk²)ₙ-CONR⁵CONR¹⁴R¹⁵, -(Alk²)NR⁵CSNR¹⁴R¹⁵ ou -(Alk²)ₙ-CONR⁵CSNR¹⁴R¹⁵,
où
Alk² est un groupe alkylène en (C₁ à C₁₂), alcénylène, en (C₂ à C₁₂), ou alcynylène en (C₂ à C₁₂),
R⁵ représente un atome d'hydrogène, un groupe -Alk¹-H, -(Alk¹)ₙ-(cycloalkyle inférieur), adamantyle, -(Alk¹)ₙ-Ar², -(Alk¹)ₙ-Ar³, (cycloalkyle inférieur)ₙAr², (cycloalkyle inférieur)ₙAr³ ou -Alk¹-substitué indépendamment par un ou plusieurs radicaux CO₂H, CO₂R⁷, Ar² ou Ar³,
où
Ar² représente un radical aromatique possédant de 6 à 14 atomes de carbone cycliques, éventuellement substitué par un ou plusieurs radicaux Alk¹, Alk¹ substitué par un ou plusieurs atomes d'halogènes, -(Alk¹)ₙ-OH, -(Alk¹)ₙ-OR⁷, -(Alk¹)ₙ-CO₂H, -(Alk¹)ₙ-CO₂R⁷, S(O)ᵣR⁷ ou NR⁸R⁹, cycloalkyle inférieur, alcoxy inférieur, -(Alk¹)ₙ-Ar¹, méthylènedioxy, éthylènedioxy, morpholino, thiomorpholino, cyano ou atomes d'halogènes,
Ar³ représente un groupe aromatique possédant de 5 à 14 atomes cycliques, au moins l'un d'entre eux étant un atome d'oxygène, d'azote ou de soufre, éventuellement substitué par un ou plusieurs radicaux Alk¹, cycloalkyle inférieur, alcoxy inférieur, CO₂H, CO₂R⁷, -(Alk¹)ₙ-Ar¹, cyano ou atomes d'halogènes,
R¹⁴ et R¹⁵ sont
a) chacun indépendamment un atome d'hydrogène ou un groupe -Alk²-H, cycloalkyle inférieur, alcoxy inférieur, adamantyle, -(Alk¹)ₙ-norbornyle, Ar², Ar³, -Alk¹- indépendamment substitué par un ou plusieurs radicaux SR⁵, COR⁵, CONR⁵R⁷, NR⁵COR⁵, NR⁵CO₂R⁵, NR⁵CONHR⁵, CO₂R⁵, OR⁵, Ar² ou Ar³, (Alk¹)ₙ-fluorényle ou (Alk¹)ₙ-indanyle, ou
b) des atomes de carbone, éventuellement substitués par un ou plusieurs radicaux alkyle inférieur, considérés ensemble avec l'atome d'azote de liaison pour former un radical hétérocyclique à 4-8 atomes : 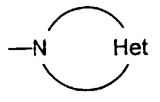 où
Het représente -O-, -CH₂-, -S(O)ᵣ, (NH)- ou -(N(Alk¹-H))-,
avec la condition que
lorsque Z est
-(Alk²)ₙ-COR⁵, -(Alk²)ₙ-CO₂R⁵ ou -(Alk²)ₙ-CO-thiopyridyle et R⁵ est un atome d'hydrogène, un groupe -Alk¹-H, cycloalkyle inférieur, -(Alk¹)ₙ-Ar¹, adamantyle ou
lorsque Z
est -(Alk²)ₙ-CONR¹⁴R¹⁵ et R¹⁴ et R¹⁵ sont
a) chacun indépendamment un atome d'hydrogène, un radical -Alk²-H, cycloalkyle inférieur, alcoxy inférieur, adamantyle, -Ar¹, benzyle, diphénylméthyle, triphénylméthyle ou -(Alk¹)ₙ-norbornyle,
ou
b) des atomes de carbone, éventuellement substitués par un ou plusieurs radicaux alkyle inférieur, considérés ensemble avec l'azote de liaison pour former un noyau hétérocyclique à 4-8 atomes tel que précédemment défini,
Y représente le radical hydroxyle,
ii) Y représente un atome d'hydrogène et
Z représente un groupe
OR⁵, OCOR⁵, OCONR¹⁴R¹⁵, NR⁵COR⁵, NR⁵CO₂R⁵, NR⁵CONR¹⁴R¹⁵ ou NR⁵CSR¹⁴R¹⁵,
iii) Y et Z considérés ensemble sont des radicaux =0, =CH-(Alk¹)ₙ-COR⁵, =CH-(Alk¹)ₙ-CO₂R⁵ ou =CH-(Alk¹)ₙ-CONR¹⁴R¹⁵,
R⁶ représente
un atome d'hydrogène ou le radical méthyle,
et les sels pharmaceutiquement acceptables de ces composés.

28. Composé de la formule (I): 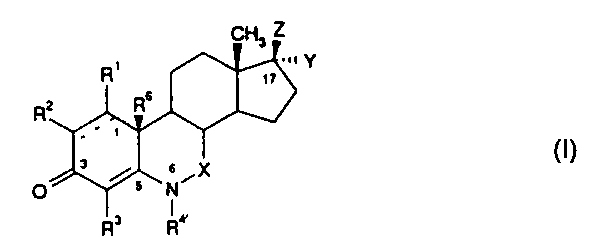 dans laquelle
R¹ et R²
i) représentent chacun indépendamment un atome d'hydrogène ou un radical alkyle inférieur et la liaison entre les carbones portant R¹ et R² est une simple liaison ou une double liaison,
ou bien
ii) considérés ensemble, ils sont un groupe -CH₂- qui forme un noyau cyclopropane et la liaison entre les carbones portant R¹ et R² est une simple liaison,
R³ est
un atome d'hydrogène, un groupe -Alk¹-H (éventuellement substitué par un ou plusieurs atomes d'halogènes), un radical cycloalkyle inférieur, cycloalkyl inférieur-alkyle inférieur, un atome d'halogène, un radical -(Alk¹)ₙ-CO₂H, -(Alk¹)ₙ-CO₂R⁷, -(Alk¹)ₙ-Ar¹, -(Alk¹)ₙ-CONR⁸R⁹, -(Alk¹)ₙ-NR⁸R⁹, -(Alk¹)ₙ-S(O)ᵣR⁷, -(Alk¹)ₙ-CN, -(Alk¹)-OH, -(Alk¹)ₙ-COR⁷ ou -(Alk¹)ₙ-OR⁷,
où
Alk¹ représente un groupe alkylène inférieur, alcénylène inférieur ou alcynylène inférieur,
n est égal à 0 ou à 1,
r est égal à 0, 1 ou 2,
R⁷ représente un groupe -Alk¹-H, -(Alk¹)ₙ-Ar¹ ou cycloalkyle inférieur,
R⁸ et R⁹ représentent chacun indépendamment un atome d'hydrogène, un groupe -Alk¹-H ou cycloalkyle inférieur,
Ar¹ représente un groupe aryle homocyclique de 6 à 14 atomes de carbone,
R⁴ est
un atome d'hydrogène, un radical -Alk¹-H, cycloalkyle inférieur, cycloalkyl inférieur-alkyle inférieur, -(Alk¹)-ₙ-S(O)ᵣR⁷, -(Alk¹)ₙ-phtalimidyle, -(Alk¹)-CO₂H, -(Alk¹)ₙ-CO₂R⁷, -(Alk¹)ₙ-COR⁷, -(Alk¹)ₙ-Ar¹, -(Alk¹)ₙ-CONR⁸R⁹, -(Alk¹)ₙ-NR⁸R⁹, -(Alk¹)ₙ-OH ou -(Alk¹)ₙ-OR⁷,
X est
un groupe 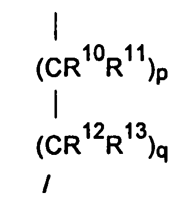 où
R¹⁰, R¹¹, R¹² et R¹³ représentent chacun indépendamment un atome d'hydrogène ou un groupe alkyle inférieur,
p et q sont chacun indépendamment égaux à 0 ou à 1,
et,
i) Y représente un atome d'hydrogène ou le radical hydroxyle et
Z est -(Alk²)ₙ-COR⁵, -(Alk²)ₙ-CO₂R⁵, -(Alk²)ₙ-COSR⁵, -(Alk²)ₙ-CONR¹⁴R¹⁵, -(Alk²)ₙ-OCO₂R⁵, -(Alk²)-OCOR⁵, -(Alk²)ₙ-OCONR¹⁴R¹⁵, -(Alk²)-OR⁵, -(Alk²)-NR^{5'}COR⁵, -(Alk²)-NR^{5'}CO₂R⁵, -(Alk²)-NR⁵CONR¹⁴R¹⁵, -(Alk²)ₙ-CONR⁵NR¹⁴R¹⁵, -(Alk²)ₙ-CONR⁵CONR¹⁴R¹⁵, -(Alk²)-NR⁵CSNR¹⁴R¹⁵ ou -(Alk²)ₙ-CONR⁵CSNR¹⁴R¹⁵,
où
Alk² est un groupe alkylène en (C₁ à C₁₂), alcénylène, en (C₂ à C₁₂), ou alcynylène en (C₂ à C₁₂),
R⁵ et R^{5'} représentent chacun indépendamment un atome d'hydrogène, un groupe -Alk¹-H (éventuellement substitué indépendamment par un ou plusieurs radicaux CO₂H, CO₂R⁷, Ar², Ar³ ou cyano), -(Alk¹)ₙ-cycloalkyle inférieur) (éventuellement substitué indépendamment par un ou plusieurs radicaux -Alk¹-H), adamantyle, norbornyle, Ar², Ar³, (cycloalkyle inférieur)-Ar² ou (cycloalkyle inférieur)-Ar³,
où
Ar² représente un radical aromatique homocyclique possédant de 6 à 14 atomes de carbone cycliques (éventuellement substitué indépendamment par un ou plusieurs radicaux -Alk²-H (éventuellement substitué indépendamment par un ou plusieurs atomes d'halogènes), un radical -(Alk¹)ₙ-OH, -(Alk¹)ₙ-OR¹⁶, -(Alk¹)ₙ-Ar³, -(Alk¹)ₙCO₂H, -(Alk¹)ₙ-CO₂R⁷, S(O)ᵣR⁷, NR⁸S(O)ᵣR¹⁶, NR⁸R⁹, CONR⁸R⁹, cycloalkyle inférieur, alcoxy inférieur, -(Alk¹)ₙ-Ar¹, un radical méthylènedioxy, éthylènedioxy, morpholino, thiomorpholino, cyano, nitro ou des atomes d'halogènes),
où
R¹⁶ représente un groupe -Alk¹-H (éventuellement substitué indépendamment par un ou plusieurs atomes d'halogènes), un radical cycloalkyle inférieur (éventuellement substitué indépendamment par un ou plusieurs atomes d'halogènes, ou groupes -Alk¹-H (éventuellement substitué indépendamment par un ou plusieurs atomes d'halogènes)) ou un radical -(Alk¹)ₙ-Ar¹ (où Ar¹ est éventuellement substitué indépendamment par un ou plusieurs radicaux alcoxy inférieur, cyano, atomes d'halogènes ou -Alk¹-H (éventuellement substitué indépendamment par un ou plusieurs atomes d'halogènes)),
Ar³ représente un groupe aromatique possédant de 5 à 14 atomes cycliques, au moins l'un d'entre eux étant un atome d'oxygène, d'azote ou de soufre (éventuellement substitué indépendamment par un ou plusieurs radicaux -Alk¹-H (éventuellement substitué indépendamment par un ou plusieurs atomes d'halogènes), radicaux cycloalkyle inférieur, alcoxy inférieur, CO₂H, CO₂R⁷, -(Alk¹)ₙ-Ar¹, cyano ou atomes d'halogènes),
R¹⁴ et R¹⁵ sont
a) indépendamment chacun un groupe hydroxyle, un atome d'hydrogène, un groupe -Alk²-H, cycloalkyle inférieur (éventuellement substitué indépendamment par un ou plusieurs radicaux cyano, R¹⁶, Ar², Ar³), alcoxy inférieur, -(Alk¹)ₙ-adamantyle, -(Alk¹)ₙ-myrantyle, -(Alk¹)ₙ-norbornyle, -Alk¹)ₙ-fluorényle, -(Alk¹)ₙ-indanyle, -Alk¹-H (éventuellement substitué indépendamment par un ou plusieurs atomes d'halogènes, radicaux cyano, cycloalkyle, SR⁵, COR⁵, CONR⁵R⁷, NR^{5'}COR⁵, NR^{5'}CO₂R⁵, NR^{5'}CONHR⁵, CO₂R⁵, OR⁵, Ar² ou Ar³), Ar² ou Ar³,
b) des radicaux alkylène (éventuellement substitués par un ou plusieurs groupes R⁷, considérés ensemble avec l'azote de liaison pour former un radical hétérocyclique à 4-8 atomes) : 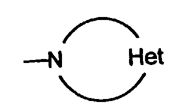 où
Het représente -O-, -CH₂-, -S(O)ᵣ, (NH)- ou -(N(Alk¹-H))-, avec la condition que
lorsque Z est -(Alk²)ₙ-COR⁵, -(Alk²)ₙ-CO₂R⁵ ou -(Alk²)ₙ-CO-thiopyridyle,
et
R⁵ est
hydrogène, -Alk¹-H, cycloalkyle inférieur, -(Alk¹)ₙ-Ar¹ ou adamantyle, ou
lorsque Z est
-(Alk²)ₙ-CONR¹⁴R¹⁵
et
R¹⁴ et R¹⁵ sont
a) indépendamment un atome d'hydrogène un groupe -Alk²-H, cycloalkyle inférieur, alcoxy inférieur, adamantyle, Ar¹, benzyle, diphénylméthyle, triphénylméthyle ou -(Alk¹)ₙ-norbornyle,
ou
b) des atomes de carbone, éventuellement substitués par un ou plusieurs radicaux alkyle inférieur, considérés ensemble avec l'azote de liaison pour former un noyau hétérocyclique à 4-8 atomes tel que précédemment défini,
Y représente le radical hydroxyle, ou
ii) Y est un atome d'hydrogène et
Z est
OR⁵, OCOR⁵, OCONR¹⁴R¹⁵, NR^{5'}COR⁵, NR^{5'}CO₂R⁵, NR⁵CONR¹⁴R¹⁵ ou NR⁵CSR¹⁴R¹⁵, et
iii) Y et Z considérés ensemble sont =O, =CH-(Alk¹)ₙ-COR⁵, =CH-(Alk¹)ₙ-CO₂R⁵ ou =CH-(Alk¹)ₙ-CONR¹⁴R¹⁵,
R⁶ est
un atome d'hydrogène ou le radical méthyle,
ainsi que les sels pharmaceutiquement acceptables de ce composé.

29. Composition pharmaceutique comprenant un composé de la formule (I), tel que défini dans l'une quelconque des revendications 1 à 28, ainsi qu'un véhicule ou excipient pharmaceutiquement acceptable.

30. Procédé in vitro d'inhibition de testostérone-α-réductases, caractérisé en ce que l'on met les testostérone-α-réductases en contact avec un composé de la formule (I), tel que défini dans l'une quelconque des revendications 1 à 28.

31. Utilisation d'un composé de la formule (I) suivant l'une quelconque des revendications à 28, ou d'un sel physiologiquement acceptable d'un tel composé en vue de la fabrication d'un médicament destiné au traitement ou à la prévention d'une maladie sensible aux androgènes ou survenant à l'intermédiaire d'androgènes.

32. Utilisation suivant la revendication 31, caractérisée en ce que la maladie sensible aux androgènes ou survenant à l'intermédiaire d'androgènes est l'hyperplasie prostatique bénigne, la prostatite, le cancer de la prostate, l'acné, la calvitie hypocratique et l'hirsutisme.

33. Composé suivant l'une quelconque des revendications 1 à 28, destiné à l'utilisation dans le domaine thérapeutique.

34. Procédé de préparation d'un composé de la formule (I), tel que défini dans la revendication 1 ou d'un sel pharmaceutiquement acceptable ou d'un solvate d'un tel composé, caractérisé en ce que l'on fait réagir
(A) un composé de la formule (IX): 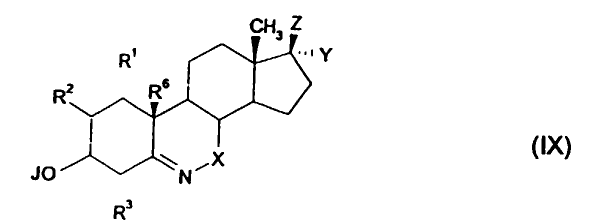 dans laquelle JO représente le radical hydroxyle ou un radical hydroxyle protégé, avec un agent oxydant, ou bien
(B) en vue de la préparation de composés de la formule (I), dans laquelle X représente -CH₂CH₂-,
on fait réagir un composé de la formule (XV): 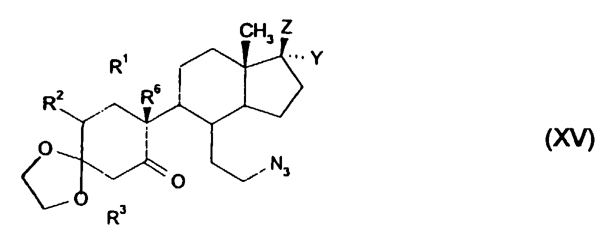 avec un agent réducteur et, si cela se révèle nécessaire et/ou souhaitable, on soumet le composé ainsi obtenu à une ou plusieurs autres réactions, qui comprennent les suivantes :
(i) conversion du composé obtenu de la formule (I) ou d'un sel ou d'un dérivé protégé de celui-ci en un autre composé de la formule (I), et/ou
(ii) enlèvement de n'importe quel groupe protecteur ou de n'importe quels groupes protecteurs, et/ou
(iii) conversion d'un composé de la formule (I) ou d'un sel de celui-ci en un sel pharmaceutiquement acceptable de ce composé.

35. Procédé suivant la revendication 34, caractérisé en ce que l'on déprotège un composé de la formule (IX), dans laquelle JO représente un radical hydroxyle protégé et on l'oxyde ensuite avec un agent d'oxydation approprié pour obtenir un composé de la formule (I), dans laquelle R⁴ représente un atome d'hydrogène.

36. Procédé suivant la revendication 35, caractérisé en ce que l'agent oxydant est un réactif de Jones.

37. Procédé suivant la revendication 34, caractérisé en ce que l'on traite un composé de la formule (IX), dans laquelle JO représente un radical hydroxyle protégé par un agent d'acylation, on le déprotège et on l'oxyde ensuite avec un agent d'oxydation approprié pour obtenir un composé de la formule (I), dans laquelle R⁴ représente un radical acyle.

38. Procédé suivant la revendication 37, caractérisé en ce que l'agent oxydant est le dichromate de pyridinium.

39. Procédé suivant la revendication 38, caractérisé en ce que l'on fait réagir un composé de la formule (XV) avec de la triphénylphosphine pour obtenir un composé de la formule (I), dans laquelle X représente un radical -CH₂CH₂-.
